# EUROPEAN PATENT SPECIFICATION

(11) **EP 0 324 659 B1**
(45) Date of publication and mention of the grant of the patent: **02.08.1995**
(21) Application number: 89300369.9
(22) Date of filing: 16.01.1989
(51) Int. Cl.: C07K 1/10, C07K 7/06

(54) **Enzymatic process for producing immunomodulating pentapeptides and intermediates for use in the process**
Enzymatischer Prozess zur Herstellung immunomodulatorischer Pentapeptide und Intermediate zu deren Herstellung
Procédé enzymatique pour produire des pentapeptides immunomodulateurs et intermédiaires utilisés dans celui-ci

(30) Priority: 14.01.1988 DK 158/88
(43) Date of publication of application: 19.07.1989
(73) Proprietor: CARLBIOTECH LTD. A/S, 3400 Hillerod (DK)
(72) Inventor: Aasmul-Olsen, Stig, DK-2942 Skodsborg (DK); Andersen, Anders J., DK-2980 Kokkedal (DK)
(74) Representative: Christiansen, Ejvind

(56) References cited:
- EP-A- 0 249 169
- EP-A- 0 288 965
- Chemical Abstracts, vol. 104, 1986, page 572, abstract 6179c, Columbus, Ohio, US; K. SUZUKI et al.: "Synthesis and antibacterial activity of N-acylated basic oligopeptide derivatives" & ANNU REP.TOHOKU COLL. PHARM., 1984, (31), 133-9

## Description

The present invention relates to a process for producing peptides of the general formula

R₁-A-B-C-D-E-R₂

wherein A, B, C, D and E represent amino acid residues coupled with peptide bonds and
- A: is a basic amino acid residue in L- or D-configuration, which is ω-amino or ω-guanidino substituted, or optionally substituted desamino derivatives of such amino acids,
- B: is a basic L-amino acid residue which is ω-amino- or ω-guanidino substituted, or B is L-Pro or L-dehydroPro,
- C: is an acidic amino acid residue in L- or D-configuration,
- D: is an unpolar, hydrophobic aliphatic L-amino acid residue or a homo- or heteroaromatic aryl- or aralkyl group containing L-amino acid residue, optionally being nitro-, hydroxy-, alkoxy-, alkyl- and/or halogen substituted on the aryl group,
- E: is an unpolar, hydrophobic aliphatic amino acid residue in L- or D-configuration or a homo- or heteroaromatic aryl- or aralkyl group containing amino acid residue in L- or D-configuration, optionally being nitro-, hydroxy-, alkoxy-, alkyl- and/or halogen substituted on the aryl group, or optionally substituted decarboxy derivatives of such amino acids,
- R₁: is H or an α-amino substituting group, such as a chemically or enzymatically cleavable α-amino protecting group, or is absent when A is an unsubstituted desamino derivative, and
- R₂: is OH or an α-carboxy substituting group, such as a chemically or enzymatically cleavable α-carboxy protecting group, or is absent when E is an unsubstituted decarboxy derivative.

The ω-substituted basic amino acids A or B are thus of the types

R₁′R₂′N - (CH₂)ₙ - CH(NH₂)COOH (I)

or 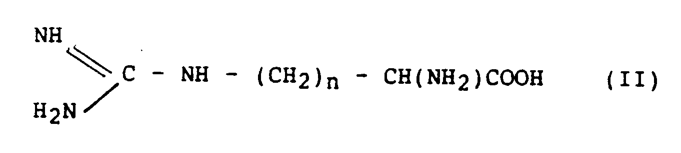
in which formulae n is 1-7, and R₁′ and R₂′ independently denote H, C₁-C₃ alkyl or pyranosyl, or R₁′ and R₂′ together form a group (CH₂)_{n′} where n′ is 3-8.

As mentioned, A may also have D-configuration and may further denote desamino derivatives of the said amino acids which, if desired, may be substituted with C₁-C₄ alkyl, hydroxy C₁-C₄ alkyl or halogen C₁-C₄ alkyl.

Preferred amino acids of type A include Arg (formula II, n=3), Lys (formula I, R₁′=R₂′=H, n=4), Orn (formula I, R₁′=R₂′=H, n=3), Homo-Arg (formula II, n=4) and Homo-Lys (formula I, R₁′=R₂′=H, n=5). The amino acids may be both in L- and D-configuration.

Independent of A, the basic amino acids B are preferably Arg, Lys, Orn, Homo-Arg, Homo-Lys in L-configuration or L-Pro.

The acidic amino acids C are preferably ω-carboxy substituted of the type

HOOC - (CH₂)ₘ - CH(NH₂)COOH (III),

where m=0-7
Preferred amino acids include Asp (m=1), Glu (m=2) or Homo-Glu (m=3) in L- or D-configuration.

The acidic amino acids C may, however, also contain an additional carboxy substituent or ω-sulphonic acid- or phonic acid group instead of the ω-carboxy group in formula III.

The unpolar hydrophobic aliphatic amino acids D are of the type

R₁ R₂ R₃ C - CH(NH₂)COOH (IV),

wherein
R₁, R₂ and R₃ independently denote H- or straight chain or branched alkyl.

Preferred acids include the L-configuration of
Val (R₁=H, R₂=R₃=CH₃)
Ile (R₁=H, R₂=CH₃, R₃=CH₃CH₂)
Leu (R₁=R₂=H, R₃=(CH₃)₂CH₂)
Nor-Leu (R₁=R₂=H, R₃=CH₃(CH₂)₂)
Nor-Val (R₁=R₂=H, R₃=CH₃CH₂)
Tert-Leu (R₁=R₂=R₃=CH₃)
Ala (R₁=R₂=R₃=H)
D may also denote L-amino acids having a homo- or heteroaromatic aryl- or aralkyl- side chain which may be nitro-, hydroxy-, alkoxy- and/or halogen substituted, expressed by the formula

Ar(CH₂)ₚ - CH(NH₂)COOH (V),

wherein p is 0-5 and Ar is an optionally substituted hetero- or homoaryl group. Preferred acids are Phe or Tyr.

Finally, the amino acids E may independently be the same as D, but both in L- and D-configuration.

E is preferably an aralkyl group containing L-amino acid having the formula V, where p=1, the ones preferred being
Tyr (Ar = p-hydroxyphenyl)
Trp (Ar = indolyl)
Phe (Ar = phenyl)
His (Ar = imidazolyl)
Nal (Ar = β-naphthyl) (3-(2′-naphthyl)-alanine)
E may also denote decarboxy derivatives of the said amino acids which, if desired, may be substituted with C₁-C₄ alkyl, hydroxy C₁-C₄ alkyl or halogen C₁-C₄ alkyl, preferably fluorine C₁-C₄ alkyl, or nitro-, alkoxy-, hydroxy- and/or halogen substituted Trp, Phe or His.

As mentioned, R₁ and R₂ may be enzymatically cleavable protecting groups, such as PGlu-, BzArg-, BzPro-, AcPhe-GlyNH₂, or R₁ may be a glycosyl acid group or an α-amino substituting group, e.g. of the urethane type (Z-, Boc or the like) or a carboxylic acid residue (Ac, Bz or the like including succinyl or trifluoracetyl). Such protecting groups are wellknown to the skilled person and are e.g. described in US patent specifications No. 4,339,534, 4,420,424 and 4,369,137 and in the literature references mentioned therein. R₁ may furthermore be H-.

R₂ may be a glycosyl amino group or an α-carboxyl substituting group, e.g. of the ester type (-OR, where R denotes methyl, ethyl, benzyl or the like), of the amino type including anilides (-NH₂, NR₃R₄, where R₃ and R₄ independently denote -H, alkyl or aryl), of the hydrazide type (-NHNHR₅, where R₅ = alkyl, aryl, -H, -CONH₂ (semicarbazide) or urethane), or R₂ may be -OH. Such protecting groups are also wellknown, cf. e.g. US patent specification No. 4,339,534.

The pentapeptides synthesized according to the invention exhibit or must be expected to exhibit immunomodulating effect as described in more detail below.

The present description employs the usual abbreviations within the peptide chemistry. The amino acid symbols are in accordance with IUPAC-IUB Commission on Biochemical Nomenclature, and the amino acids are in L-form unless otherwise stipulated. The following additional abbreviations are used:

| | | | |
|---|---|---|---|
| Z=Cbz | = benzyloxycarbonyl | HONSu | = N-hydroxysuccinimide |
| BOC | = tert.butoxycarbonyl | DDC | = dicyclohexyl-carbodiimide |
| Me | = methyl | DMF | = dimethylformamide |
| Et | = ethyl | TRIS | = tris(hydroxy-methyl)methylamine |
| tBu | = tert.butyl | NMM | = N-methylmorpholine |
| Glp | = pyroglutamyl | DME | = dimethoxyethane |
| Ac | = acetyl | TEA | = triethylamine |
| Bzl | = benzyl | HOBT | = 1-hydroxybenzo-triazol |
| Bz | = benzoyl | HONp | = p-nitrophenol |
| iPr | = isopropyl | Fmoc | = 9-fluorenyl-methyloxycarbonyl |
| Z(OMe) | = 4-methoxy-benzyloxy-carbonyl | HOOBT | = 3,4-dihydro-3-hydroxy-4-oxo-1,2,3-benzotriazine |
| SAR | = sarcosine | | |

In the following the nomenclature (..S₂,S₁,S₁′,S₂′...) has been used for defining the subsite-specificity of the enzymes, and analogously (..P₂,P₁,P₁′.P₂′...) for the corresponding binding position of the substrates in these sites, as stated by Schechter and Berger in Biochem. Biophys. Res. Comm. Vol. 27, p. 157-162 (1967).

Recent years have seen strong interest in the use of pentapeptides with the above structures for treatment of numerous disease involving defects in the immune function. A special one of these peptides is thymopentin
H-ArgLysAspValTyrOH,
which has a biological effect similar to that of the natural long chained polypeptide thymopoietin II, described in Schlesinger et al. in Cell 5, p. 361-365 (1975) and in US patent specification No. 4 002 740 (1977), and of which it constitutes fragment 32-36.

The biological effect hereof is i.a. described by M.E. Weksler et al., J. Exp. Med 148, p. 996-1006 (1978) and Goldstein, G. et al., Science 204, p. 1309-1310 (1979), and US patent specification No. 4 190 646 (1979). It appears that in particular there is an effect on the T-cell immune system, particularly the differentiation, maturing and the activity of T-cells, but not on the B-cell system. A survey of the effects is given in Survey of Immunologic Research, 4, (S1), p. 1-156 (1985) which also described experimental treatment of a variety of diseases.

It appears that thymopentin has modulatory effect on the immune system of animals and humans, and thus is widely usable for treatment of diseases caused by inadequate or otherwise abnormal immune function, an over- or underreaction. Similarly, the prognosis for treatment of non-responding individuals with infectious diseases using the other therapeutics can be significantly improved in certain cases. Example are e.g. the treatment of the immune deficiency disease DiGeorges Syndrom (congential Thymus deficiency), the treatment of autoimmune diseases with an unbalance between B- and T-cells and excess production of antibodies e.g. Rheumatoid arthritis and Systemic lupus erythematosis, and finally improvement of the prognosis for the treatment or prevention of chronic infections in i.a. leprosy and acute and chronic virus infections, e.g. Hepatitis B and Herpes simplex. Thus, it is obvious that the substance thymopentin possesses great potential value.

A peptide having a similar structure
H-ArgLysGluValTyrOH,
called spleninopentin after the corresponding natural hormone splenin (cf. Audhya et al., Biochemistry 20, p. 6195-6200 (1981) is described by Audhya et al., in Proc. Nat. Acad. Sci. (USA) 81, p. 2847-2849 (1984) and causes, like the natural hormone besides T-cell differentiation also the differentiation and maturing of B-cells and may thus be useful i.a. in the treatment of X-bound infantile hypogammaglobulinemi where this mechanism is defective.

More generally, peptides of the type herein described with C=Glu, i.e. known from US patent specifications No. 4 505 853 and 4 420 424, obtain spleninopentin-like effects, while the group with C=Asp obtain thymopentin-like effects, provided that certain structural demands are fulfilled, cf. Heavner et al, Archives of Biochemistry and Biophysics 242, pp 248-255 (October 1985).

From here it is i.a. seen that thymopentin-like effect in these assays is dependent upon the positively charged Arg in the A-position and the negatively charged Asp in the C-position, and that there is a demand for a hydrophobic amino acid in position E, which may be both Tyr, Phe, Trp, Val or His and substituted Tyr, while there are greater possibilities of variation in Position B and D. I.a. in US patent specification No. 4 505 853 this has been used to suggest more enzyme resistant analogues with i.a. B = Pro, while analogues substituted in Pos. E with Phe, Trp or His in Danish application No. 2892/85 and in the above article are said to be potent to an extent unknown from previous technique, the assay explicitly stressing an increased activity for Phe-substituted analogues.

Peptides of the above kind can be synthesized by known chemical coupling reactions, e.g. as described in Gross & Meienhofer (eds.): "The Peptides", Vol. 1 & 2, Academic Press, N.Y.: Analysis, Synthesis and Biology (1979 & 1980). It is, however, a common feature of these processes that they necessitate extensive use of protecting groups on the amino acid side chains, which entails additional synthesis steps with deprotection and consequently losses and side reactions and in some cases solubility problems; and often necessitate extensive use of organic solvents and severe reaction conditions. The high risk of racemization is another common feature, cf. i.a. D.S. KEMP in the above work, Vol. 1 p. 315-382, under the relatively severe synthesis conditions as is the risk of a variety of other side reactions, of. e.g. Bodanszky, M. et al., Side Reactions in Peptide Synthesis (Int. J. of Meth. in synthetic Org. Chem.), 5, p. 333-355 (1981). Thymopentin involves other special problems which will be mentioned below.

The classical Solid-Phase Method according to Merrifield, J. Am. Chem. Soc. 85, p. 2149-2154 (1963) is thus used in its original or slightly modified form in the patent specifications and applications Nos. US 4 190 646; US 4 258 152; SE 446 867; US 261 886; EP 18.182; DK 149093; US 4 505 853 and DK applications No. DK 5455/84 and DK 2892/85, although the possibility of classical standard solution-phase methods is mentioned and a few examples are given. In addition to the above usual disadvantages of chemical coupling synthesis according to classical method, it is a further drawback of the solid-phase methods that they are unsuitable, i.e. inconvenient and uneconomic when synthesizing large amounts (>100 g), cf. the comments in US patent specification No. 4 369 137 and EP patent specification No. 42.291. The product obtained may also be relatively impure due to side reactions and incomplete reactions during the process and severe reaction and deblocking conditions.

Examples of synthesis by classical solution-phase methodes are given in US patent specification No. 4 505 853 and 4 547 489.

US patent specification Nos. 4 298 523 and 4 369 137 and the corresponding EP patent specification No. 42.291 concern solution-phase methods for synthesis of peptides with R₁ = H, A = Arg, B = Lys or Sar, C = Asp or Glu, D = Val or Sar, E = Tyr and R₂ = -NH₂, -OH or optionally substituted -OBzl.

They describe three strategies; two fragment coupling strategies, both with fragment coupling in C, and a stepwise reverse method; i.e. classical. In all cases the peptide bonds are obtained by classical, known chemical methods, primarily by means of active esters, although also activation by mixed or symmetrical anhydrides, acid chloride and the azide methods is mentioned. In reality, the patents and the application are so drafted that other activation methods are precluded.

DE offenlegungsschrift No. 3421614 A1 and DK application No. 2549/85 relate to a solution-phase synthesis procedure for synthesizing Arg-Lys-C-Val-E-R₂, where C is Glu or α-amino adipic acid in L or D-configuration, E is Trp or Tyr in L or D-form, and R₂ is -H, -NH₂, -NH-R, or -OR,where R is alkyl or aryl. However, the method is also said to be usable more generally for analogues i.a. with A or B=Orn or homo-Arg. These compounds affect the T-cell maturing.

Classical condensation methods are employed, e.g. DCC/HOOBt, and it is said that the risk of racemization is smaller here - but not eliminated. Standard protecting groups are employed, and recently Fmoc or primarily Z and Benzyl. It is said that by previously known synthesis methods (i.a. from US patent specification No. 4 420 424) both Trp and Tyr will form biproducts.

The novel feature of this procedure resides in synthesizing the side chain protected derivative R₁-A(x)-B(x)-C(x)D-OH and finally condense it with the H-Trp-R₂ or H-Tyr-R₂ derivatives, subsequently cleaving the R₁ and X-substituents and optionally R₂ by catalytic transfer hydrogenation in formic acid/dimethylformamide-or /dimethylacetamide-mixtures. It is simultaneously stressed as a problem that deprotection of e.g. ZArg(Z₂)Lys(Z)Glu(OBzl)ValTrpOBzl, which is not slightly soluble due to the large number of protecting groups, is not otherwise readily possible. Catalytic transfer hydrogenation with formic acid must, however, be expected to cause side reactions, such as formylation.

The present invention relates to novel processes for synthesizing peptides having the formula

R₁-A-B-C-D-E-R₂

defined in more detail in the above formula specification - use being made of proteolytic enzymes. The processes of the invention are characterized in what is stated in the characterizing part of claims 1 and 3
While the above peptides have not been made earlier by enzymatic synthesis, the basic principle proper in enzymatic processes for peptide synthesis is known. A detailed background study is made in US patent specification No. 4 339 534 and the corresponding EP patent specification No. 17.485.

Enzymatic peptide synthesis has in some cases been used advantageously for industrial scale processes, cf. i.a. the survey article by F. Widmer & J. T. Johansen in "Synthetic Peptides in Biology and Medicine", pp. 79-86, Elsevier (1985) which also mentions the general advantages of the method, but from which it is also evident that the method is not yet universal, generally and readily usable for the average person skilled in the art, a viewpoint shared by M. Bodanszky in Int. J. Pept. Protein res. 25, p. 449-474 (1985) stating that the biochemical formation of peptide bonds is a promising method but not yet generally applicable, which also goes for fragment couplings. Consequently Bodanszky concludes that classical peptide synthesis methods will be of great importance also in the future.

It could therefore not be expected with certainty by the average person skilled in the art that the peptides of the above formula having a particular sequence containing a basic-basic-acidic-hydrophobic-hydrophobic amino acid some of which may be on D-form would lend themselves to enzymatic peptide synthesis. by the processes according to the invention
As further explained in detail below having regard to some of the preferred individual coupling conditions, they also differ significantly from prior art reactions using similar enzymes.

As a more specific illustration of prior art enzymatic peptide synthesis a number of examples neither of which describes the incorporation of D-amino acids or peptides containing D-amino acids are given:

Applicant's abovementioned EP patent specification No. 17485 describes a process for producing peptides of the general formula

Aₓ-Bₓ-Zₓ

wherein Aₓ represents an N-terminal protected amino acid residue or an optionally N-terminal protected peptide residue having a C-terminal L-amino acid residue, and Bₓ represents an L-amino acid residue, and Zₓ is OH or a C-terminal protective group, by reaction of a substrate component with an amine component in the presence of an enzyme, and, if desired, cleavage of optional terminal protective groups to provide a peptide of the formula

Aₓ-Bₓ-Zₓ

which is characterized by reacting a substrate component selected from
amino acid esters, peptide esters, depsipeptides, or optionally N-substituted amino acid amines or peptide amines, or optionally N-terminal protected peptides of the formulae

Aₓ-ORₓ, Aₓ-NR_{y}R_{y}′

or

Aₓ-Xₓ-OH

wherein Aₓ is as defined above,
Rₓ is alkyl, aryl, heteroaryl, aralkyl or an α-desamino fragment of an amino acid residue,
Rₓ and R_{y}′ are each hydrogen, alkyl, aryl, heteroaryl or aralkyl, and
Xₓ is an L-amino acid residue,
with an amine component (nucleophile) selected from optionally N-substituted L-amino acid amides, L-amino acids or L-amino acid esters, of the formula

H-Bₓ-NR_{z}R_{z}′

or

H-Bₓ-OR_{w}

wherein Bₓ is as defined above,
R_{z} and R_{z}′ are each hydrogen, hydroxy, amino, alkyl, aryl, heteroaryl or aralkyl, and
R_{w} is hydrogen, alkyl, aryl, heteroaryl, or aralkyl,
in the presence of an L-specific serine or thiol carboxypeptidase enzyme originating from yeast or of animal, vegetable or other microbial origin, in an aqueous solution or dispersion at pH 5-10.5, preferably at a temperature of 20-50°C. The preferred enzyme is carboxypeptidase Y from yeast, called CPD-Y in the following. (It is noted that in order to avoid confusion with the present symbol meaning the above-mentioned symbol meanings are not identical with those used in EP patent No. 17485).

This patent is based on the at that time surprising recognition of the applicability of carboxypeptidase enzymes in enzymatic peptides synthesis.

As described and exemplified in detail CPD-Y has a very broad specificity and numerous coupling reactions have been exemplified. It is stated, however, that amino acids whose side chains containing carboxylic groups (Asp or Glu) are difficult to incorporate as amine components, and that heterocyclic amino acids like Pro and His are extremely difficult to incorporate as free acids. In the patent no examples of preparation of pentapeptides are given. However, the production of Met-enkefalin Tyr-Gly-Gly-Phe-Met-OH by tryptic cleavage of
Bz-Arg-Tyr-Gly-Gly-Phe-Met-OH
produced by stepwise synthesis catalyzed by CPD-Y is illustrated in the parallel AU patent No. 545416.

This amino acid sequence is very different in character from the claimed, particularly by containing neither Pro nor acidic amino acids.

A number of tetra- and tripeptides are synthesized, neither of which contain a basic-acidic-hydrophobic or Pro-acidic-hydrophobic acid tripeptide sequence, let alone a corresponding tetrapeptide sequence containing a further N-terminal basic amino acid. As further explained below with reference to Andersen A. J. CPD-Y cannot catalyze the formation of an Arg-Pro bond.

All in all, based on the above patent-family, the average person skilled in the art would have serious doubts whether enzymatic peptide synthesis would be feasible for pentapeptides of the claimed particular structure.

Another representative example of enzymatic peptide synthesis is US patent No. 3972773 (Isowa et al.) corresponding to DE patent No. 2518256.

The patent describes a process for producing a peptide of the formula

X-A-B-C-Y

wherein
- A: is Ala, Gln, Asn, ω-BOC-Lys, Leu, Gly, Glu, Glu(OCH₃), Pro, or A can be A₁-A₂ where A₁ represents a hydrophilic amino acid residue, which can be Ala, Gln, Asn, ω-BOC-Lys, Leu, Gly, Glu, Glu(OCH₃) or Pro and A₂ is Val, Met, Leu or Gln
- B: is Phe, Tyr, Leu, Met, Glu, Asp, Gln, Asn or Trp
- C: is Phe, Leu, Ile, Tyr, Cys(SBzl), Ser(OBzl), Trp or Met and
- X and Y: are protective groups or terminal protected amino acid or peptide residues,
which comprises reacting a peptide X-A-B-OH with an amino acid or peptide H-C-Y in the presence of the enzyme pepsin.

In the patent specification the criticality of the A-B-C sequence as defined above is stressed, and it appears that the given definition does not match with the sequence basic-basic or Pro-acidic-hydrophobic-hydrophobic, since the only basic amino acid (Lys) is used as α,ω-BOC-Lys, i.e. protected to neutralize the positive charge. In some of the examples X is BOC-Arg(NO₂), i.e. again the basic character is neutralized and the solubility of the end product is decreased.

The fact that the basic amino acids must be protected in order to be incorporated would demotivate a person skilled in the art from using the known process for the production of the present compounds, since the basic character is crucial for the immunomodulating activity and since removal of the side-chain protective groups would result in a number of unwanted byproducts which are difficult to separate.

It does appear from Isowa's examples, that the definition of A, B and C cannot be combined at random. Thus Pro is never bound to an acidic amino acid (Glu or Asp), nor for that matter are the protected and therefore not basic α,ω-BOC-Lys or BOC-Arg(NO₂).

In the only example where Asp is used, the yield is only 17%. In the survey "Proteasen als Biokatalysatoren für die Peptidsynthese", Die Pharmacie, (1982) 37, p. 101, it is shown that pepsin does not catalyze the reaction of Z-Val-Tyr-OH and H-Phe-ODpm, the yield being 0%.

Since Arg-Lys-Asp-Val-Tyr is the most important of the compounds to be produced according to the present invention, the person skilled in the art would have serious misgivings with regard to the applicability of enzymatic peptide synthesis based on a closer study of Isowa et al.

The enzymatic reactions take place in solution; however, the enzymes may be in suspension, emulsion or immobilized in a completely or partly aqueous medium containing 0-90% of a water miscible or immiscible organic solvent and under mild pH-conditions, pH 5-10.

Thus, it is possible to work under conditions where racemization does not occur. Nor do several others of the said side reactions occur under such mild conditions.

It is possible to omit the side chain protecting groups which means fewer synthesis steps and purifications and smaller losses, also under possibly severe deblocking conditions. Further, this ensures fine solubility in aqueous media, which both alleviates the syntheses and the purifications and makes feasible purification by reverse phase HPLC and ion exchange chromatography. In the process according to claim 1 A and B are unprotected on the side chain However, in certain cases it is advantageous also in the enzymatic couplings to introduce a few side chain protecting groups, as the product then precipitates from the aqueous medium or is extracted into an organic phase with consequent almost quantitative yield. This alternative is possible in some of the variants according to claim 3. C is preferably protected with -OBzl, which entails a surprisingly fine enzymatic result, irrespective of C being Asp or Glu, also with C in P₁-Position in the substrate.

Here and in the following, "substrate" and "nucleophile" in connection with enzymatic couplings mean the carboxy component and the amine component, respectively, in the peptide bond formed.

Finally, the both absolute and kinetic stereospecificity of the enzymes used makes it possible in certain positions both to incorporate D-amino acids, e.g. if E is in D-form, and to obtain an enzymatic kinetic or absolute resolution if a mixture is used, due regard being taken to the characteristics of the enzyme used. This again offers two synthetic advantages, viz. partly that relatively impure starting substances, which are consequently cheaper, can be used, partly that one or more steps can be carried out by classical coupling technique, if economical, and then by means of the enzymes in the following steps to exclude a possible racemic isomer not participating in the further reaction or being converted very slowly and inefficiently. Reference may e.g. be made to Thorbek, P. & Widmer, F. (1985). Enzymatic synthesis of D-amino acid containing peptides. In "Peptides: Structure and Function, Proceedings of the 9th Americal Peptide Symposium". Esd. Deber C.M. Hruby V. J. and Kopple K.D., pp. 359-362. Illinois, Pierce.

From this reference it appears i.a. that while on the substrate side the degree of stereospecificity is high, which can be utilized both for the coupling step proper and for e.g. formation of E-R₂ bonds by deblocking stereospecifically enzymatically, there is on the nucleophile side a possibility of incorporating D-amino acids in certain positions, depending on the enzyme, while the relatively poorer yields entail that racemic impurities on the nucleophile side can be eliminated by corresponding enzymatic steps, if a pure L-isomer is desired.

Under certain conditions it is also possible to use N-α-unprotected amino acid derivatives in D-configuration as substrates for D-L bond-formation, cf. e.g. patent application No. WO 88/06187 published August 25, 1988.

The process according to the invention for producing R₁-A-B-C-D-E-R₂, as defined more closely in the formula specification, by use of enzymes, can roughly be divided according to the coupling strategy used. Such six main strategy types are stated below in the reaction schemes I to VI. Here Z₍ₙ₎, Y₍ₙ₎ and X₍ₙ₎ respectively are an α-amino-, an α-carboxy- and a ω-side chain-H-substituting group on the n'th amino acid, counted from A towards E. Z₍ₙ₎ and X₍ₙ₎ may, however, also denote H, and Y₍ₙ₎ may denote OH. As X is preferably -H in a strategy with minimal side chain protection it is only included where it is particularly relevant, which in no way should be considered limiting to the invention. This also goes for the brief mention of side chain protecting or deprotecting steps in the reaction sequences. Thus, in the following "unprotected" amino acids means "non-side chain protected", unless otherwise stated in the context.

The coupling reaction proper can take place in four fundamentally different ways in the individual steps:
1) Enzymatic condensation.
2) Enzymatic aminolysis of amides.
3) Enzymatic aminolysis of esters.
4) Condensation by means of a chemically activated α-carboxy group.

The first three reaction types are described in more detail in Widmer, F. & Johansen, J. T. in "Synthetic peptides in Biology and Medicine", Alitato, K. et al. (eds), Elsevier (1985) and the references mentioned therein; this description is hereby incorporated by reference. To facilitate understanding the three reaction types are illustrated by the following examples from Widmer & Johansen, Op. cit.

### 1. Condensation:

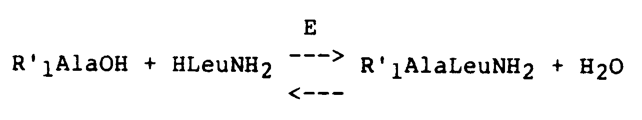

### 2. Aminolysis of amides (Transpeptidation):

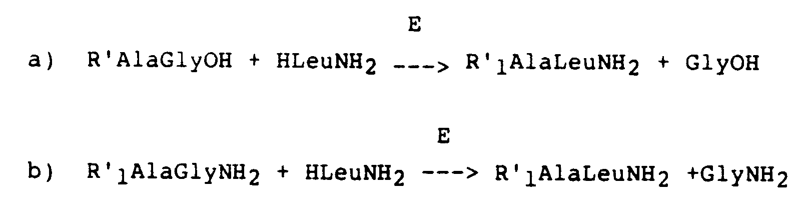

### 3. Aminolysis of esters:

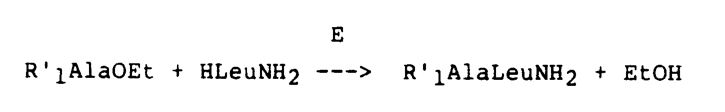

(R'₁ = H or a protecting group; E = enzyme)
Similarly, methods of chemically activating an α-carboxy group for the coupling are mentioned in Gross-Meienhofer (eds.), "The Peptides", Vol. 1 & 2, Academic Press, N. Y.: Analysis, Synthesis and Biology (1979 & 1980), which is also incorporated by reference.

As to the substitution structure when forming the peptide bond betwen the n'th and n+1'st amino acid residue it should briefly be emphasized:
Z₍ₙ₊₁₎ is always H- in these reaction types.

In the case "Condensation" Y₍ₙ₎ is always -OH.

In the case "Enzymatic aminolysis of amides" Y₍ₙ₎ is -N-R₃R₄, where R₃ and R₄ independently are alkyl or aryl, or H or optionally R₃ = H and R₄ = peptide residue, in which case the reaction is a genuine transpeptidation. Normally, however, it is a simple amide or optionally an anilide.

In the case "Enzymatic aminolysis of esters" Y(ₙ) is - OR, where R = alkyl or aryl of simple or complex or optionally substituted nature. Preferably, it is simple aliphatic or aromatic esters, e.g. -OMe, -OEt, -OiPr or -OBzl.

In the fourth method of chemical activation Y₍ₙ₎ may e.g. be an active ester, e.g. -ONSu, -OOBt or -ONp, which can be formed by reaction with a carbodiimide, such as DCC, or simply the activated carbodiimide complex, or it may be an azide group, a mixed or symmetrical anhydride, an acid chloride or the like.

By the processes according to the invention the above four condensation methods can be used as supplement to each other, dependent on the amino acid sequence and possible protecting groups. If a few chemical activation steps are introduced it may, however, be necessary to carry out suitable enzymatic steps, e.g. coupling steps, ensuring that wrong isomers possibly formed are eliminated. A special case of this type involving no coupling is a C-terminal modification which e.g. takes place upon enzymatic introduction of the E-R₂ bond. N-terminal modification can also be used.

Other types of C-terminal modification, both chemical and enzymatic, in which Y₍ₙ₎ is changed prior to a coupling, can take place during the synthesis. For reasons of simplicity, most of these modifications are, however, left out of the reaction schemes I-VI. This should in no way be limiting to the invention. An example of the use of this type of modification in the strategy is enzymatic deamidation succeeded by a chemical esterification leading to an Y₍ₙ₎ ester, i.a. as mentioned by Breddam, K., Carlsberg Res. Commun. 49, p. 535-554.

The following should be noted about identity, introduction and removal of the groups R₁, R₂ and X₃: For reasons of clarity and simplicity it is in all strategies statd that R₂ is not introduced until after the last coupling step, whereafter R₁ is introduced and X₃ finally removed; i.e. in 3 separate steps. This is, however, in no way to be limiting to the invention. If e.g. Z₁ = Cbz- and Y₅ = X₃ = OBzl, it will be possible to remove all three groups simultaneously by catalytic hydrogenation. Hereby R₁ and R₂ can also be introduced simultaneously, if there are H- and -OH respectively. In certain cases it will also be expedient if R₁ and R₂ are introduced from the beginning; e.g. if R₁ = peptide residue, e.g. PGlu and if R₂ = -NH₂. Then the schemes should be read R₁ = Z₁, and R₂ = Y₅ and the corresponding synthesis step is left out. Finally, X₃ can be removed early, e.g. by simultaneous hydrogenation of X₃ = OBzl and Z₃ = Cbz-, whereafter the scheme should be read X₃ = -H, and the last step is left out. X₃ can also from the beginning be -H. More generally, Xₙ may be all conventional protecting groups of the side chains in question known the the person skilled in the art, (or -H), as well as enzymatically cleavable ones, such as Glp, just as Zₙ can be all conventional amino protecting groups, including enzymatically cleavable ones, e.g. BzArg-, or -H. Particularly preferred ones are, however, Z₍ₙ₎ = Cbz- or Boc- and X₃ = -OBzl or -H. The protecting groups can be split off by catalytic hydrogenation or acid treatment, but in general all conventional deprotecting methods can be used, including enzymatic deprotection.

In all the strategies I to IV the intermediate Z₁-A(X₁)-B(X₂)-Y₂ is formed, where Y₂ most often is an ester and B-Y₂ surprisingly can be coupled as such. Most often X₁ = X₂ = -H, as is the case in the process according to claim 1 The latter unprotected intermediate has proved to be particularly useful for esterolytic enzymatic successive coupling, i.a. for fragment coupling with endoproteases. In strategy I, successive stepwise coupling proceeds, one amino acid residue at a time, from A towards E. In strategy II, fragment coupling is made with the fragment CDE-Y₅, where C may be side chain protected, but surprisingly does not need to be so in enzymatic couplings, which also goes for the corresponding fragment coupling with CD-Y₄ in strategy III. In strategy II the fragment CDE-Y₅ is synthesized by stepwise reverse procedure, where the fragment DE-Y₅ is surprisingly catalytically efficient in enzymatic reactions of the condensation type. Strategy II is finished by introducing E-Y₅, quite analogously with strategy I. Stragegy IV is finished by a strategy II-like reaction with coupling with the fragment DE-Y₅, thus achieving the same catalytic advantages as in strategy II, additional surprisingly fine results being achieved with X₃ = -OBzl and X₁ = X₂ = H in some cases, quite as is the case with strategy V ending with a similar step. Here the fragment Z₁ABC-Y₃ is synthesized, C optionally being protected, by a stepwise reverse procedure ending by the coupling with Z₁A-Y₁. A similar coupling ends strategy VI, where the fragments Z₂-BC-Y₃ and DE-Y₅ have been prejoined. One further strategy is possible, viz. stepwards reverse.

The invention also relates to protected dipeptides, tripeptides and tetrapeptides mentioned in claims 21-28. These intermediates are particularly suitable for the above synthesis strategies as will be further explained below: Structurally related oligopeptides are disclosed in CA 104:6179c, EP-A-288 965, EP-A-249 169, US-A-3 362 947, DE-A-1 184 770, FR-A-2 294 715, GB-A-2 086 910, and EP-A1-67 425.

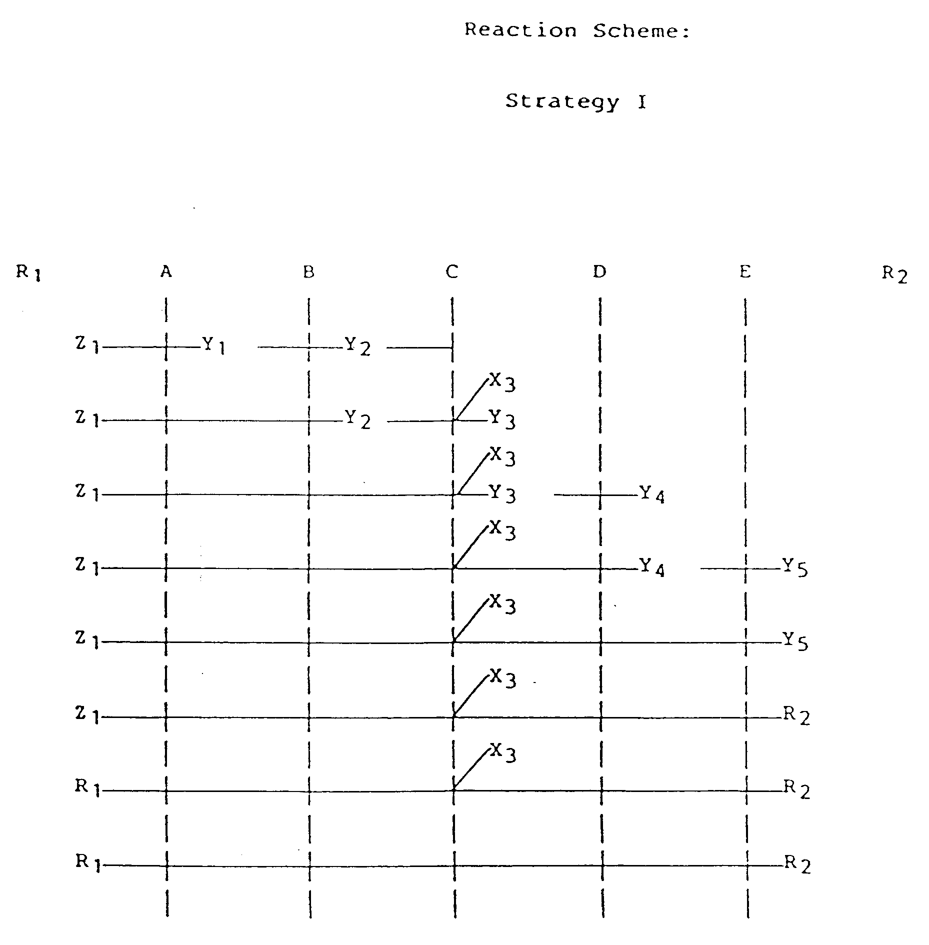

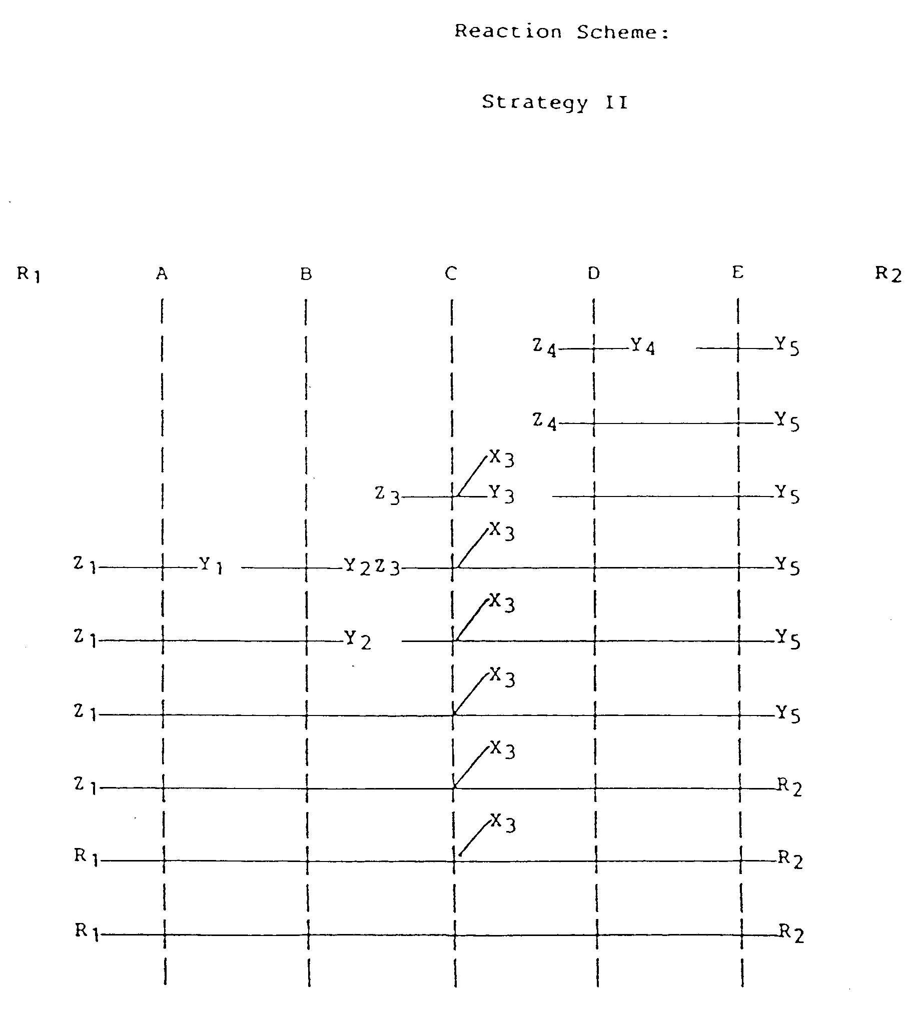

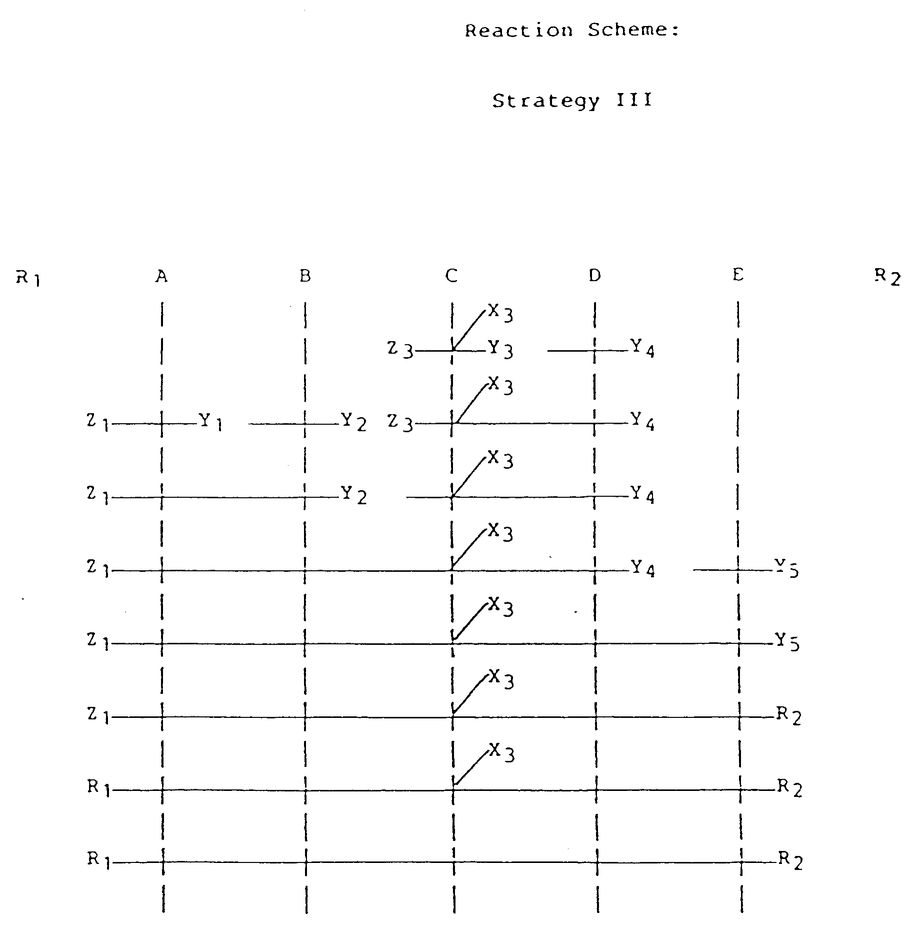

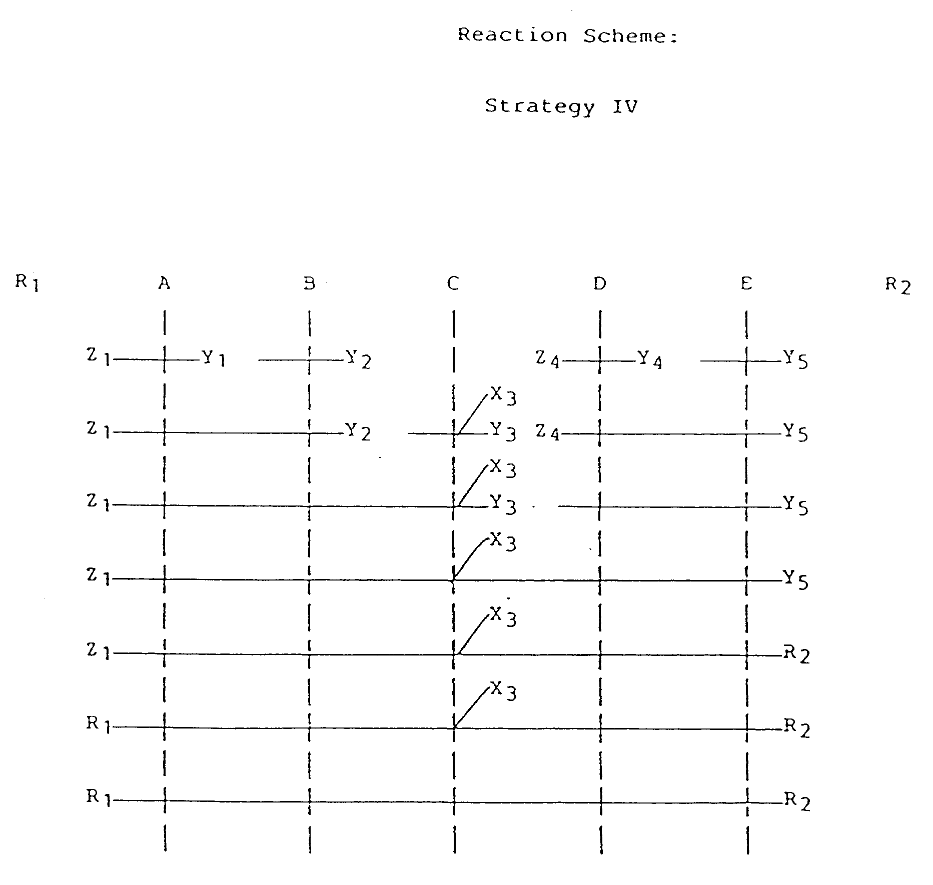

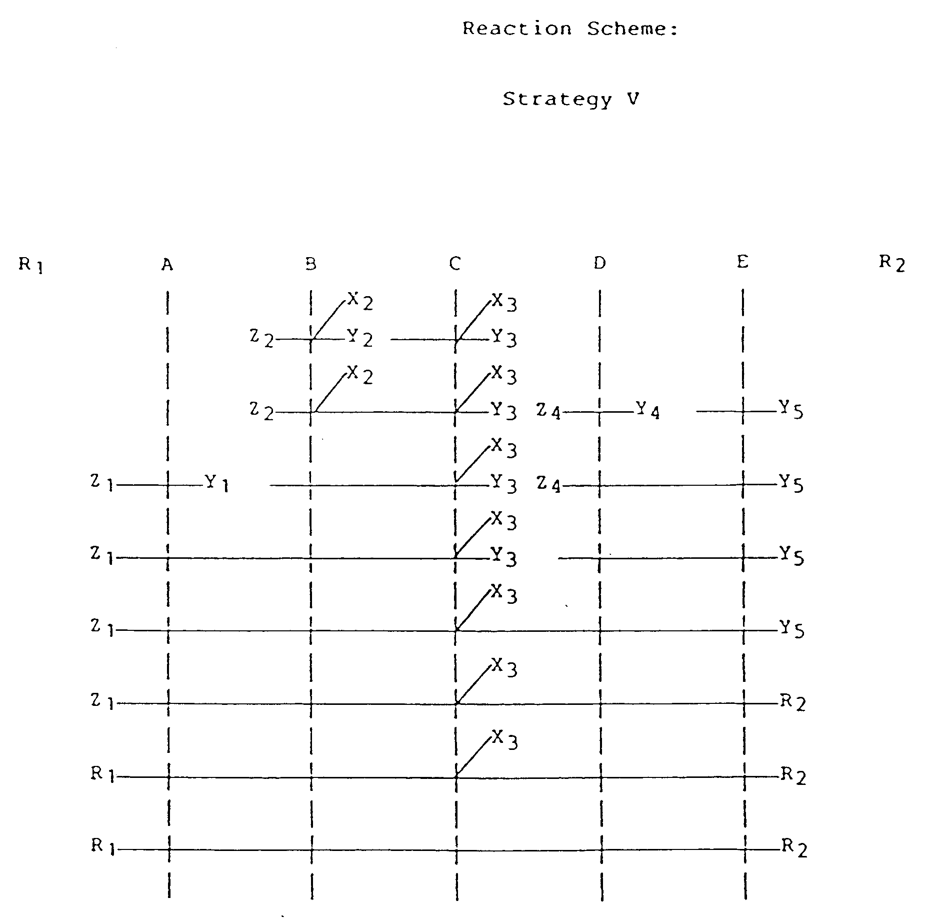

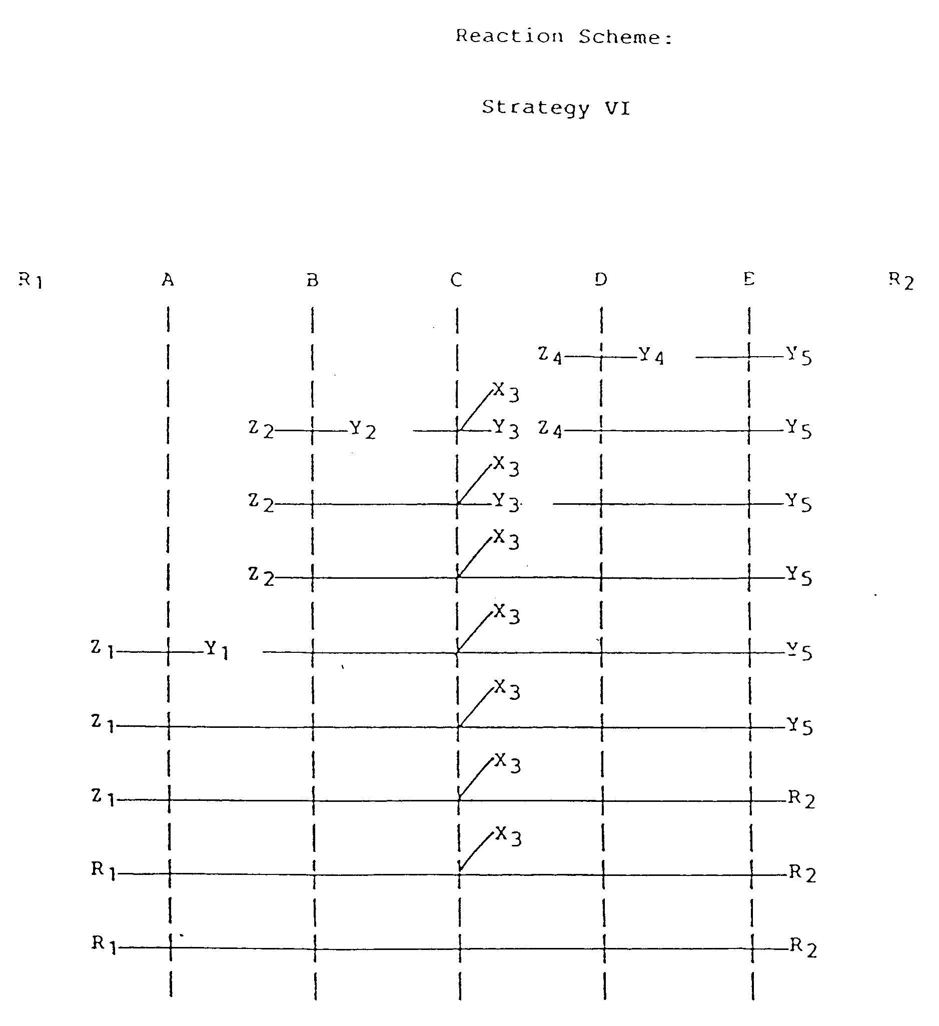

### Enzymatic methods of bond formation

According to the present invention an endoprotease or a dipeptidyl-carboxypeptidase or -peptidase can be used for enzymatic formation of the peptide bonds if the n'th + 1'st amino acid is bound to an amino acid n + 2 via a peptide bond, and if not, both endoproteases and mono-carboxypeptidases can be used. The enzymes may both be produced by yeast or be of animal, vegetable or microbial origin, and they may act via a covalent or non-covalent mechanism, with e.g. serine-, thiol- or metallo groups as activating groups. The enzymes may be in solution, suspension or in emulsion or in immobilized form optionally bound to a carrier substance. The reactions take place in a partly aqueous medium, in the presence of 0-90% of a water miscible or immiscible organic solvent; preferably 0-40% of water miscible solvents. pH ranges from 3-12; preferably 4-8 in condensation reactions, and 8-10 in esterolytic reactions. It is generally preferred to use mild reaction conditions between pH 5-10, but dependent on the enzyme used, the temperature and the reactants, pH may lie within the interval 3-12. The temperature is 0-70 °C, preferably 20-35 °C dependent on the enzyme and the conditions as such.

The nucleophile concentration lies within the interval 0.05-2.0 M, preferably 0.1-1.0 M, the substrate concentration in the interval 0.01-1.0 M, and the enzyme concentration in the interval 0.5-200 »m, preferably 1-5- »m, dependent upon the reaction types.

According to need, salts, such as NaCl and CaCl₂, and/or detergents or other additives, such as urea and guanidine hydrochloride may be added to the reaction mixtures, and similarly thiol stabilizing reagents, such as β-mercaptoethanol and dithiothreitol (DTT) and/or metal chelating compounds, such as EDTA, may be added according to need; e.g. in case of thiolproteases.

In the following a number of concrete examples will be given of carboxyl ester hydrolase (E.C. 3.1) and peptide hydrolase (E.C. 3.4), enzymes capable of forming the bonds between A, B, C, D, & E. A survey of these, grouped according to type and enzyme source, appears from table I. This table comprises enzymes particularly advantageous for use in reactions according to the invention, but other peptidases must be considered capable of catalyzing similar reactions. The same goes for chemically or biologically modified peptidases or other optionally biosynthetic catalysts. The sources stated in the table are only included as examples. The table also includes examples of enzymes which can be used for the formation of E-R₂ or R₁-A bonds or for other similar N- or C-terminal modifications, such as deamidation and deesterification.

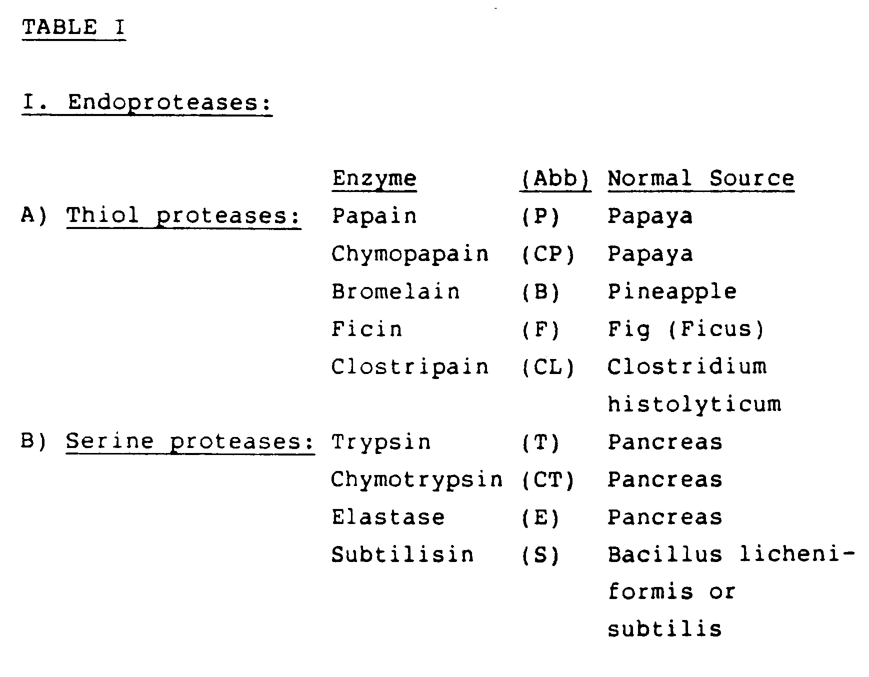

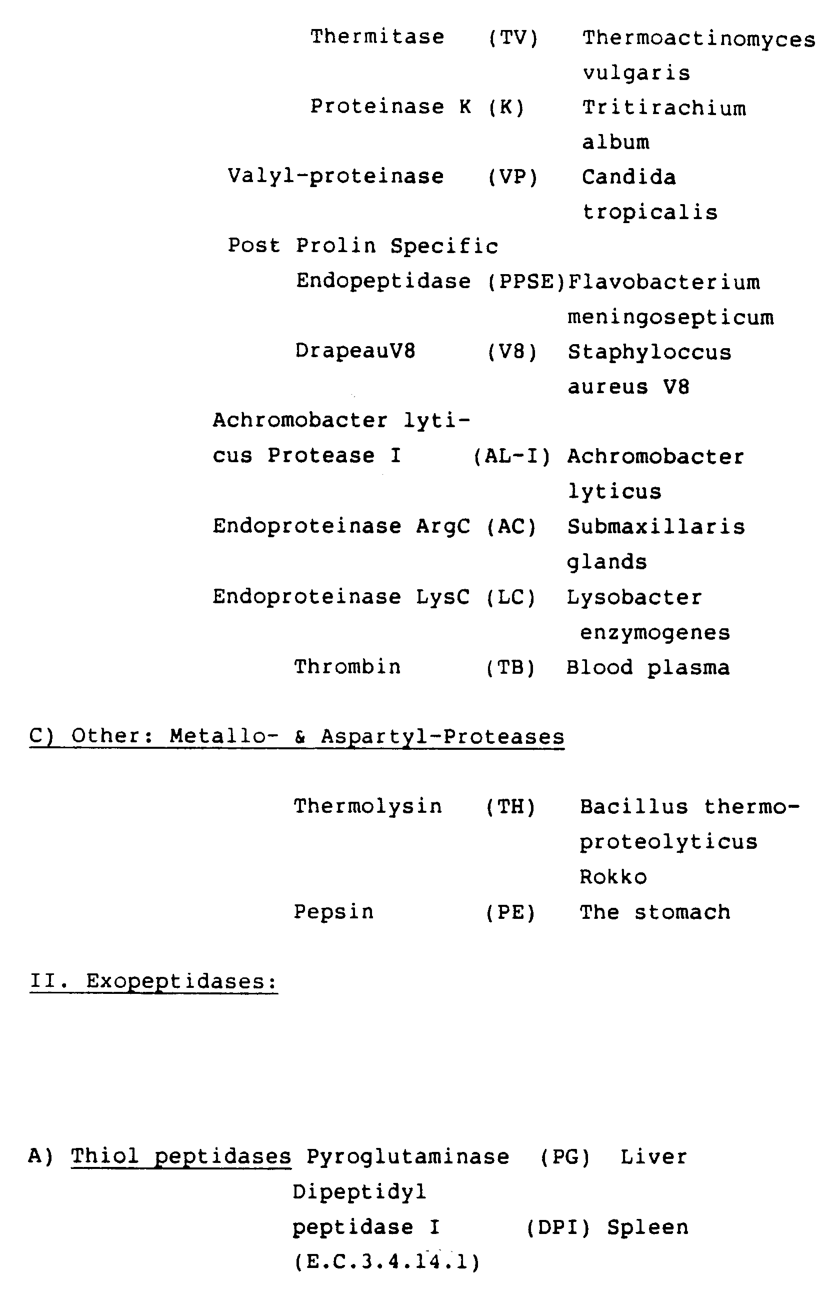

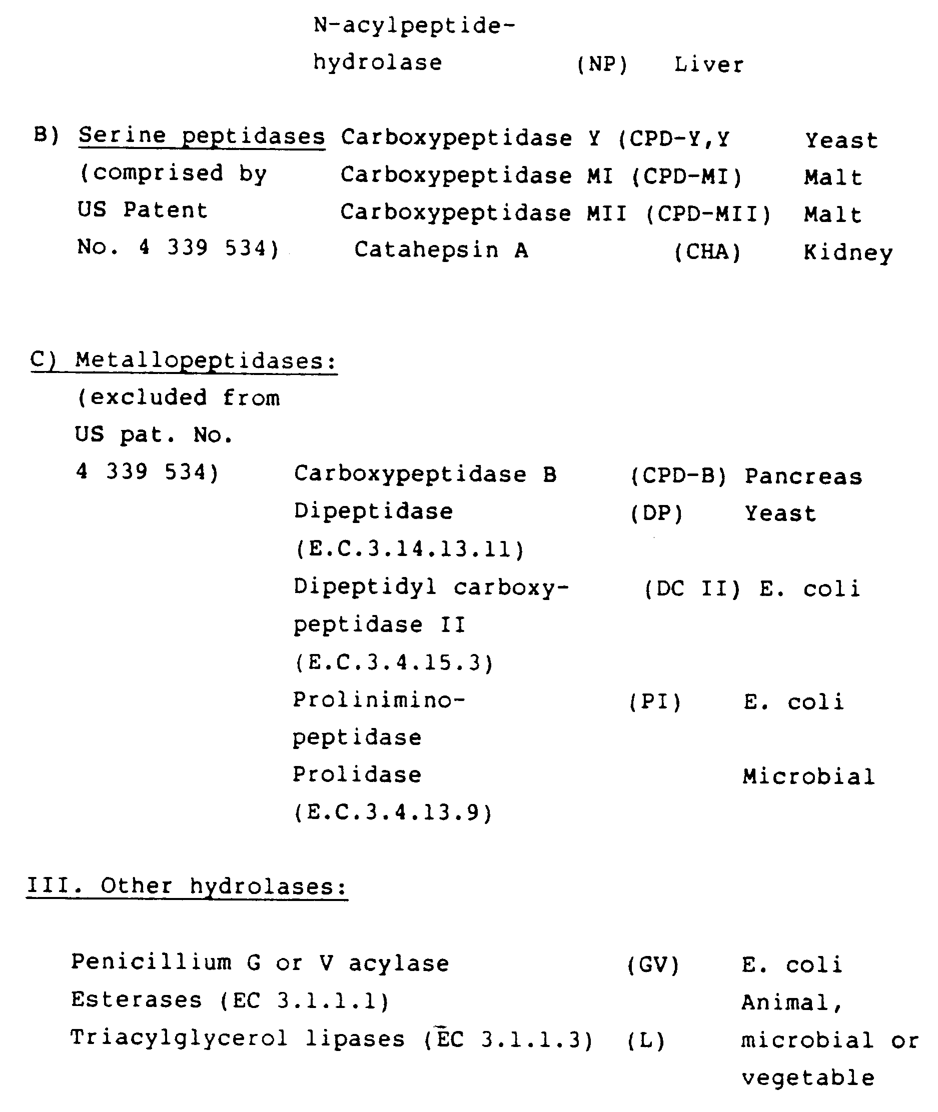

For a closer description of these enzymes reference is made, as regards the enzymes well known at that time, to Perlmann, G.E. et al., in Colowich, S. P. & Kaplan, N. O. (eds.) Methods of Enzym. 19, Academic Press (1970), and 45, (1976), and Fruton, S., Adv. Enzymol. 53, p. 239, Wiley (1982), Abassi, A et al. Biol. Chem. Hoppe-Seyler, 367, p. 441-45 (1986) and finally Breddam, K., Carlsberg Res. Commun. 51, p. 83-128 (1986). Commonly used lipases and esterases are mentioned in G.M.R. Tombo et al., in "Biocatalysis in Organic Media", Laane C. et al. (Eds.), p. 43, Elsevier, Amsterdam (1987). All of these articles are hereby incorporated by reference.

Generally is should be noted that the fact that a given peptide bond is hydrolyzable with a given enzyme does not unconditionally mean that the same peptide bond can also be synthesized by that enzyme; as an example, reference is made to Andersen, A.J. (1985). Enzymatic synthesis of arginine proline peptide bonds using clostripain as a catalyst. In "Peptides: Structure and Function, Proceedings of the 9th American Peptide Symposium". Eds. Deber C.M., Hruby V.J. and Kopple K.D., pp. 355-358. Illinois, Pierce, giving 6 references describing in vain efforts of enzymatically synthesizing bonds with Pro as nucleophile where the enzymes used, i.a. CPD-Y, are capable of or must be considered capable of hydrolyzing the corresponding bonds. Andersen, however, demonstrated that the bond could be synthesized with clostripain, as far as known the only reported enzyme with this effect.

According to their hydrolytic properties the enzymes can, however, be placed in main groups having similar mechanism of effect and substrate specificity, and it must be expected that such enzymes often possess mutually uniform, qualitatively synthetic properties. When an enzyme from such a main group is found efficient in the formation of a certain bond in a certain formula specification, it can often be deduced that other enzymes from the same group should be capable of forming the same bond.

In view of this, claim 10, 12, 14, 16, 18 and 20 state usable categories of enzymes for the individual bond types, while the corresponding claims 11, 13, 15, 17, 19 and 21 state main croups comprising usable as well as the preferred enzymes within each main group.

For a better understanding of this main group division a number of examples are given below, the abbreviations of table I being used for the individual enzymes. This explanation and the following more specific study will enable the skilled person to select enzymes usable for the individual couplings, dependent upon the carboxy- and amine component present.

The serine endoproteases T, AL-I, AC and LC and TB and the thiol endoprotease CL all exhibit activity towards basic amino acids in the S₁-position, where CL, TB and AC mostly are arginyl specific, while AL-I and LC are mostly lysyl specific. The thiol endoproteases P, CP, B and F belong to a group of plant thiol endoproteases having a similar S₁-specificity towards positively charged amino acids, however, with a strong influence from hydrophobic groups in the S₂-position.

Dependent upon the concrete formula specification they are consequently capable of mediating the formation of the A-B or B-C bond, or usable for introducing the R₁-A-bond, if Z₁ is an optionally hydrophobically substituted basic amino acid residue other than A and B.

The "papain"-group is furthermore usable for introducing E-R₂, D being a hydrophobic amino acid residue.

The serine endoproteases CT and S and TV exhibit activity for hydrophobic amino acids in the S₁-position, and are thus usable for introducing C-D, where C is a hydrophobically protected amino acid residue. If C is unprotected, V8 can be used - having preference for negative charges in the S₁-position. TH can be used both with protection and unprotected C, provided that D (the S′₁-position) is hydrophobic.

D and E both being hydrophobic amino acid residues it follows that all the above enzymes - due regard being taken in each case - are usable for the D-E bond, as long as the said enzymes do not also cleave the C-D bond.

Finally, when C is hydrophobically protected, the D-E bond can furthermore be established with P, CP, B and F.

In particular if D is a small hydrophobic amino acid residue, such as Ala, Val or Ile, elastase will be the one most usable besides the carboxypeptidase CPD-Y.

D and E both being hydrophobic, the E-R₂ bond can be introduced with P, CP, B, CT, S and TV (the last two particularly with esters). When the D-E bond is resistent and Y₅ is a hydrophobic amino acid residue, TH or PE are usable. When Y₅ is an ester group, a lipase or esterase may also be used.

In addition to the enzymes mentioned above, dependent upon the formula specification and the nature of Z₁, various other enzymes can be used for introduction of the R₁-A bond. Thus, serine endoproteases with hydrophobic S₁-preference, such as CT, S and TV, are usable if Z₁ is a hydrophobic α-protected amino acid residue other than D. If Z₁ is an acetylated amino acid residue NP can be used, and if Z₁ is an unprotected amino acid residue a suitable aminopeptidase can be used e.g. when Z₁ = Pro the enzyme PI. If e.g. Z₁ = phenoxyacetyl, R₁-A can be introduced by means of Penicillium V acylase.

For the sake of clarity, after this somewhat general study of the criterions for the choice of usable enzymes, the individual bond types will be explained in the following. The main weight is put on the enzymes preferred in the individual steps, dependent upon the various strategies.

The following endoproteases should be especially emphasized as examples of enzymes usable for forming bonds between A and B: Clostripain and Papain and Chymopapain, particularly if Z₁ is Cbz-, Boc-, or AcPhe-, and trypsin, particularly if Y₂ is other than an ester of if the B-C bond has already been formed. Clostripain is preferred if B = Pro, cf. i.a. Andersen, A.J. op. cit. In stragegies I to IV, the A-B bonds are so formed that also exopeptidases can be used. Examples are CPD-Y and CPD-MII, both comprised by US patent specification No. 4 339 534. Here it is particularly advantageous that Y₂ is other than an ester. CPD-MII is i.a. described by Breddam, K. in Carlsberg Res. Commun. 51, p. 83-128 (1986).

Examples of preferred endoproteases for forming bonds between B and C are trypsin, in particular if the C-D bond has already been formed and X₃ = Y₃ = -OBzl and Postprolin Specific endopeptidase if B = Pro. In strategies I, IV, V, VI exopeptidases may also be used for forming the B-C bond. Particular emphasis should be given to carboxypeptides MI & MII, and Y should be emphasized, in particular if X₃ is other than -H and Y₃ = -NH₂.

The C-D bond can e.g. be established by means of the endopeptidases Drapeau V8, if X₃ = -H, and e.g. by chymotrypsin or subtilisin if X₃ is other than -H. In both cases it is possible to use Thermolysin. Surprisingly fine results have been obtained with Thermolysin if Y₃ = -OH. In strategies I and III it is furthermore possible to form the C-D bond by means of exopeptidases. Here particular emphasis should be given to the carboxypeptidase Y and MII, in which case Y₄ = -OH is also possible. Alternatively, lipases or esterases may be used.

The D-E bond can be established by endoproteases, such as e.g. Elastase, if D is e.g. Ile or Val, or Chymotrypsin if D is Leu, preferably if Y₅ is other than ester or E is an amino acid residue in D-configuration. In all strategies it is possible to establish this bond by means of exopeptidases, and here Y is the preferred carboxypeptidase in all cases. Alternatively, lipases or esterases may be used.

It is also the enzyme particularly preferably for the stereospecific C-terminal modification capable of introducing the E-R₂ bond. However, use can also be made of endoproteases having activity for hydrophobic amino acid residues, e.g. Chymotrypsin and Subtilisin. Alternatively, esterases or lipases may be used.

The R₁-A bond can be introduced by means of e.g. Pyroglutaminase if Z₁ is Glp, or the endoproteases Clostripain if e.g. Z₁ is BzArg and A = B = Lysin, or Papain if R₂ = -OH has already been introduced. If Z₁ = Pro, proliniminopeptidase can be used, and if Z₁ is an acetylated amino acid residue, N-acylpeptide hydrolase can be used.

In the following some of the prior art enzymatic couplings will be dealt with in more detail. These couplings offer certain points of resemblance to some of the couplings preferred according to the invention, but they have in no instance been used in connection with synthesis of peptides of the kind herein described.

Thus Mitin et al., Int. J. Pept. Protein Rec. 23, p. 528-34 (1984) describe the use of papain in various peptide synthesis. Mitin uses acylamino acid alkylesters as carboxyl component, in practive Bz- or Z-protected amino acid methylesters, such as BzAlaOCH₃, ZGlyOCH₃ and BzArgOCH₃, the latter corresponding to Zl-A-Y₁ according to the invention. However, Mitin's amine component is always amino acid- or peptide amides or -tert. butylesters, such as Asp(OH)ValNH₂, ValNH₂ or LeuOtBu, or unprotected peptides, such as GlyPheLeu or PheGly.

The use of amino acid amides, amino acid-tert.butylesters, amino acid-phenylhydrazides or di- or tripeptides as amine components (nucleophiles) is all in all very widespread in enzymatic peptide synthesis. The reason is probably that, if possible, an unambiguous synthesis with one well defined coupling product is desired. This cannot be expected if the amine component e.g. is a simple alkyl ester of an amino acid or a peptide, because the coupling product formed is often again a substrate for the enzyme used. The coupling product will thus react further to form a new product and a polymerization has started. In such a mixture it may be difficult to isolate the individual products, quite apart from the fact that the yield of the desired coupling product will be low. On the other hand, by using amine components which are carboxy protected, e.g. amides, or being quite unprotected, coupling products are obtained which are far more stable towards the above secondary hydrolysis, as peptides having a carboxyterminal with e.g. an amide group are very often converted at a factor 1000-10.000 times slower than the corresponding ester substrate.

It is thus particularly surprising that papain can provoke coupling between ZArg-alkyl- or -benzyl esters and HLys-alkyl- or -benzyl esters, as papain exhibits specificity towards basic amino acids on the carboxy side of the decomposition product with consequent risk of a secondary hydrolysis of the ZArgLys-ester formed, cf. Tzuzuki et al., J. Biochem. 88, 669 (1980) and Morihara, Tibtech, June 87, Vol. 5, p. 168. Consequently it could not be expected that lysine could be used without side chain protection. On a whole, the ZArgLys-alkyl esters, particularly ZArgLysOEt are surprisingly easy to synthesize in high yields on the basis of cheap starting materials.

Furthermore, these alkyl esters, in particular ZArgLysOEt, are particularly interesting intermediates of the type Z₁-AB-Y₂ used in the process according to claim 1 which can emcompass all of strategies I, II, III and IV.

The said non-side-chain protected intermediate has turned out particularly advantageous in esterolytic enzymatic successive coupling, in particular in fragment coupling with endoproteases, such as trypsin (strategy II and III) and in stepwise coupling (strategy IV).

It is particularly surprising - as demonstrated below in example A (strategy II) - that ZArgLysOEt (Z₁-AB-Y₂) can be fragment coupled in the presence of trypsin with HAspValTyrNH₂ (CDE-Y₅) in a yield as high as 50% without side chain protection of C, as Oka and Morihara, J. Biochem, 87, p. 1057 (1977) have demonstrated that BzArgOEt only couples with HGluNH₂ in a yield as low as 11% with trypsin.

Further, as demonstrated in example E (strategy III) ZArgLysOEt also couples with unprotected HAspValOEt (CDY₄) in the presence of trypsin in a yield as high as 65%.

It is furthermore surprising that the thiol endoprotease bromelain, which as mentioned above belongs to the same class as papain and has the same primary specificity, is capable of hydrolyzing the α-ethylester ZArgLysOEt completely without cleaving the peptide bond between Arg and Lys. Thus, as demonstrated below in example E, bromelain can be used to mediate the synthesis of ZArgLysAspValOEt by fragment coupling in a yield as high as 63% without side reactions.

As demonstrated in example C (strategy IV) ZArgLysOEt can also be coupled with HGlu(OBzl)₂ which is easy and cheap to produce as compared with HGlu(OBzl)OH in the presence of trypsin under formation of ZArgLysGlu(OBzl)₂, which is an interesting intermediate.

Both protecting groups are removed by catalytic hydrogenation, and the ArgLysGlu-peptide was characterized.

For use in the successive couplings it is, however, preferred to maintain the side chain protecting group (X₃); not for reasons of synthesis, as would be the case if it was a classical chemical synthesis, but because by maintaining the side chain protecting group under the reaction conditions chosen it is achieved that the coupling product, as opposed to the reactants, precipitates. This precipitation means that the coupling product is removed from the reaction mixture, whereby the reaction equilibrium is shifted to the benefit of the synthesis, and consequently the product.

The elegant feature of the synthesis thus resides in ZArgLysGlu(OBzl)₂ being treated with a suitable protease having esterase activity, in the said case subtilisin, so that the α-benzylester is selectively split off in a pure synthesis without side product. Without in any way purifying this product, the reaction mixture is mixed with another suitable protease, in this case thermolysin, and the bond from C to D is established.

Irrespective of the coupling being Z₃C(X₃) + DE-Y₅ (strategy II), Z₁ABC(X₃) + DE-Y₅ (strategies IV and V) or Z₃C(X₃) + D-Y₄ (strategy III), thermolysin is a preferred enzyme for the bond from C to D.

Isowa et al., Bull. Chem. Soc. Jpn., 52(3), p. 796-800 (1979) describes that N-protected and side chain protected Asp and Glu can be coupled with e.g. ValOMe, LeuOMe, IleOMe and ValNH₂, LeuNH₂ and MetNH₂ in the presence of thermolysin. Also Isowa et al., Tetrahedron Letters No. 28, p. 2611-2612, 1979 and EP patent appln. No. 99585, have coupled N-protected and side chain unprotected Asp and Glu with L-Phe-alkylesters invariably leading to an addition product (salt) of the dipeptide and the Phe-alkylester, e.g. Z-L-Asp-L-PheOMe·L-Phe-OMe. When racemic substrates or nucleophiles were used, only L-isomers were involved in the condensation to give L-L dipeptides, while the amine part of the salts were almost exclusively D-isomers. These authors, however, only investigated dipeptide synthesis and possible optical resolution and the yields obtained are considerably lower than those obtained according to the invention when comparable substrates and nucleophiles are used. The former Isowa et al. thus convert Z(OMe)Glu(OBzl)OH with HValNH₂ and obtain a yield of 66%, while Oka and Morihara, J. Biochem, 88, p. 807-813, 1980, react ZPheOH with HValNH₂ in a yield of 49%. These yields are to be compared with the below 90% yield obtained according to the invention.

The high stereospecificity of thermolysin both with regard to the S₁-position (Isowa et al.) and especially in the S₁′-position, (Jakubke et al. Die Pharmazie, 37, p. 89-106, 1982). On this background it was surprising that it was possible as shown below in example Q to incorporate a D-amino acid derivative Z-D-asp(OBzl)OH as substrate in a thermolysin-catalyzed reaction in the S₁-position and that it was possible as shown below in example S to incorporate HPheD-tyrNH₂ having a D-amino acid in the S₂'-position as the nucleophile in analogous process.

Morihara et al., Biochem. Biophys. Res. Comm., 84 (1), 1978, p. 95-101 have demonstrated that thermolysin is capable of transpeptidizing between two hydrophobic amino acids. Jakubke et al. op.cit have shown that both Val and Tyr are able to act as substrates for thermolysin. In view of this, it could not be foreseen that amino components of the type DE-Y₅, such as ValTyrNH₂ without problems could be coupled with C (Asp or Glu), as a cleaving of the bond between Val and Tyr could be expected with consequent risk of transpeptidation, This aspect of the invention is the basis for the process according to claim 3. Similarly it was surprising that as shown in example R HTyrTyrOEt could be coupled to Glu without transpeptidation, not least because as shown in these and other examples Val and Tyr are also capable of acting as nucleophiles in similar reactions. However, it surprisingly turned out that ValTyrNH₂ acts as an activating nucleophile at the thermolysin coupling with Asp or Glu, thus even making it possible to use extraordinarily low amounts of thermolysin, and that ValTyrNH₂ is resistent to hydrolysis and/or transpeptidation by thermolysin. Thus, ValTyrNH₂ is approx. 10-100 times more catalytically efficient than typical values of other, shorter nucleophiles, which correspondingly reduces the required amount of enzyme.

This entails the further advantage that a relatively impure ValTyrNH₂ can be used for the coupling, e.g. synthesized by chemical coupling as described in more detail below. Thus, a certain racemization of Val in the Val-Tyr nucleophile can be tolerated, as such impurities will be eliminated by the thermolysin in the succeeding coupling step.

It is known from the literature (Matos et al., Biotechnology Letters 9, (4),223-236 (1987) to synthesize dipeptides also in LD-configuration by lipase-catalyzed reaction of a protected uncharged amino acid ester with a D-amino acid derivative, viz. D-alaOiPr and D-val-OBzl. However, it has never in these reactions been tried to use longer peptides as substrate or substrates containing charged amino acids, such as Arg, Lys and Asp, and Valin have only been used as nucleophile, and that in ester-form.

On this background it could not be expected that as shown in example O longer peptides containing basic and acidic unprotected amino acids can be used as substrate with Valin-ester as the activating ester. D-valOBzl has previously been used as a nucleophile. As the product thus obtained is stable it could not be expected that L-Val-ester substrates are particularly suited also for incorporating other D-nucleophiles.

This recognition solves a synthesis problem, as it is known from the literature (cf. Thorbek & Widmer, Peptides, op.cit p. 359) that while chymotrypsin is capable of coupling D-nucleophiles, it is inactive towards valin-substrates (Fruton, 1982, op.cit), while CPD-Y and elastase, which are active towards valin-substrates, do not at all and very poorly, respectively, couple D-amino acids.

It is also known from the literature (cf. Walter and Yoshimoto, JACS 17, (20), 4139-4144 (1978) that Postprolin-specific-endoprotease exhibits a high stereospecificity in the S₂-position. On this background it was highly surprising that it was possible as shown in example T to catalyze the synthesis of ZDargProAspValOEt by means of this enzyme.

The method according to the invention is described in more detail in the following examples employing the following.

### General procedures

All reactions in the examples described were followed by analytical reverse-phase HPLC using suitable gradients of elution systems containing 50 mM triethylammoniumphosphate, pH 3.0, 0%-80% acetonitril. The elution was followed by an UV-detector at a suitable wavelength within the interval 220 nm-280 nm. The syntheses were carried out at room temperature unless otherwise indicated.

In the reaction course in the enzymatic syntheses, pH was from the beginning adjusted to a fixed value within the interval 7-9, and this value was maintained during the entire course by current addition of a suitable base. Where not otherwise indicated, the products were isolated by reverse-phase HPLC and solidified by freeze drying.

The isolated products were hydrolyzed in 6 M hydrochloric acid in vacuum for 16-36 h at 100°C and subsequently identified by amino acid analysis (hereinafter AAA).

Throughout the examples the symbol "Z" is used to denominate a benzyloxycarbonyl group, while the symbol "Cbz" is used in the claims to avoid confusion with the protective groups generally demoninated Z₁,Z₂ etc.

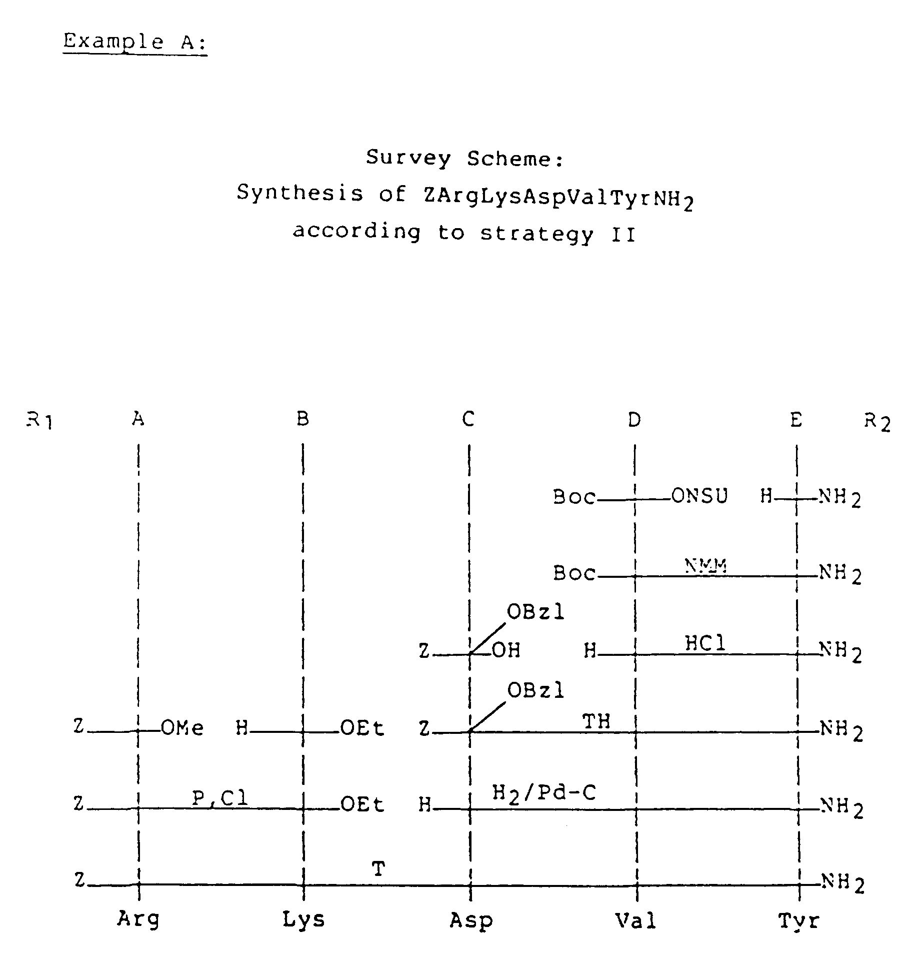
Example A:

Survey Scheme:
Synthesis of ZArgLysAspValTyrNH2
according to strategy II


R1 A B C D E R2

Boc ONSU H NH2

Boc NMM NH2

OBzl
Z OH H HCl NH2

OBzl
Z OMe H OEt Z TH NH2

Z P,Cl OEt H H2/Pd-C NH2

Z T NH2

Arg Lys Asp Val Tyr

### Example A:

### Synthesis of ZArgLysAspValTyrNH₂(Z-Thymopentin-amide) according to strategy II

1) Synthesis of ZArgLysOEt.2AcOH
a) Synthesis of ZArgOMe.HCl
Reaction scheme: 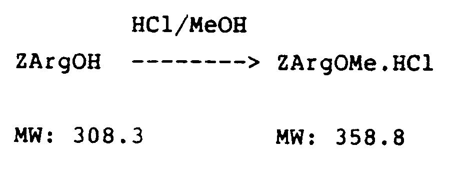 Procedure:
69.5 g ZArgOH (225 mmole) were slurried in 360 ml dry methanol and dry HCl was added to a concentration of approx. 2 M HCl with cooling. The starting substance dissolved during this process. Additional HCl was added until the conversion was more than approx. 95% complete according to analytical HPLC, the mixture standing at room temperature with stirring for approx. 2 hours. The reaction mixture was subsequently repeatedly evaporated to dryness in vacuum with intervening resolution in dry methanol.
Yield: 78.4 g (97%) (amorphous glass)
b) Synthesis of ZArgLysOEt.2AcOH by papain-coupling
Reaction scheme: 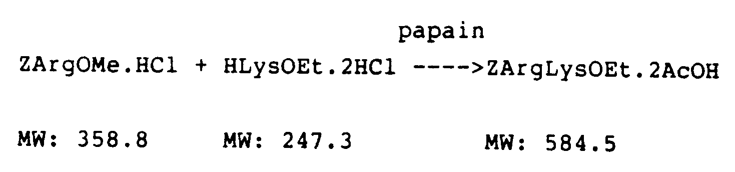 Procedure:
264 g L-Lysine ethyl ester dihydrochloride (1.07 mole) were dissolved in a mixture of 570 ml H₂O and 500 ml MeOH, and 1.6 g EDTA.NaH₂O were added, whereafter pH was adjusted to 8.5 with 290 ml NaOH-solution. 77.0 g ZArgOMe.HCl (215 mmole) from the previous step were dissolved in 820 ml methanol and added, pH was readjusted to 8.5 and 0.75 β-mercaptoethanol was added. The reaction was then initiated by adding 0.1 g papain, preactivated in a β-mercaptoethanol-solution. The reaction was followed by analytical HPLC, standing at room temperature with stirring. After approx. 2 hours a yield of approx. 80% and a conversion exceeding 95% were observed. The reaction was then discontinued by pH-adjustment to 2.5 and the mixture was then purified.
Yield according to HPLC: 80% (determined at 254 nm)
c) Purification of ZArgLysOEt.2AcOH
The reaction mixture from the previous step was evaporated for methanol and purified by RP-HPLC C18-columns. The columns were eluted using a gradient with 50 nM AcOH and increasing alcohol content in H₂O. The pure product fractions were combined and repeatedly evaporated to dryness with dry ethanol.
Yield: 90.5 g (72%) amorphous glass)

| Analysis: | | |
|---|---|---|
| AAA: | Arg: | 1.02 |
| | Lys: | 0.99 |

d) Synthesis of ZArgLysOEt by clostripain-coupling
Reaction scheme: 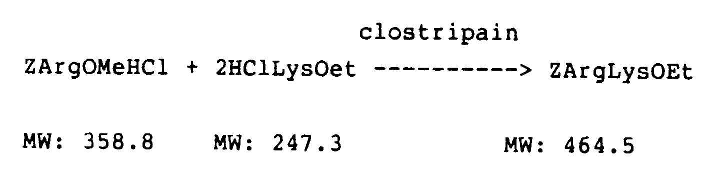 Procedure:
2.47 g LysOEt.2HCl (10 mmole) were dissolved in 12 ml H₂O + 2 ml EtOH (99.9%). 0.22 g Ca-acetate + 31 mg DTT were added, pH was adjusted to 8.5 with 0.95 ml TEA. 0.72 g ZArgOMe.HCl (2 mmole) was added and pH readjusted to 8.5 with 0.25 ml TEA. H₂O was added to 20 ml. The reaction was initiated by adding 2 mg clostripain preactivated in a DTT-solution. Followed by analytical HPLC. After 20 hours all substrate had been converted.
Yield according to HPLC: 90% (determined at 254 nm)
2) Synthesis of HCl.HValTyrNH₂
a) Synthesis of BocValONSu 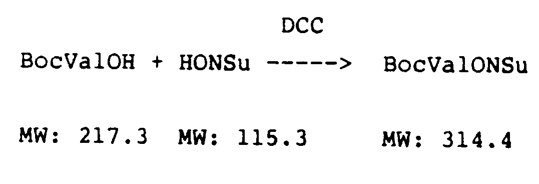 Procedure:
76.5 g Boc-L-Valin (352 mmole) were slurried in 850 ml methylene chloride at room temperature until almost in solution. 38.0 g (330 mmole) N-hydroxysuccinimide were then added, cooling to 0°C in ice bath, the reaction subsequently being initiated by addition of 80.0 g DCC (388 mmole) dissolved in 150 ml ice cooled methylene chloride. Stood for 1 hour at 0 °C and overnight at 4 °C. The product obtained was in solution. According to analytical HPLC, the starting substance had been converted after approx. 20 hours, and the reaction mixture was filtered for DCU formed and evaporated to dryness, whereafter it proceeded to the next step.
Yield according to HPLC: 100%
b) Synthesis of BocValTyrNH₂
Reaction scheme 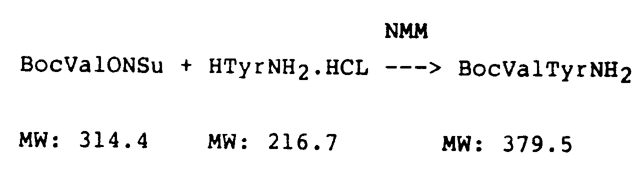 Procedure:
The 110.7 g BocValONSu (352 mmole) from the previous step were dissolved in 1.0 l DMF and 114.5 g L-tyrosinamidehydrochloride (528 mmole), and the reaction was initiated by adding 59 ml N-methyl-morpholin. The reaction stood at room temperature with stirring, and was followed by analytical HPLC. 100 ml AcOH were added after approx. 1 1/2 hours, and after standing for some time the mixture was filtered and subsequently evaporated to dryness in vacuum.
Yield according to HPLC: 95% (determined at 254 nm)
c) Isolation of BocValTyrNH₂
The reaction mixture was redissolved in absolute EtOH and the product was precipitated by addition of water and ice cooling, whereafter it was filtered off, washed and dried.
Yield: 113.5 g (85%)
d) Synthesis of HCl.HValTyrNH₂
Reaction scheme: 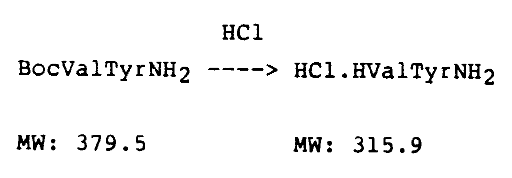 Procedure:
The 113.5 g dry BocValTyrNH₂ (299 mmole) from the previous step were slurried in 2.0 l 2.0 dry HCl in EtOAc, and stood with stirring for 2 hours. According to analytical HPLC the reaction has stopped and the mixture was evaporated in vacuum.
Yield: 94.4 g (100%)

| Analysis: | | |
|---|---|---|
| AAA: | Val: | 1.08 |
| | Tyr: | 0.92 |

e) Synthesis of HCl.HValTyrNH₂ with elastase catalysis
Reaction scheme: 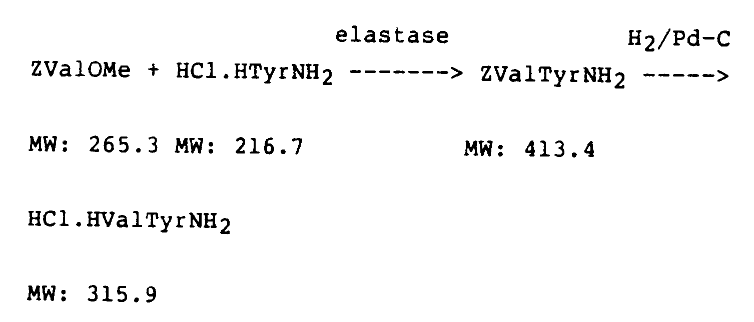 Synthesis of ZValTyrNH₂
Procedure:
2.16 g HTyrNH₂.HCl (10 mmole) were dissolved in 3.4 ml DMF and 4 ml 0.5 M TRIS mixed with 0.18 g KCl. Adjustment of pH to 8.6 with NaOH-solution. Volumetric adjustment to 18 ml with H₂O. 0.3 g ZValOMe (1.1 mole) is added to the reaction mixture, and the reaction nitiated by adding 2.5 ml of a solution of 50 mg elastase in 5 ml H₂O. Followed on analytical HPLC. After 2 days approx. 35% product is formed.
Yield according to HPLC: 35% (determined at 254 nm)
Synthesis of HCl.ValTyrNH₂
RP-HPLC isolated product from the previous step is taken into solution and mixed with 10% (w/w) 10% palladium-on-carbon, followed by hydrogenation on a Parr-hydrogenation apparatus at a hydrogen pressure of 3 bar in the presence of 2 mole equivalents HCl in the solution. The reaction mixture was subsequently filtered and evaporated.
3) Synthesis of HAspValTyrNH₂
a) Synthesis of ZAsp(OBzl)ValTyrNH₂
Reaction scheme: 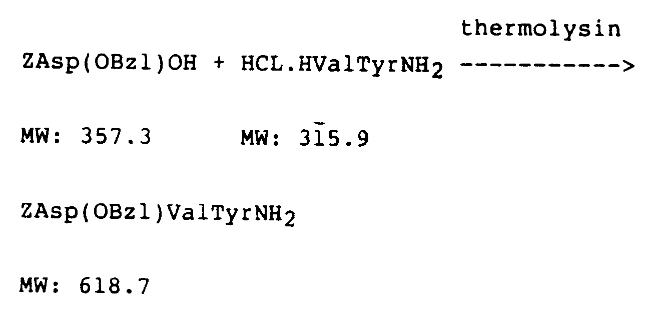 Procedure:
10.5 g HCl.HValTyrNH₂ (33.2 mmole) were dissolved in a mixture of 90 ml H₂O and 8 ml DMF. 1.82 g CaCl₂.6H₂O and 0.5 g TRIS were added, and pH was adjusted to 7 with NaOH. 5.98 g (16.7 mmole) N-α-Z-L-aspartic acid β-benzylester were dissolved in 34 ml DMF and added, pH being readjusted to 7. The reaction was initiated by adding a solution of 47 mg thermolysin in 14 ml H₂O. The product precipitation initiated shortly after, and the mixture was left to stand at room temperature with stirring for approx. 1 day until analytical HPLC showed good conversion. pH was occasionally reduced with a HCl-solution. The product was isolated by filtering off and washed with a DMF-water-mixture, followed by 50 mM AcOH.
An additional 4.06 g ZAsp(OBzl)OH (11.4 mmole) as solid matter were added to the solution, pH currently being readjusted to 7. Shortly after renewed product precipitation started, the product after a couple of days being filtered off and washed as described above, and by continuous concentration of the solution and prolonged standing and addition of further 20 mg thermolysin, a total degree of conversion of ZAsp(OBzl)OH of approx. 90% according to HPLC was obtained. The filter cake proceeded directly to the next step.
Yield according to HPLC: 90% (determined at 254 nm)

| Analysis: | | |
|---|---|---|
| AAA: | Asp: | 1.10 |
| | Val: | 1.03 |
| | Tyr: | 0.86 |

b) Synthesis of AspValTyrNH₂
Reaction scheme: 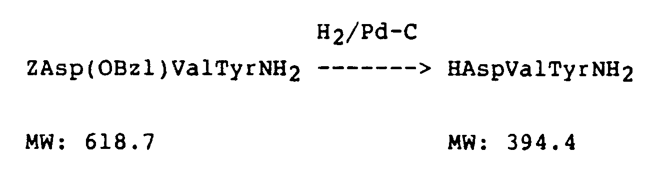 Procedure:
The filter cake from the previous step containing approx. 15.5 g (25 mmole) ZAsp(OBzl)ValTyrNH₂ was slurried in 175 ml DMF and 25 ml AcOH and some HCl-solution. Now 0.15 g 10% (w/w) palladium-on-coal slurried in 2 ml H₂O was added, hydrogenation was performed in a Parr-hydrogenation apparatus at a hydrogen pressure of 3 bar, until analytical HPLC showed that the reaction had ended, whereafter the catalyst was filtered off and washed and the solution evaporated.
Yield according to analytical HPLC: 98% (determined at 254 nm)

| Analysis: | | |
|---|---|---|
| AAA: | Asp: | 1.00 |
| | Val: | 1.02 |
| | Tyr: | 0.98 |

c) Synthesis of ZAspValTyrNH₂ with side chain unprotected starting substances
Reaction scheme: 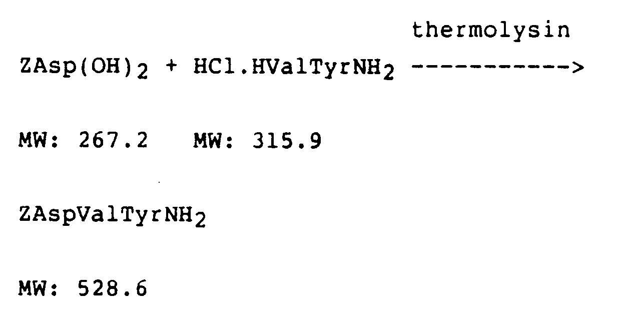 Procedure:
20.0 g HCl.HValTyrNH₂ (63 mmole) were dissolved in 190 ml H₂O and mixed with 0.61 g TRIS and 1.86 g CaCl₂.-2H₂O. pH was adjusted to 7 with NaOH-solution and maintained at that value, 8.45 g N-α-Z-Aspartic acid (32 mmole) being added. The volume was then adjusted with an additional 20 ml H₂O, and the reaction was initiated by adding 48 mg thermolysin dissolved in 2 ml H₂O. The mixture was left to stand with stirring at room temperature for 5 days, during which period the product precipitated. Conversion according to analytical HPLC: 85%.
The adsorbance ratio between abs. at 278 and abs. at 254 nm indicates that the product contains the chromophores Z and Tyr in the ratio 1:1.
Yield according to HPLC: 85% (determined at 254 nm)
Isolation of ZAspValTyrNH₂:
The aqueous reaction mixture with sediment was extracted with ethylacetate phases, whereafter the product was filtered off and washed with water. It was repeatedly evaporated from dry ethanol.
Yield: 1.35 g (80%)
By catalytic hydrogenation the product can be converted to AspValTyrNH₂.
4) Synthesis of ZArgLysAspValTyrNH₂.AcOH via fragment coupling
Reaction scheme: 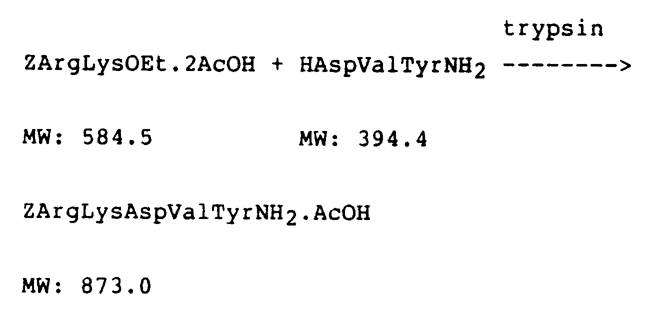 Procedure:
2.6 g guanidine hydrochloride were pulverized and mixed with 52 mg CaCl₂.6H₂O followed by 2.6 ml of a neutralized solution of 0.16 g TRIS in H₂O. Stirring continued until everything was in solution. A 6.6 ml solution of 2.21 g HAspValTyrNH₂ (5.6 mmole) in acid DMF was subsequently added, whereafter pH was adjusted to 8.5 with 12 N NaOH-solution. 1.49 g ZArgLysOEt.2AcOH (2.5 mmole) were then added with stirring. The reaction was initiated by adding 12 mg trypsin dissolved in 0.18 ml H₂O. The reaction was followed on analytical HPLC and after 1 1/2 hours it was more than 95% complete. pH was then adjusted to 2.0 with 10 N HCl, and the reaction mixture was then purified.
Yield according to HPLC: 50% (determined at 254 nm)
Isolation of ZArgLysAspValTyrNH₂
The reaction mixture was purified by RP-HPLC on C18-columns with alcohol, 50 mM AcOH-mixture as eluent. Fractions containing pure product were combined, evaporated and freeze dried.
Yield: 0.98 g (45%) (white, amorphous powder)

| Analysis: | | |
|---|---|---|
| AAA: | Asp: | 1.03 |
| | Arg: | 1.08 |
| | Val: | 1.00 |
| | Tyr: | 0.89 |
| | Lys: | 0.99 |

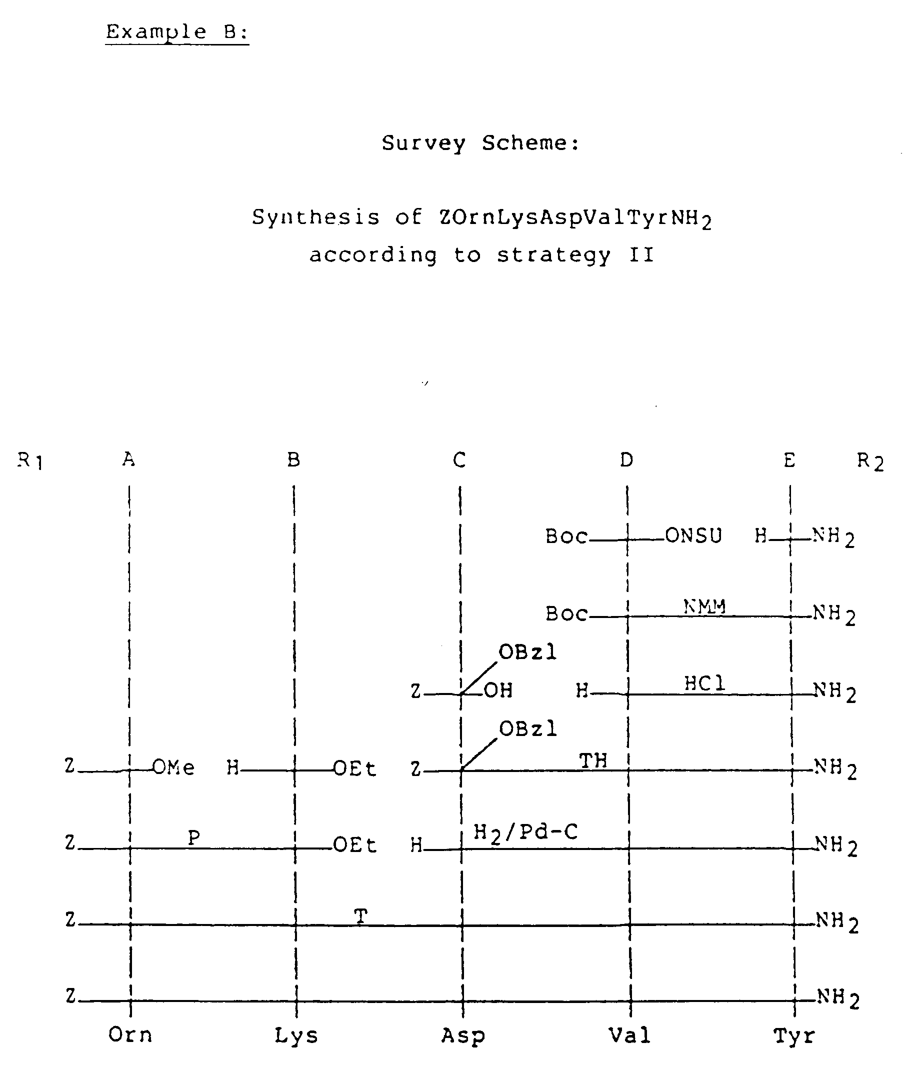
Example B:

Survey Scheme:

Synthesis of ZOrnLysAspValTyrNH2
according to strategy II


R1 A B C D E R2

Boc ONSU H NH2

Boc NMM NH2

OBzl
Z OH H HCl NH2

OBzl
Z OMe H OEt Z TH NH2

Z P OEt H H2/Pd-C NH2

Z T NH2

Z NH2

Orn Lys Asp Val Tyr

### Example B

### Synthesis of ZOrnLysAspValTyrNH₂ according to strategy II

1) Synthesis of ZOrnithin-methyl ester:
Reaction scheme: 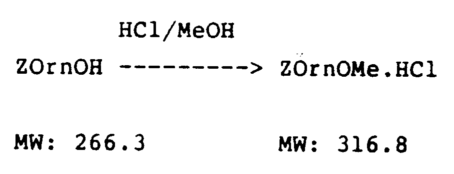 Procedure:
2.0 g Cbz-L-Ornithin (7.5 mmole) were mixed with 30 ml dry methanol and 16 ml 7.2 M dry HCl in methanol. The mixture was left to stand with stirring for 1 1/2 hours at room temperature and was followed by analytical HPLC. Evaporated to dryness and repeatedly evaporated from dry methanol (vac. < 40 °C).
Yield: 2.4 g (100%) (amorphous)
2) Synthesis of ZOrnLysOet.2AcOH
Reaction Scheme: 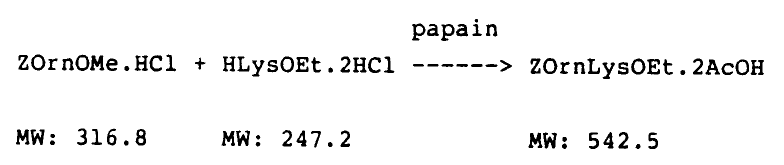 Procedure:
8.2 g lysine ethylester-dihydrochloride (33 mmole) were dissolved in 19 ml H₂O and 10 ml methanol and pH was adjusted to 7.5 with 3 ml 6 N NaOH. 1.3 ml 0.1 M EDTA (Na-salt)and 1 ml 1 M β-mercapto ethanol were added. 2.1 g Z-L-Ornithin-methylester (6.6 mmole) dissolved in 16.4 methanol were then added.
pH was now readjusted to 7.5 with 6 N NaOH, the volume was adjusted to 66 ml with H₂O, and the reaction initiated by adding 0.24 ml of a 20 mg/ml papain-solution preactivated in 0.3 M β-mercapto ethanol for 40 min at 40 °C. The reaction was followed by analytical HPLC. After approx. 40 min the substrate was more than 95% converted, and the reaction was discontinued by adjusting pH to 3.0 with 10 N HCl.
Yield compared to hydrolysis according to analytical HPLC: 90% (254 nm)
The mixture was then purified.
Purification of ZOrnLysOEt.2AcOH
The reaction mixture was evaporated for methanol in vacuum and diluted with H₂O. It was subsequently placed on a RP-HPLC C18-column, preequilibrated with 50 mM AcOH, pH 3.0. Elution was with successive column volumes of alcohol/H₂O, 50 mM AcOH and increasing alcohol content.
The product fractions containing the product were combined, evaporated to dryness and freeze dried.
Yield: 2.3 g (64%) (white, amorphous powder)

| Analysis: | | |
|---|---|---|
| AAA: | Lys: | 1.00 |
| | Orn: | 1.00 |

3) Synthesis of ZOrnLysAspValTyrNH₂
Starting substances: ZOrnLysOEt.2AcOH as synthesized according to example B,2) H-AspValTyrNH₂ in 0.9 M solution as synthesized according to example A,3).
Reaction scheme: 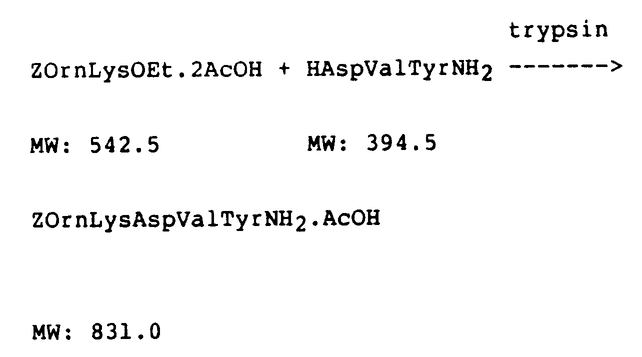 Procedure:
2.34 g guanidine hydrochloride were mixed with 32 mg CaCl₂.6H₂O and 2.24 ml 0.5 M TRIS-solution and 5.6 ml acid DMF-solution containing 1.9 g AspValTyrNH₂ (4.8 mmole). pH was adjusted (quickly) to 8.5 with 1.9 ml 12 N NaOH. 1.18 g ZOrnLysOEt.2AcOH (2.2 mmole) were added as a powder, and the reaction was initiated by adding 11 mg trypsin in 0.2 ml 0.1 M CaCl₂-solution. The reaction was followed by analytical HPLC. After 45 min. an additional 0.4 g ZOrnLysOEt (0.7 mmole) and further 11 mg trypsin in 0.2 ml 0.1 M CaCl₂ solution were added. After 1 1/2 hours the reaction was complete, and pH was reduced to 2.3 with 2.3 ml 10 N HCl.
Synthesis compared to hydrolysis according to quantization by HPLC (UV: 254 nm): 72%.
Purification of ZOrnLysAspValTyrNH₂:
The reaction mixture was combined with a similar one based on 0.15 g ZOrnLysOEt.2AcOH (0.3 mmole), i.e. a total of 1.73 g ZOrnLysOEt. The mixture was diluted and placed on a RP-HPLC C18-column, preequilibrated with 50 mM AcOH. Elution was with alcohol/water, the 50 mM AcOH-mixture with increasing alcohol content.
The product fractions were evaporated and freeze dried.
Yield: 1.23 g (56%) (white, amorphous powder)
Analysis of the product obtained gave the following result:

| | | |
|---|---|---|
| AAA: | Asp: | 1.08 |
| | Tyr: | 0.94 |
| | Val: | 0.98 |
| | Lys: | 0.95 |
| | Orn: | 1.06 |

No foreign or free amino acids.

| | |
|---|---|
| UV-maximum: | 275 nm (UV 293 nm in 0.1 N NaOH) |
| Acetate content: | 8.3% (HPLC) |
| Chloride content: | < 0.05% (Potentiometric) |
| Water content: | 5.5% (Karl Fischer) |

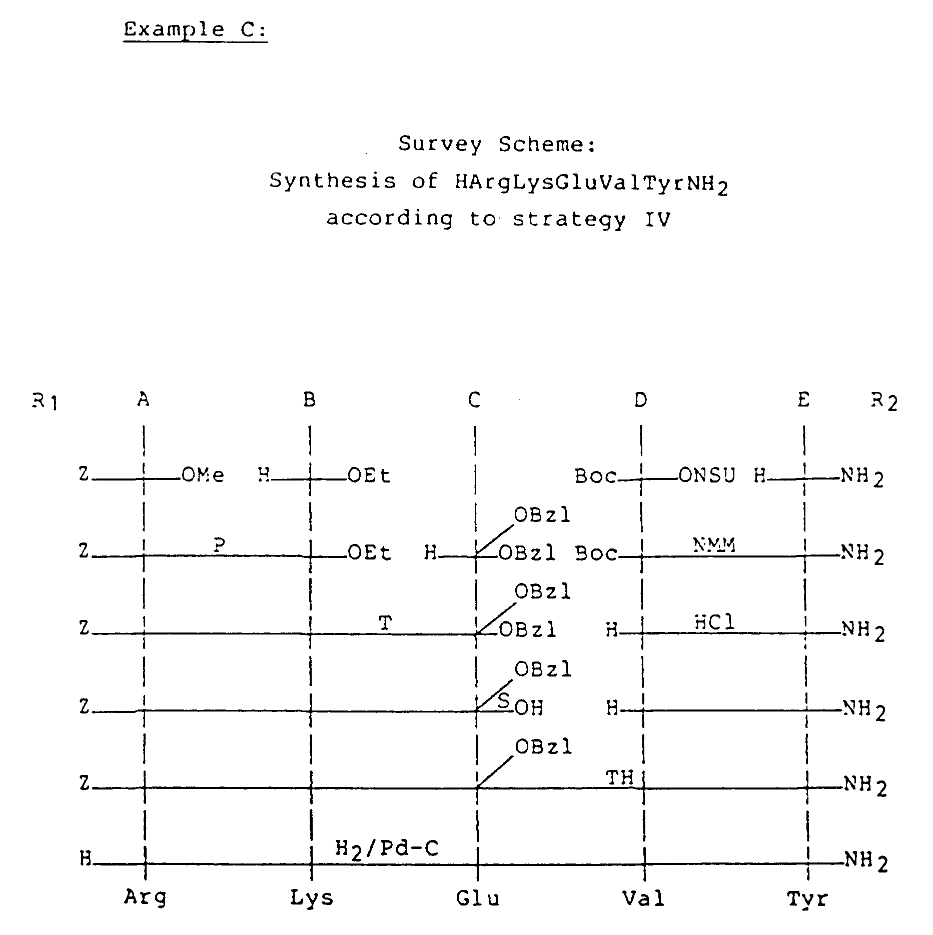
Example C:

Survey Scheme:
Synthesis of HArgLysGluValTyrNH2
according to strategy IV


R1 A B C D E R2

Z OMe H OEt Boc ONSU H NH2

OBzl
Z P OEt H OBzl Boc NMM NH2

OBzl
Z T OBzl H HCl NH2

OBzl
Z S OH H NH2

OBzl
Z TH NH2

H H2/Pd-C NH2

Arg Lys Glu Val Tyr

### Example C:

### Synthesis of HArgLysGluValTyrNH₂ (Spleninopentin-α-amide) according to strategy IV

1) Synthesis of ZArgLysOEt.2AcOH
(Synthesized as described in example A,1)
2) Synthesis of ZArgLysGlu(OBzl)₂
Reaction scheme: 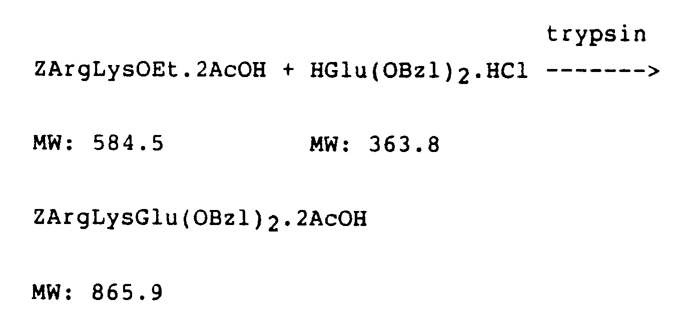 Procedure:
7.63 g ZArgLysOEt.2AcOH (13.0 mmole) and 7.9 g HCl.HGlu(OBzl)₂ (21.7 mmole) were dissolved in a mixture of 27 ml methanol and 11 ml H₂O. pH was adjusted to 8.0 with aqueous NaOH and the reaction was initiated by adding 2 mg trypsin (Bovine) dissolved in 0.15 ml 0.1 M CaCl₂. The reaction mixture was now left to stand with stirring for 70 min at room temperature, pH being held constant at 8.0 with aqueous NaOH, and was followed on analytical HPLC. The conversion was then complete, and the reaction was discontinued by adjusting pH to 3 with 6 N HCl, whereafter the mixture was purified.
Purification of ZArgLysGlu(OBzl)₂:
The reaction mixture was placed on a cation exchange column, elution was with a salt gradient with increasing amounts of ammonium acetate. The product fractions hereby eluted were evaporated for solvent and diluted to a clear solution with acetic acid, the solution subsequently being placed on a RP-HPLC C18-column and purified with increasing alcohol content in 50 mM AcOH. In respect of the eludated product fractions analytical HPLC showed a homogeneous, pure product.
Yield: 4,2 g (37%)

| Analysis: | | |
|---|---|---|
| AAA: | Glu: | 1.01 |
| | Arg: | 1.05 |
| | Lys: | 0.94 |

3) Special characterization of the ArgLysGlu-peptide
Reaction Scheme 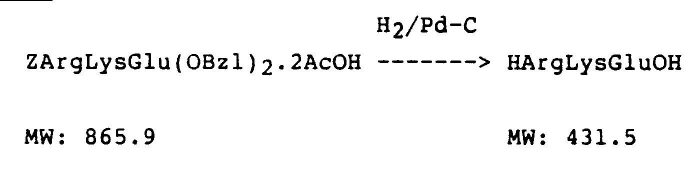 Procedure:
4.2 g ZArgLysGlu(OBzl)₂ (4.8 mmole), synthesized according to the above method, were dissolved in an acetic ethanol/water mixture and mixed with 10% w/w 5% palladium on carbon, followed by hydrogenation overnigh at a hydrogen pressure of 3.0 bar in a Parr-hydrogenation apparatus. The mixture was subsequently filtered.
Purification of HArgLysGluOH:
The above solution was placed on a cation exchange column, which was then eluted with NH₄Ac-solution. The product fractions were combined and desalted. After evaporation and freeze drying a white, amorphous powder was obtained.
Yield: 2.0 g (96%; corrected for water and acetate)
This product is characterized in the following analysis results:

| Specific rotation α_{D}²⁵ -4,3° (C=1.5% (w/w) in water) | |
|---|---|
| Acetate content: | 0.2% (HPLC) |
| Chloride content: | < 50 ppm (AgNO₃-precipitation) |
| Water content: | 7.9% (Karl Fisher) |
| Perchloric acid titration: | 88.2% product content |
| Foreign or free amino acids: | 0.7% Phe |
| AAA: | Glu: 0.99 |
| | Arg: 1.09 |
| | Lys: 0.92 |
| HPLC purity: | 99.8% (220 nm) |
| Sequence analysis: | Arg, Lys, Glu = 1 2 3 |

4) Synthesis of HCl. HValTyrNH₂:
Synthesized as described in example A,2).
5) Synthesis of ZArgLysGlu(OBzl)ValTyrNH₂:
a) ZArgLysGlu(OBzl)OH-synthesis
   Reaction scheme: 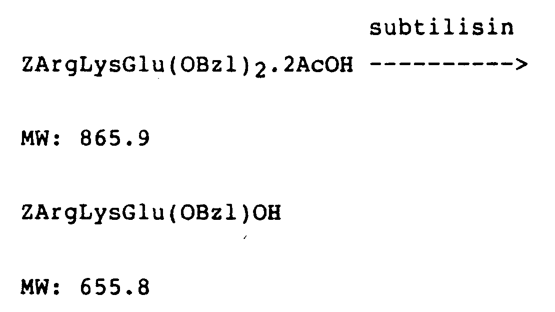 Procedure:
   1.08 g ZArgLysGlu(OBzl)₂.2AcOH (1.2 mmole) and 0.19 g potassium chloride were dissolved in 12.5 ml 0.2 M TRIS and 10 ml ethanol. pH was adjusted to 8.0 with 2 N NaOH. The reaction was initiated by addition of 0.3 ml of a 5 mg/ml subtilisin A-solution. The reaction was left to stand with stirring for 100 min. at room temperature and was monitored on analytical HPLC. During the course, the α-benzyl ester was selectively hydrolysed on the glutamic acid. The enzyme was then inactivated by lowering pH to 3 with HCl-solution.
   Yield according to HPLC: 95% (determined at 254 nm)
b) Synthesis of ZArgLysGlu(OBzl)ValTyrNH₂
   Reaction scheme: 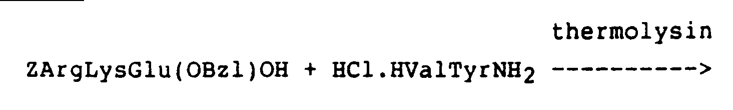 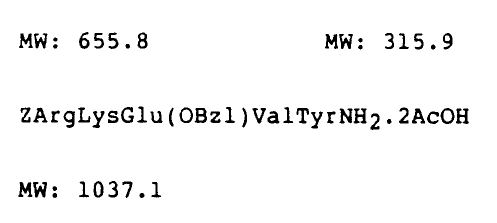 Procedure:
   The ZArgLysGlu(OBzl)OH solution from the previous step was evaporated to 6 ml, hereby removing the organic solvent, and 0.97 g HCl.HValTyrNH₂ (3.1 mmole) and 0.1 g CaCl₂.2H₂O were added. Dilution to 12 ml to a thin suspension, pH being adjusted to 7. The reaction was initiated with 15 mg thermolysin added as a solid. After 1 hour remaining precipitates were dissolved, and the product precipitation started. Standing overnight at room temperature with stirring. The reaction was followed by analytical HPLC. The product precipitated almost quantatively and was filtered off after pH adjustment to 4.
   Yield: about 90%

6) Synthesis of HArgLysGluValTyrNH₂:
1.0 g saline matter from the previous step was dissolved in 600 ml 60% EtOH, 1 ml AcOH and 0.1 10% w/w palladium on carbon were added, followed by hydrogenation for some hours in a Parr-hydrogenation apparatus at a hydrogen pressure of 3.0 bar. The solution was subsequently filtered. The filtrate was evaporated and the remainder analyzed. The result was as follows:

| Analysis: | | |
|---|---|---|
| AAA: | Glu: | 0.97 |
| | Arg: | 1.06 |
| | Tyr: | 1.04 |
| | Val: | 1.03 |
| | Lys: | 0.91 |

| | |
|---|---|
| Foreign or free amino acids: | < 0.1% (AAA) |
| HPLC-purity: | 95.6% (220 nm) |

Chloride was detected.

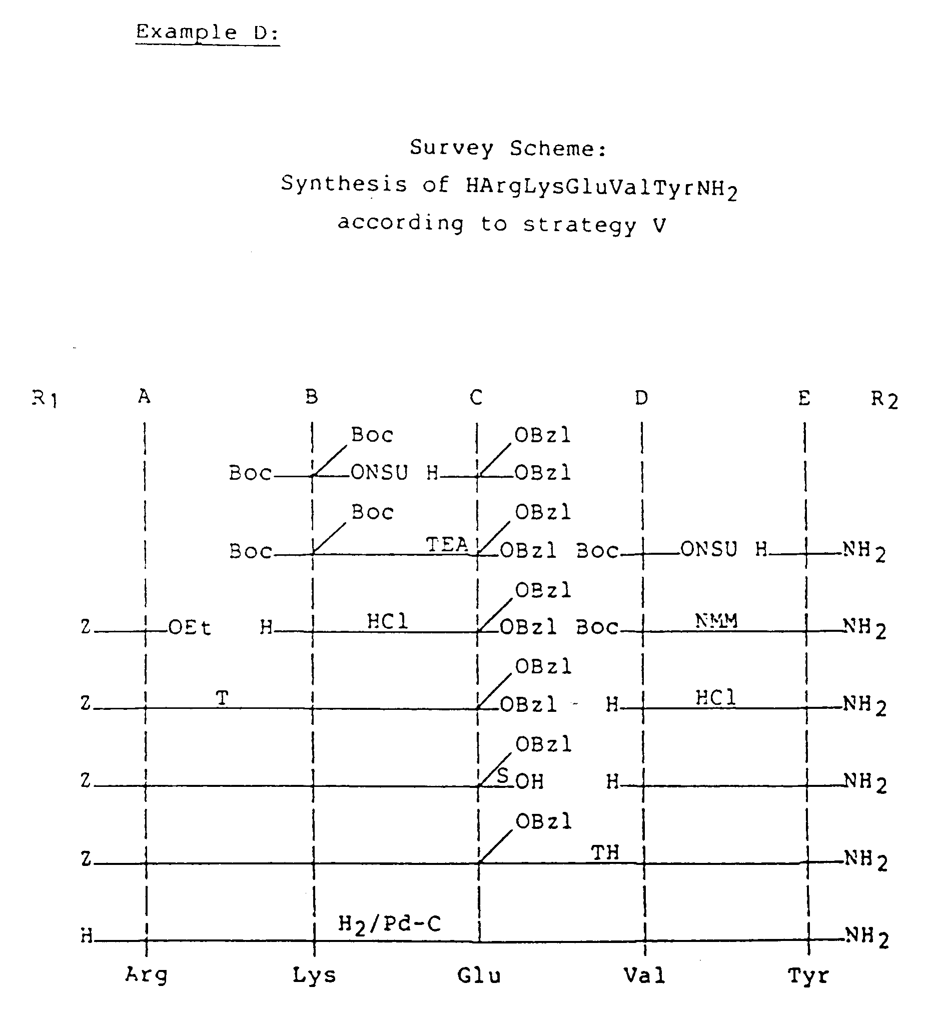
Example D:

Survey Scheme:
Synthesis of HArgLysGluValTyrNH2
according to strategy V

R1 A B C D E R2

Boc OBzl
Boc ONSU H OBzl

Boc OBzl
Boc TEA OBzl Boc ONSU H NH2

OBzl
Z OEt H HCl OBzl Boc NMM NH2

OBzl
Z T OBzl H HCl NH2

OBzl
Z S OH H NH2

OBzl
Z TH NH2

H H2/Pd-C NH2

Arg Lys Glu Val Tyr


### Example D:

### Synthesis of HArgLysGluValTyrNH₂ (Spleninopentin-α-amide) according to strategy V

1) Synthesis of Boc₂LysGlu(OBzl)₂:
Reaction scheme: 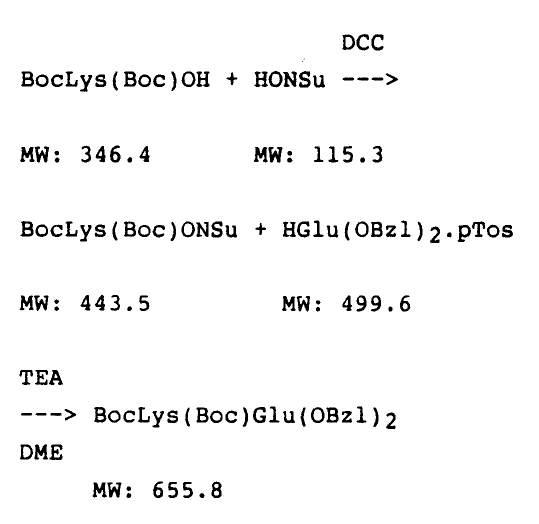 Procedure:
9.0 g BocLys(Boc)OH (26 mmole) and 3.3 g n-hydroxy succinic imide (28.6 mmole) were dissolved in 50 ml DME and cooled in ice bath. The reaction was initiated by adding 5.9 g dicyclohexyl carbodiimide (28.6 mmole) dissolved in 40 ml cold DME. After 2 hours it was checked on analytical HPLC that the conversion was complete. 13.5 g H-Glu(OBzl)₂ p-tosylate (22 mmole) dissolved in 60 ml DME and 3.74 ml triethyl amine (22 mmole) were subsequently added. The mixture was left to stand overnight at room temperature. Then an additional 2.7 g H-Glu(OBzl)₂ p-tosylate and and equivalent amounts of TEA were added, and the mixture was left to stand for another 3 hours, whereafter it was filtered, evaporated and redissolved in EtOAc. The ethyl acetate phase was washed with natrium bicarbonate and citrate acid solution and NaCl-solution, dried with MgSO₄, filtered and evaporated.
2) Synthesis of 2HCl.LysGlu(OBzl)₂:
Reaction Scheme: 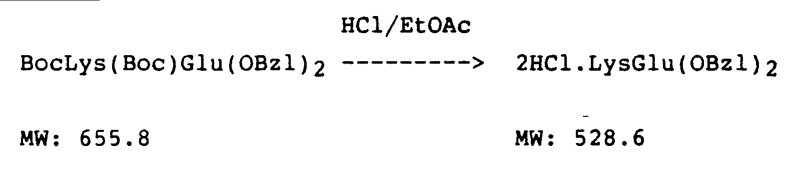 Procedure:
50 ml of a solution of dry 4M HCl in EtOAc were added to the remainder from the above synthesis. The mixture was left to stand for 45 min with stirring at room temperature, the mixture was then evaporated and precipitated from EtOAc/petrolether. The product was filtered off, dissolved in MeOH and purified by preparative HPLC on RP-HPLC C18 columns with a linear elution gradient with increasing amounts of alcohol in 50 mM AcOH/H₂O.
The product fractions were combined and evaporated.
Yield: 9.0 g (65%)
3) Synthesis of ZArgLysGlu(OBzl)₂.2AcOH:
Reaction scheme: 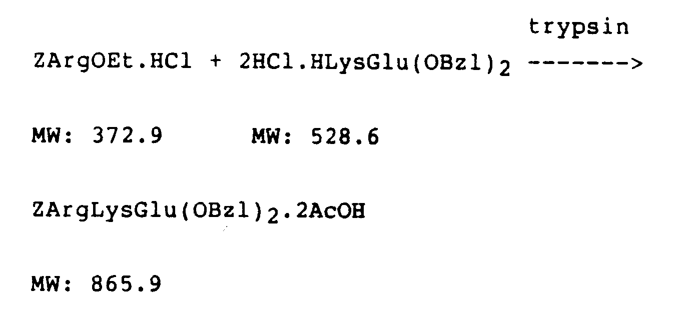 Procedure:
21 g LysGlu(OBzl)₂.2HCl (39.7 mmole) and 11.1 g ZArgOEtHCl (29.8 mmole) were dissolved in 60 ml DME + 139 ml H₂O. pH was adjusted to 8.0 with TEA and the reaction was initiated by adding 0.2 ml of a 1 mM (Bovine) trypsin solution. The reaction was followed by analytical HPLC. After 2 hours the reaction was discontinued by lowering pH to 3 with 6 N HCl when all substrate had been converted.
Purification of ZArgLysGlu(OBzl)₂.2AcOH
The mixture was filtered and placed on RP-HPLC C18-columns. Elution was then with increasing concentration of alcohol in 50 mM AcOH resulting in isolation of the product as a pure product. The product fractions were combined and evaporated.
Yield: 7.7 g (30%)

| Analysis: | | |
|---|---|---|
| AAA: | Glu: | 1.09 |
| | Arg: | 0.95 |
| | Lys: | 0.96 |

4) Special characterization of the ArgLysGlu peptide
Reaction scheme: 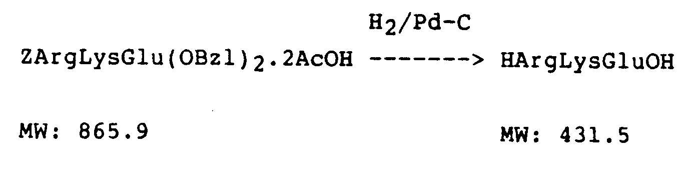 Procedure:
52.0 g ZArgLysGlu(OBzl)₂ (60 mmole), synthesized according to the above procedure, were dissolved in an acetic ethanol/water mixture and mixed with 10% w/w 5% palladium-on-charcoal, followed by hydrogenation overnight at a hydrogen pressure of 3.0 bar in a Parr-hydrogenation apparatus. The mixture was subsequently filtered.
Purification of HArgLysGluOH:
The above solution was placed on a cation exchange column which was then eluted with NH₄Ac-solution. The product fractions were combined and desalted. After evaporation and freeze drying a white, amorphous powder was obtained.
Yield 26.9 g (95%; corrected for water and acetate)
This product is characterized by the following analysis results.

| Analysis: | | |
|---|---|---|
| AAA: | Glu: | 0.96 |
| | Arg: | 1.06 |
| | Lys: | 0.99 |

| | |
|---|---|
| Foreign or free amino acids: | Not found (AAA) |
| Specific rotation | α_{D}²⁵: -5.1° (C=1.5% (w/w) in H₂O₎ |
| Acetate content: | 4.4% (HPLC) |
| Chloride content: | <50 ppm (AgNO₃-precipitation) |
| Water content: | 7.6% (Karl Fischer) |
| Perchloric acid titration: | 91.4% product content |
| HPLC purity: | 99.1% (220 nm) |
| TLC: | Rf.: 0.71 (cellulosise eluted with isopropanol/ammonia water 1:1) |
| Sequence analysis: | Arg, Lys, Glu = 1 2 3 |

5) Synthesis of ZArgLysGlu(OBzl)OH, HClValTyrNH₂, ZArgLysGlu(OBzl)ValTyrNH₂ and H.ArgLysGluValTyrNH₂
Synthesis as described in example C,4-6)

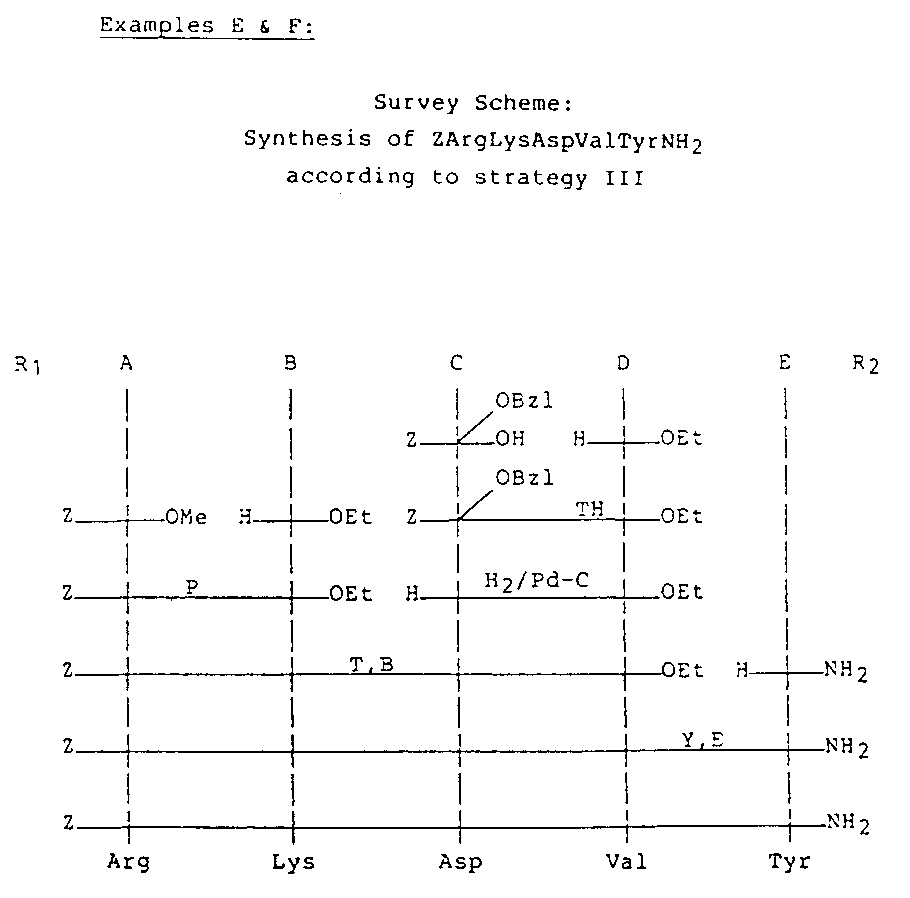
Examples E & F:

Survey Scheme:
Synthesis of ZArgLysAspValTyrNH2
according to strategy III

R1 A B C D E R2

OBzl
Z OH H OEt

OBzl
Z OMe H OEt Z TH OEt

Z P OEt H H2/Pd-C OEt

Z T,B OEt H NH2

Z Y,E NH2

Z NH2

Arg Lys Asp Val Tyr


### Example E:

### Synthesis of ZArgLysAspValTyrNH₂ (Z-Thymopeptin amide) with CPD-Y catalyzis according to strategy III

1) Synthesis of ZArgLysOEt.2AcOH
Synthesis according to example A,1)
2) Synthesis of ZAsp(OBzl)ValOEt
Reaction scheme: 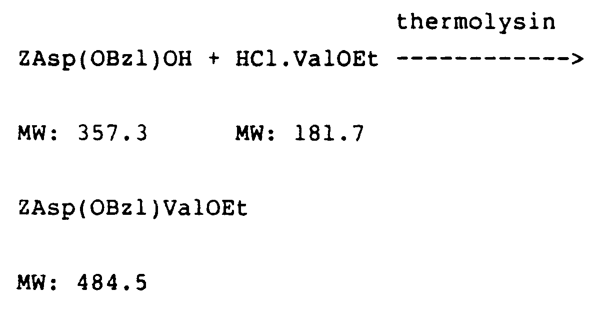 Procedure:
45.5 g HValOEt.HCl (250 mmole) were dissolved in 50 ml DMF and 390 ml H₂O mixed with TRIS and 7.6 g CaCl₂.2H₂O (50 mmole). pH was adjusted to 7 with approx. 15 ml NaOH-solution. 35.4 g ZAsp(OBzl)OH (99 mmole) dissolved in 200 ml DMF were added, and pH readjusted to 7 with approx. 20 ml 12 N NaOH. The reaction was initiated by adding 1.1 g thermolysin suspended in 250 ml H₂O. The product precipitation initiated after 15 min, and the reaction was left to stand with vigorous stirring for 3 days, whereafter the product could be filtered off, washed with H₂O and dissolved in an ethyl acetate which was washed with water phases, evaporated to dryness, washed with EtOH/water and evaporated with dry ethanol and methanol.
Yield: 47 g (98%) oil (Ninhydrin negative)
3) Synthesis of HAspValOEt.AcOH
Reaction scheme: 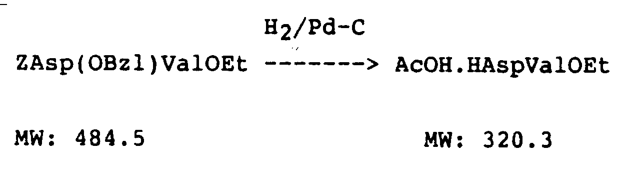 Procedure:
47 g (97 mmole) ZAsp(OBzl)ValOEt were dissolved in 1250 ml ethanol and mixed with 250 ml H₂O and 12 ml AcOH (212 mmole), followed by 4.7 g 5% (w/w) palladium-on-charcoal slurried in H₂O. The mixture was then hydrogenated overnight at a hydrogen pressure of 3 bar in a Parr-hydrogenation apparatus. The catalyst was subsequently filtered off, and the filtrate, for which analytical HPLC indicated more than 99% conversion, was repeatedly evaporated from H₂O.
Yield: 31 g (100%)

| Analysis: | | |
|---|---|---|
| AAA: | Asp: | 1.00 |
| | Val: | 1.00 |

4) Synthesis of ZArgLysAspValOEt.AcOH
a) With trypsin catalysis
   Reaction scheme: 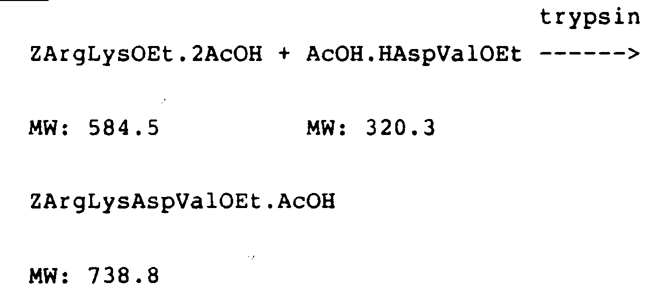 Procedure:
   30 g AcOH.HAspValOEt (94 mmole) were slurried in 61 ml DMF and 27 ml 0.25 M TRIS, 0.05 M CaCl₂ and 8.5 ml 10 N HCl. When everything was in solution an additional 5 ml 50% DMF were added, and pH adjusted to 8.5 with approx. 14 ml 12N NaOH. 12.0 g ZArgLysOEt.2AcOH (21 mmole) were then added as solid matter, and pH was readjusted to 8.5. The reaction was then initiated by adding 12.7 mg trypsin dissolved in 1.8 ml H₂O. The reaction was followed by analytical HPLC. After 50 min approx. 70% had been converted, and after 90 min. an additional 11.9 g (20 mmole) ZArgLysOEt.2AcOH were added as solid matter and an additional 12,7 mg trypsin, after 150 min. an additional 12.5 g (21 mmole) ZArgLysOEt.2AcOH were added as solid matter and after another 30 min. 35 mg trypsin dissolved in H₂O/DMF. After 4 1/2 hours 95% of the substrate had been converted, and pH was adjusted to 3.5 with 10 N HCl. The mixture was then purified.
   Yield according to HPLC: 65% (determined at 254 nm)
b) With bromelain catalysis
   Reaction scheme: 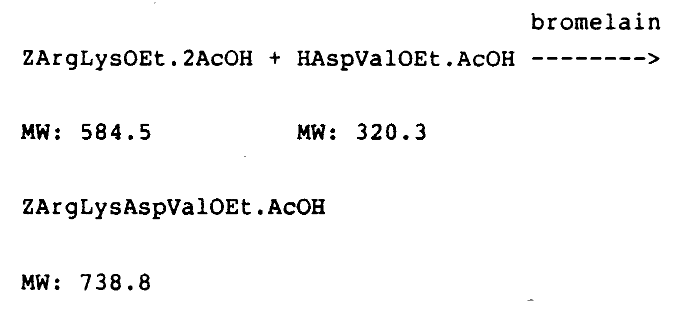 Procedure:
   0.26 g AspValOEt.AcOH (0.8 mmole) were slurried in 0.7 ml DMF and 0.8 »l H₂O and a little HCl-solution. When everything was in solution 0.088 ml 0.1 M EDTA was added, and pH adjusted to 8.5 with NaOH-solution. 117 mg ZArgLysOEt.2AcOH (0.2 mmol) were added as a powder, and pH was readjusted to 8.5. Then 20 »l 1 M BME and 0.1 ml H₂O were added, and the reaction was initiated with 6 »l solution of 10 mg/ml bromelain, preactivated at 35 °C with BME. Reaction time approx. 2 hours.
   Yield according to HPLC: 63% (determined at 254 nm)

5) Purification of ZArgLysAspValOet.AcOH
The reaction mixture from the previous step was diluted with H₂O and purified by RP-HPLC C18-columns. Elution was done with 50 mM AcOH followed by a gradient with increasing content of alcohol in 50 nM AcOH. Fractions containing purified product were combined, evaporated and freeze dried. The result was a white, amorphous powder.
Isolated yield: 27.3 g (60%)
Purity according to HPLC (254 nm): 98.3%

| Analysis: | | |
|---|---|---|
| AAA: | Asp: | 1.00 |
| | Arg: | 1.05 |
| | Val: | 1.06 |
| | Lys: | 0.95 |

6) Synthesis of ZArgLysAspValTyrNH₂.AcOH
Reaction scheme: 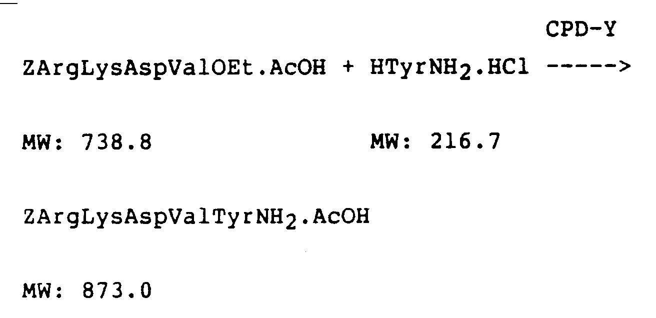 Procedure:
17.3 g HTyrNH₂.HCl (80 mmole) were mixed with 30 ml DMF, 80 ml of a 0.5 M neutralized TRIS-solution and 8 ml of a 0.1 M EDTA-solution. pH was adjusted to 8.8 with approx. 7 ml 6 N NaOH. The clear solution was mixed with 6.8 g ZArgLysAspValOEt.AcOH (9.2 mmole) and additionally 72 ml H₂O were added and pH readjusted to 8.8. The reaction was then initiated by adding 15 ml of a 18 mg/ml CPD-Y solution. The reaction was followed by analytical HPLC. After 20 hours, 85% of the substrate had been converted. After standing, the product deamidated in situ.
Yield according to HPLC: 62% (determined by 254 nm and corrected for unconverted substrate).

| Analysis: | | |
|---|---|---|
| AAA: | Asp: | 1.16 |
| | Arg: | 1.09 |
| | Tyr: | 0.78 |
| | Val: | 1.00 |
| | Lys: | 0.97 |

### Example F:

### Synthesis of ZArgLysAspValTyrNH₂ with elastase catalysis according to strategy III

1) Synthesis of ZArgLysAspValOEt.AcOH
Synthesis as described in example E,1-5.
2) Synthesis of ZArgLysAspValTyrNH₂
Reaction scheme: 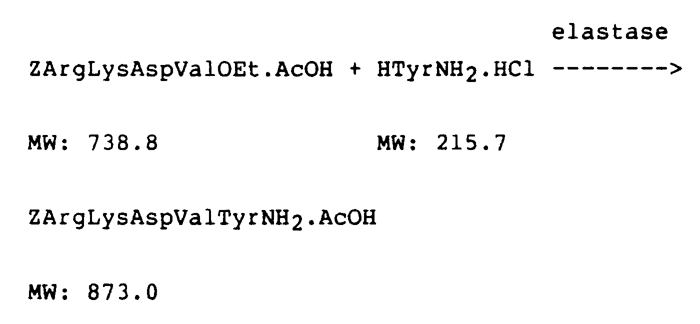 Procedure:
4.6 g HTyrNH2.HCl (21 mmole) were mixed with 4 ml DMF, 8 ml of a 0.5 M TRIS solution and 0.15 g KCl (2 mmole). pH was adjusted to 8.5 with 2.5 ml 6 N NaOH. The clear solution was mixed with 0.68 g ZArgLysAspValOEt.AcOH (0.92 mmol), and an additional 2.5 ml H₂O were added and pH readjusted to 8.5 with NaOH-solution. The reaction was then initiated by adding 3.1 ml of a 17 mg/ml elastase solution. The reaction was followed by analytical HPLC. In less than 20 hours all substrate had been converted.
Yield according to HPLC: 21% (determined at 254 nm)

| Analysis: | | |
|---|---|---|
| AAA: | Asp: | 1.16 |
| | Arg: | 1.20 |
| | Tyr: | 0.77 |
| | Val: | 0.83 |
| | Lys: | 1.05 |

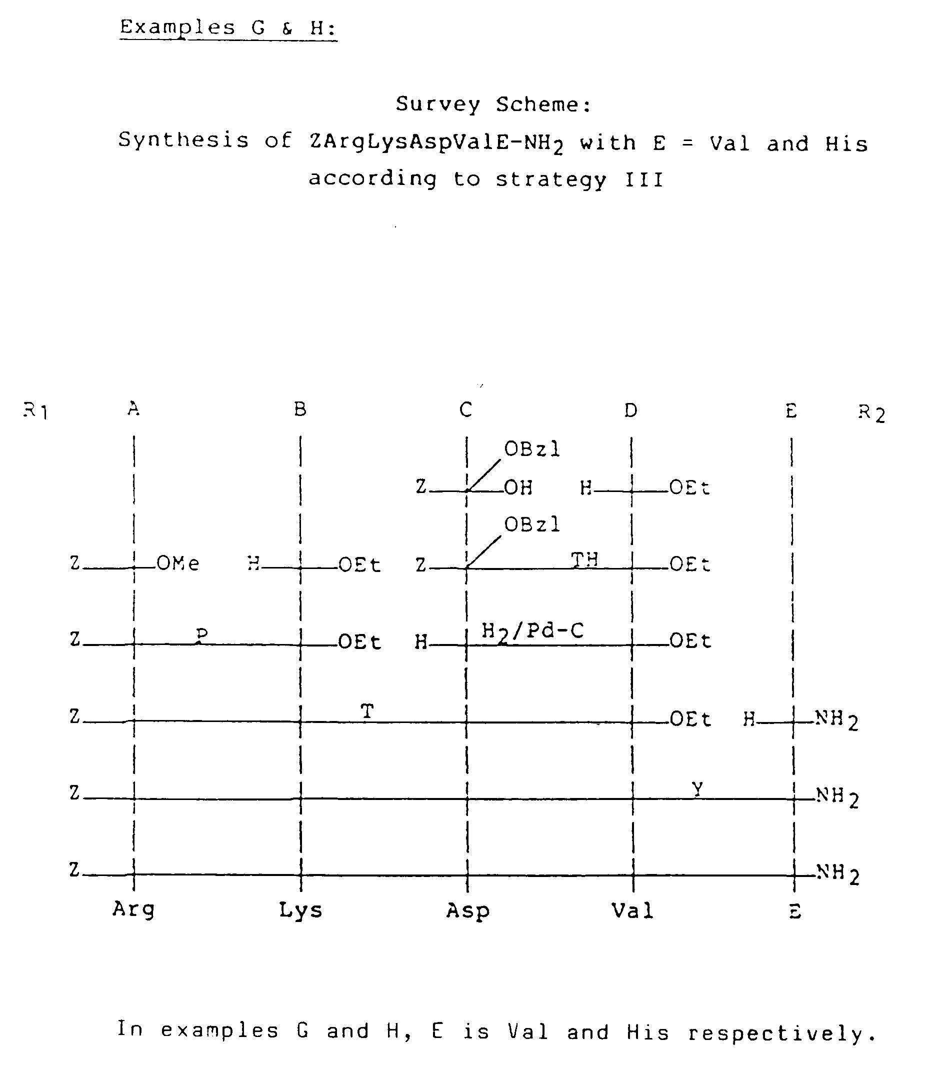
Examples G & H:

Survey Scheme:
Synthesis of ZArgLysAspValE-NH2 with E = Val and His
according to strategy III

R1 A B C D E R2

OBzl
Z OH H OEt
OBzl
Z OMe H OEt Z TH OEt
Z P OEt H H2/Pd-C OEt
Z T OEt H NH2
Z Y NH2
Z NH2
Arg Lys Asp Val E


In examples G and H, E is Val and His respectively.

### Example G:

### Synthesis of ZArgLysAspValValNH₂ according to strategy III

1) Synthesis of ZArgLysAspValOEt.AcOH
Synthesized according to Example E,1-5)
2) Synthesis of ZArgLysAspValValNH₂
Reaction Scheme: 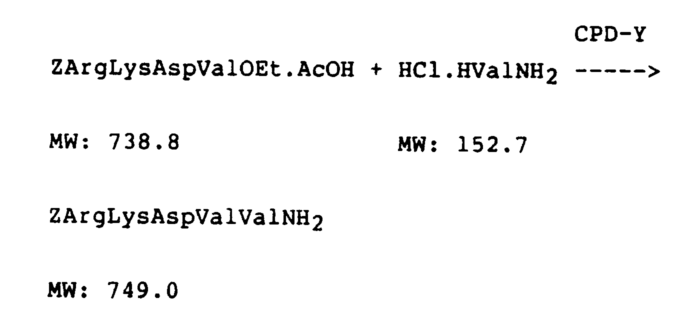 Procedure:
93 mg HCl.HValNH₂ (0.6 mmol) were mixed with 0.3 ml DMF and 0.8 ml 0.5 M TRIS and 0.08 ml 0.1 M EDTA. pH was adjusted to 8.9 with NaOH-solution. 70 mg ZArgLysAspValOEt.AcOH (0.1 mmol) were added to the solution. pH was readjusted to 8.9 and 0.74 ml H₂O was added. The reaction was initiated by adding 0.15 ml of a 18 mg/ml CPD-Y solution. Reaction time 20 hours at room temperature with stirring.
Yield according to HPLC: 40% (determined at 254 nm)

| Analysis: | | |
|---|---|---|
| AAA: | Asp: | 0.99 |
| | Arg: | 1.02 |
| | Val: | 1.97 |
| | Lys: | 1.02 |

### Example H:

### Synthesis of ZArgLysAspValHisNH₂ according to strategy III

1) Synthesis of ZArgLysAspValOEt.AcOH
Synthesis as described in example E,1-5)
2) Synthesis of ZArgLysAspValHisNH₂
Reaction scheme: 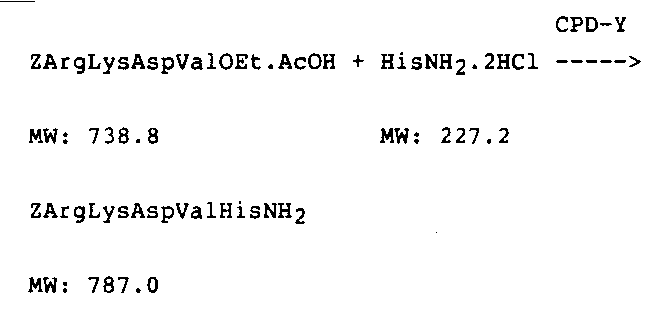 Procedure:
181 mg H-HisNH₂.2HCl (0.8 mmol) were mixed with 0.3 ml DMF, 0.8 ml 0.5 M TRIS and 0.08 ml 0.1 M EDTA. pH was adjusted to 8.8 with NaOH-solution. 68 mg ZArgLysAspValOEt.AcOH (0.09 mmol) were added to the solution, and pH was readjusted to 8.8. The volume was adjusted with 0.72 ml H₂O. The reaction was initiated by adding 0.2 ml of a 19 mg/ml CPD-Y solution. Followed by HPLC. Reaction time 6 hours at room temperature with stirring; result, 75% conversion.
After standing, deamidation takes place in situ.

| Analysis: | | |
|---|---|---|
| AAA: | Asp: | 1.05 |
| | Arg: | 1.05 |
| | Val: | 0.95 |
| | His: | 1.02 |
| | Lys: | 0.93 |

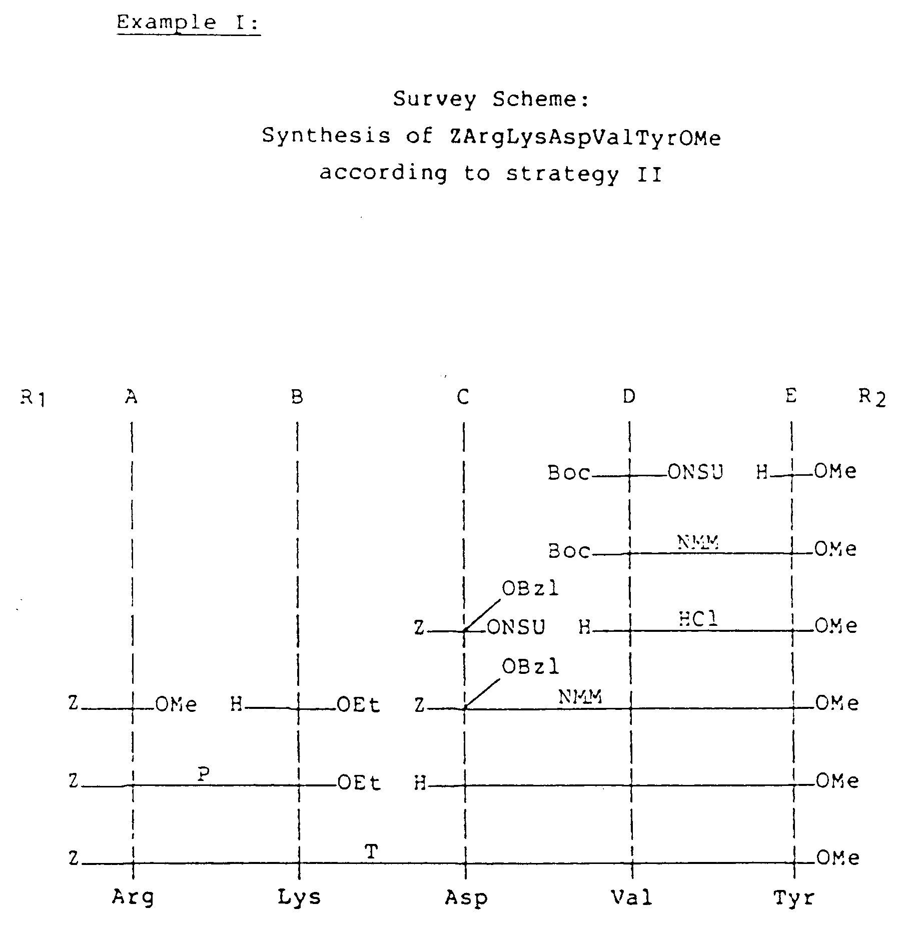
Example I:

Survey Scheme:
Synthesis of ZArgLysAspValTyrOMe
according to strategy II

R1 A B C D E R2

Boc ONSU H OMe

Boc NMM OMe

OBzl
Z ONSU H HCl OMe

OBzl
Z OMe H OEt Z NMM OMe

Z P OEt H OMe

Z T OMe

Arg Lys Asp Val Tyr


### Example I:

### Synthesis of ZArgLysAspValTyrOMe (Z-Thymopentin-α-methylester) according to strategy II

1) Synthesis of ZArgLysOEt.2AcOH
Synthesis as described in example A,2)
2) Synthesis of BocValTyrOMe
Reaction chart: 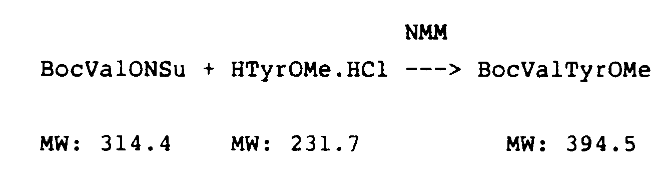 Procedure:
4.27 g HTyrOMe.HCl (18 mmole) were dissolved in 30 ml DMF and mixed with 2.5 ml triethlamine (18 mmole), whereafter 3.28 g (10 mmol) BocValONSu (synthesized according to example A,2) dissolved in 25 ml DMF were added. The reaction mixture was left to stand overnight at room temperature with stirring, whereafter the reaction mixture was evaporated, redissolved and purified by preparative RP-HPLC on C18-columns which were subsequently eluted with 50 nM AcOH gradient with increasing alcohol content in 50 nM AcOH. The product fractions were combined, evaporated and reevaporated from ethanol.
Isolated Yield: 3.74 g (95%)
3) Synthesis of HValTyrOMe.HCl
Reaction scheme: 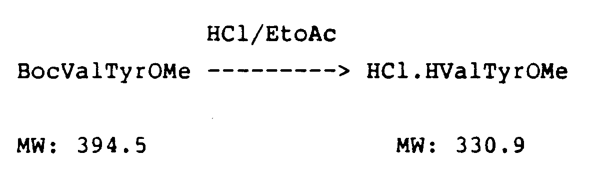 Procedure:
3.68 g BocValTyrOMe (9.3 mmole) were dissolved in 105 ml dry ethyl acetate mixed with 70 ml 2.7 N dry HCl in ethyl acetate. The mixture was left to stand for 3 hours at room temperature with stirring, and was subsequently evaporated to dryness and repeatedly from EtOAc (according to HPLC approx. 95% conversion).
Yield: 3.1 g (100%)
4) Synthesis of ZAsp(OBzl)ValTyrOMe
Reaction scheme: 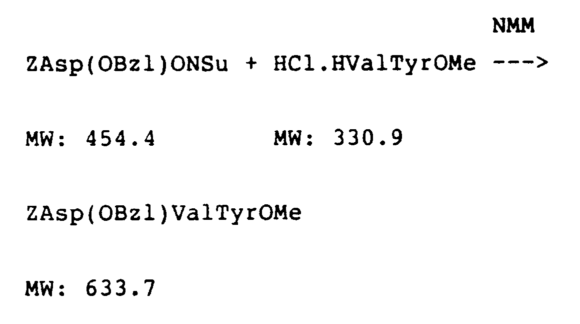 Procedure
2.3 g HCl.HValTyrOMe (7.0 mmole) were mixed with 2.0 g ZAsp(OBzl)ONSu (4.4 mmole) dissolved in 14 ml DMF Followed by 0.75 ml (6.9 mmole) N-methyl morpholine. Standing overnight at room temperature with stirring. Hereby the product started precipitating. Followed by analytical HPLC. 28.5 ml H₂O were added during which the product was precipitated, filtered off and washed with DMF/water-mixture.
5) Synthesis of HAspValTyrOMe.AcOH
Reaction scheme: 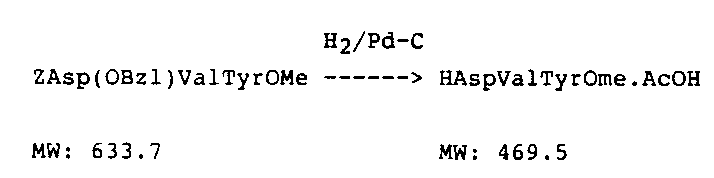 Procedure:
3.2 g ZAsp(OBzl)ValTyrOMe (5.1 mmole) were slurried in 75 ml DMF mixed with 25 ml AcOH, and 3.5 g 5% (w/w) palladium-on-charcoal, slurried in 5 ml H₂O, were added. Then hydrogenation was at a hydrogen pressure of 3 bar overnight in a Parr-hydrogenation apparatus, whereafter the reaction was complete according to analytical HPLC. The product was brought into solution by adding an additional 50 ml AcOH, the catalyst was filtered off and the product was subsequently evaporated to dryness.
Yield: 2.3 g (96%)
6) Synthesis of ZArgLysAspValTyrOMe.AcOH
Reaction scheme: 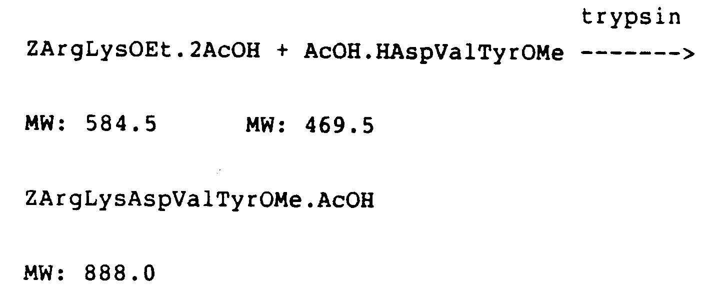 Procedure:
1.40 g HAspValTyrOMe.AcOH (3.0 mmole) were dissolved in 9.0 ml DMF and 4.0 ml 0.5 M TRIS solution, 3.0 ml 0.1 M CaCl₂ solution, 3.0 ml H₂O and 1.86 g LiCl (4,4 mmole) under brief acid pH. pH was adjusted to 8.5, 1.33 g ZArgLysOEt.2AcOH (2.3 mmole) were added. The reaction was initiated by adding 0.3 ml of a 11 mg/ml solution of trypsin. The conversion was followed by analytical HPLC. The reaction was complete after approx. 20 min, and pH was adjusted to 2.
Yield according to analytical HPLC: 33% (determined at 254 nm)
The product is characterized by:

| Analysis: | | |
|---|---|---|
| AAA: | Asp: | 0.94 |
| | Arg: | 0.94 |
| | Tyr: | 1.08 |
| | Val: | 1.09 |
| | Lys: | 0.95 |

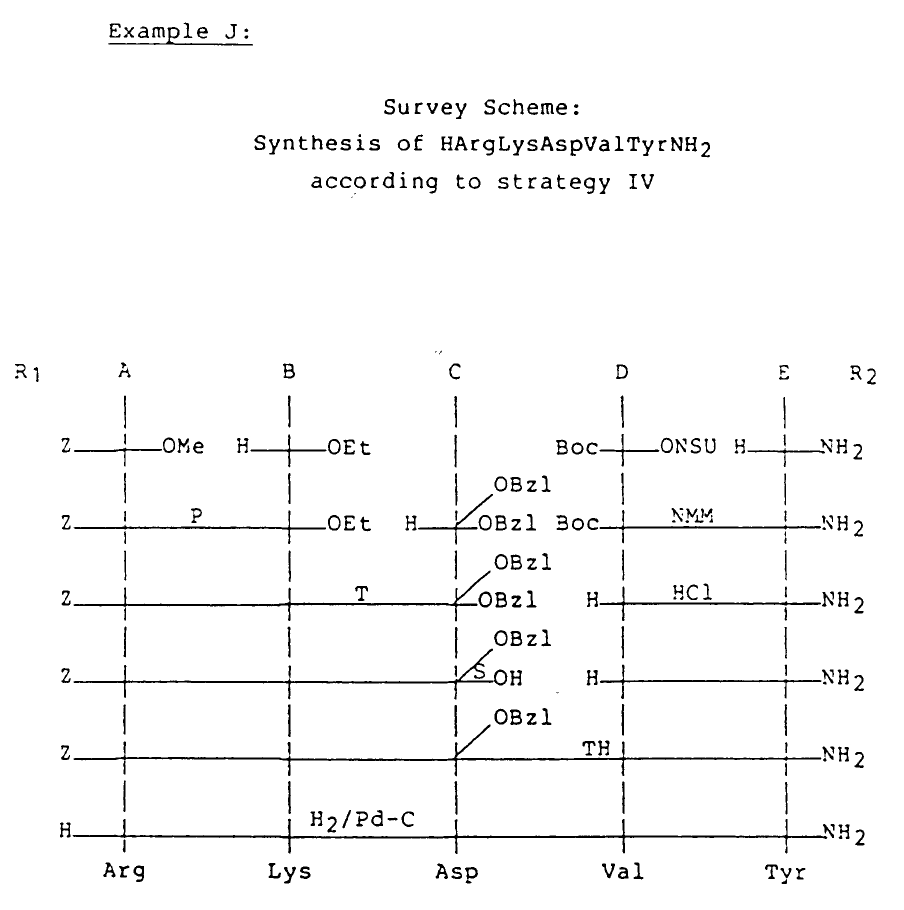
Example J:

Survey Scheme:
Synthesis of HArgLysAspValTyrNH2
according to strategy IV

R1 A B C D E R2

Z OMe H OEt Boc ONSU H NH2

OBzl
Z P OEt H OBzl Boc NMM NH2

OBzl
Z T OBzl H HCl NH2

OBzl
Z S OH H NH2

OBzl
Z TH NH2

H H2/Pd-C NH2

Arg Lys Asp Val Tyr


### Example J:

### Synthesis of H-ArgLysAspValTyrNH₂ according to strategy IV

H-ArgLysAspValTyrNH₂ is synthesized analogously with H-ArgLysGluValTyrNH₂ as described in example C with the following steps:
1) Synthesis of ZArgLysOEt.2AcOH
   Synthesized according to example A,1)
2) Synthesis of ZArgLysAsp(OBzl)₂
   Reaction Scheme: 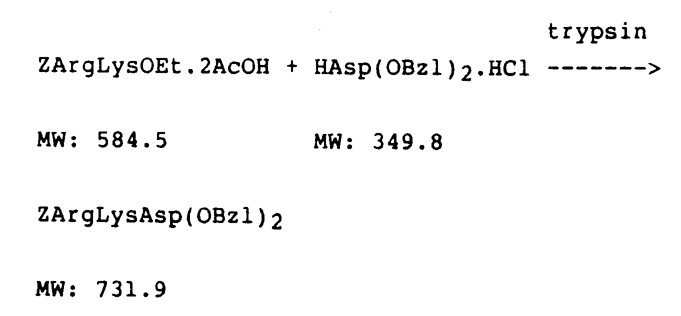 Procedure:
   5.3 g H-Asp(OBzl)₂.HCl (15 mmole) and 0.4 g CaCl₂.2H₂O and 1.3 g TRIS were dissolved in 15 ml DMF + 6 ml H₂O, and pH was adjusted to 8.5 with NaOH-solution. 1.9 g ZArgLysOEt.2AcOH (3.3 mmole) were added as solid matter, and the reaction was initiated by adding 0.1 ml of a solution of 16 mg trypsin in 0.5 ml CaCl₂-solution. The reaction is followed by analytical HPLC, and at suitable conversion additionally 2 times 1.9 g ZArgLysOEt.2AcOH are added as solid matter. In 4 hours the reaction is complete, and pH is adjusted to 3.0, the mixture subsequently being purified.
   Yield according to analytical HPLC: 55% (determined af 254 nm)
   Purification of ZArgLysAsp(OBzl)₂
   The solvent is removed from the product fraction and subsequently the evaporated and redissolved product fraction is placed on a RP-HPLC C18-column and purified with a gradient with increasing content of alcohol in 50 mM AcOH. The product fractions are combined and evaporated.
3) Synthesis of HCl.HValTyrNH₂
   Synthesis as described in example A,2)
4) Synthesis of ZArgLysAsp(OBzl)ValTyrNH₂
   a) ZArgLysAsp(OBzl)OH synthesis
      Reaction scheme: 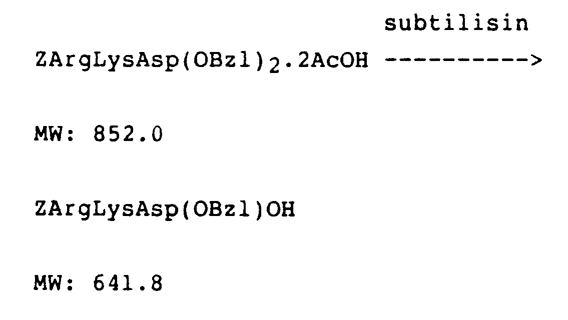 Procedure:
      4.5 g ZArgLysAsp(OBzl)₂.2AcOH (5.3 mmole) are dissolved in 42 ml EtOH + 57 ml H₂O and mixed with 1.3 g neutralized TRIS-solution and 0.8 g KCl. pH is adjusted to 8.0 with NaOH-solution, and the reaction is initiated by adding 0.7 ml of a solution of 41 mg subtilisin in H₂O. pH is maintained at 8.0 with NaOH-solution, and the reaction is followed by analytical HPLC until completed after 1 hour. Hereby the α-benzyl ester is selectively hydrolyzed, and the enzyme is inactivated by lowering pH to 3 with HCl. Possible residues of HAsp(OBzl)₂ can now be removed by extraction with EtOAc at pH 8. Evaporated to 35 ml and transferred to the next step.
      Yield according to HPLC: 95% (determined at 254 nm)
   b) ZArgLysAsp(OBzl)ValTyrNH₂ synthesis
      Reaction scheme: 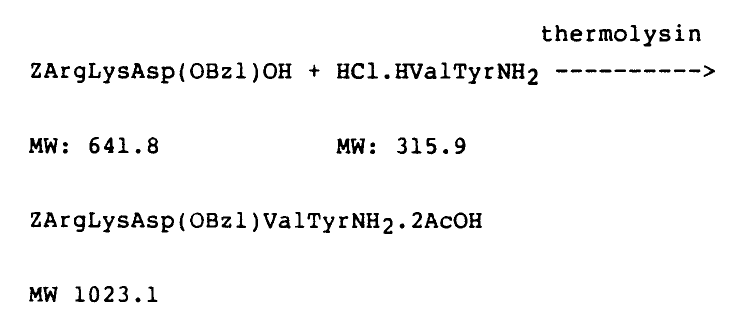 Procedure:
      The ZArgLysAsp(OBzl)OH solution from the previous step is evaporated to 35 ml and 4.2 g HCl.HValTyrNH₂ (13.3 mmole) and 10 ml H₂O and 0.4 g CaCl₂.2H₂O are added, pH subsequently being adjusted to 7 with NaOH solution, and 80 g thermolysin are added to initiate the reaction. The mixture is left to stand for 2 days at room temperature with stirring. pH is adjusted to 3 when the reaction yield is optimal.
      Yield according to HPLC: approx 40% (determined at 254 nm)
      Purification of ZArgLysAsp(OBzl)ValTyrNH₂
      The reaction mixture is filtered. The filter cake is washed carefully with 50 mM AcOH, and subsequently dissolved in alcohol/H₂O and purified on a RP-HPLC C18-column by elution with alcohol/water, 50 mM AcOH. The product fractions are combined and evaporated.
5) Synthesis of HArgLysAspValTyrNH₂
   1.0 g product from the previous step is dissolved in 600 ml 60% EtOH, mixed with 0.1 ml AcOH and 0.1 g 10% (w/w) palladium-on-charcoal, followed by hydrogenation overnight in a Parr-hydrogenation apparatus at a hydrogen pressure of 3 bar. The solution is subsequently filtered and the filtrate evaporated to dryness, redissolved and freeze dried.

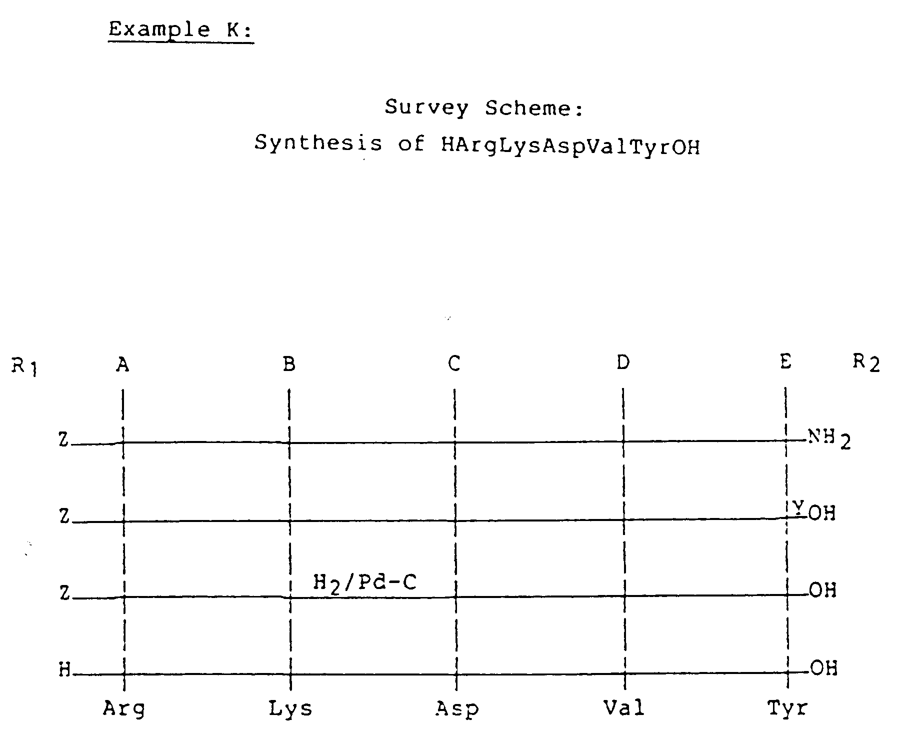
Example K:

Survey Scheme:
Synthesis of HArgLysAspValTyrOH

R1 A B C D E R2

Z NH2

Z Y OH

Z H2/Pd-C OH

H OH

Arg Lys Asp Val Tyr


### Example K:

### Synthesis of Thymopentin-acetate HArgLysAspValTyrOH.AcOH from ZArgLysAspValTyrNH₂.AcOH via enzymatic deblocking

1) Synthesis of ZArgLysAspValTyrOH
Reaction scheme: 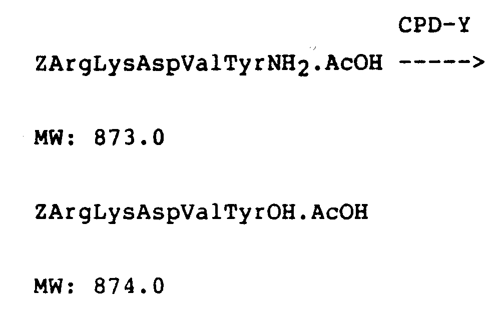 Procedure:
6.8 g ZArgLysAspValTyrNH₂.AcOH (7.8 mmole) were mixed with 80 ml 0.5 M TRIS, 8 ml 0.1 M EDTA, 1.2 g NaCl (20 mmole) and 30 ml DMF. pH was adjusted to 9.0 with approx. 8 ml 6 N NaOH. Clear solution. 62 ml H₂O were added. The reaction was initiated by adding 20 ml CPD-Y (18.8 mg/ml). The reaction was complete in less than 3 hours. Followed by analytical HPLC at 254 nm and 278 nm.
Yield: 98% according to HPLC
The absorbance ratio between abs. at 278 nm and abs. at 254 nm is identical for product and substrate.
Thus, the product contains the chromophores at both ends of the molecule, Z and Tyr in the ratio 1:1.
Isolation of ZArgLysAspValTyrOH.AcOH
The reaction mixture was purified by preparative RP-HPLC on C18-columns and alcohol/water mixtures as eluent with 50 mM AcOH as buffer. The product fractions were combined, evaporated and freeze dried.
Yield: 6.4 g (94%) (white powder)
2) Synthesis of HArgLysAspValTyrOH.AcOH (Thymopentin acetate)
Reaction scheme: 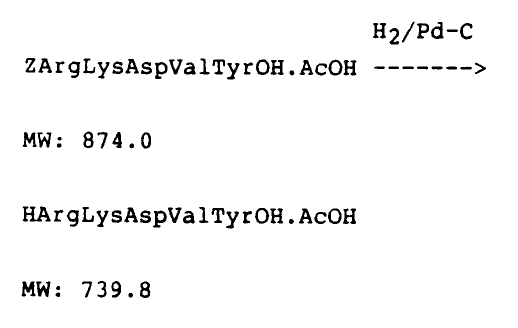 Procedure:
13.1 g ZArgLysAspValTyrOH.AcOH (15.0 mmole) were dissolved in 1 l 40% ethanol/water and mixed with 1.3 g 10% (w/w) palladium-on-charcoal slurried in water and 5 ml acetic acid.
The solution was hydrogenated at a hydrogen pressure of 3 bar in a Parr-hydrogenation apparatus until analytical HPLC indicated almost complete conversion of the starting substance.
Yield according to HPLC: 98%
Isolation of HArgLysAspValTyrOH.AcOH
The reaction mixture was filtered and evaporated. The remainder was redissolved in aqueous AcOH buffer and subjected to column chromatographic purification, the pure product fractions subsequently being combined and evaporated and finally freeze dried from a water/acetic acid mixture.
Yield: 10.7 g (90%; corrected for water and acetate)
Analysis of a product thus obtained, a white, amorphous powder without foreign particles, gave the following results:

| | |
|---|---|
| UV maximum: | 275 nm |
| Product content: | 86.7% (UV 293 nm in 0.1 N NaOH) |
| Specific rotation α_{D}²⁵: | - 22,1° (1% w/w in 0.1 N AcOH) |
| Acetate content: | 9.4% |
| Chloride content: | negative (AgNO₃-precipitation) |
| Water content: | 4.7% (Karl Fisher) |
| Foreign or free amino acids: | None (at AAA) |

| Analysis: | | |
|---|---|---|
| AAA: | Asp: | 0.95 |
| | Arg: | 1.06 |
| | Tyr: | 0.95 |
| | Val: | 0.98 |
| | Lys: | 1.07 |

| | |
|---|---|
| Sequence analysis: | Arg, Lys, Asp, Val, Tyr = 1 2 3 4 5 |
| HPLC purity: | 99.7% (220 nm) |

Example L:

Synthesis of HArgLysGluLeuDtrpNH2 and
HArgLysGluLeuLTrpNH2 according to strategy I

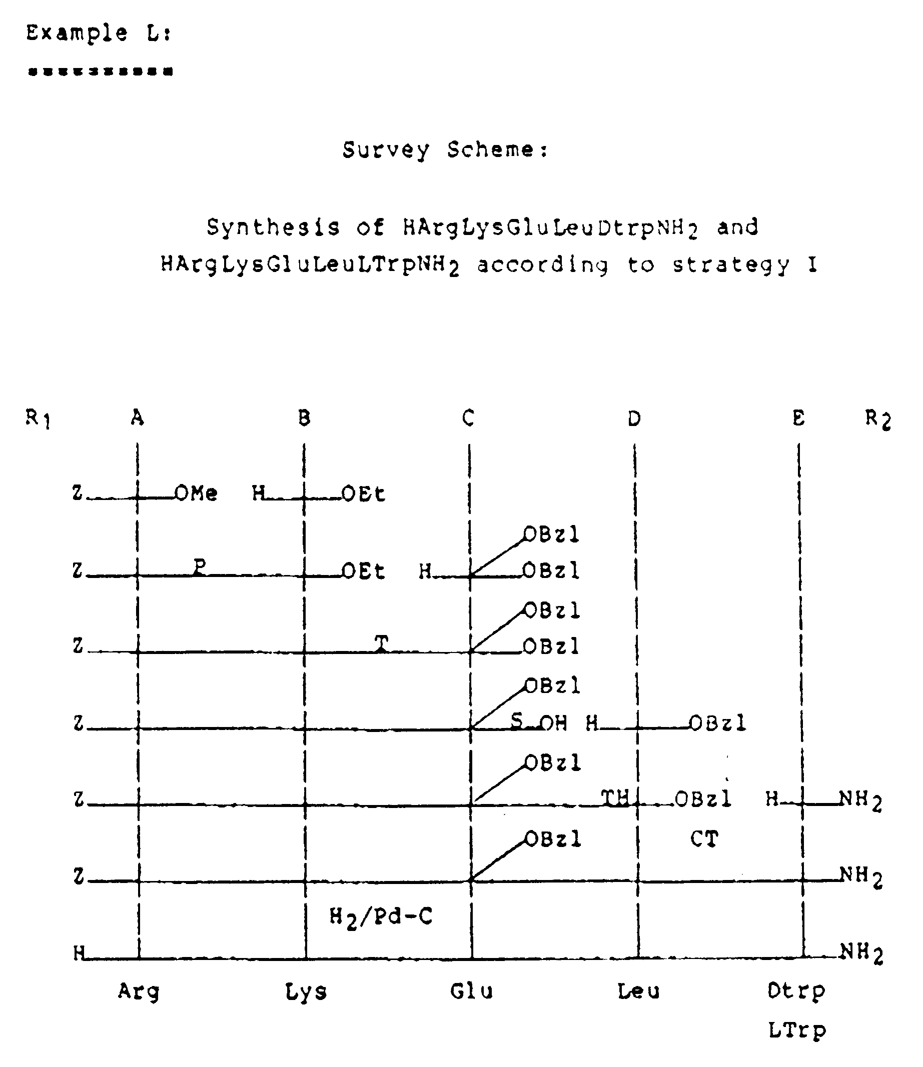
Example L:

Survey Scheme:

Synthesis of HArgLysGluLeuDtrpNH2 and
HArgLysGluLeuLTrpNH2 according to strategy I

R1 A B C D E R2

Z OMe H OEt
OBzl
Z P OEt H OBzl
OBzl
Z T OBzl
OBzl
Z S OH H OBzl
OBzl
Z TH OBzl H NH2
OBzl CT
Z NH2
H2/Pd-C
H NH2

Arg Lys Glu Leu Dtrp
LTrp


1) Synthesis of ZArgLysGlu(OBzl)OH·AcOH
(Synthesized as described in Example C,5a)
The compound was isolated as acetate following purification by C₁₈ RP-HPLC, evaporation and freezedrying.
2) Synthesis of ZArgLysGlu(OBzl)LeuOBzl·2AcOH
Reaction scheme: 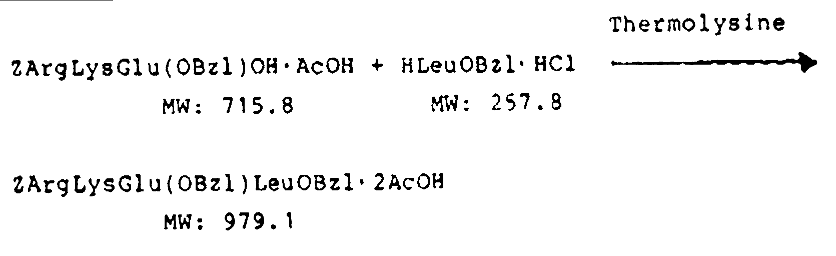 Procedure:
2.00 g ZArgLysGlu(OBzl)OH·AcOH (2.8 mmole) and 3.00 g HLeuOBzl·HCl (11.6 mmole) were dissolved in 24 ml of H₂O, and 0.19 g of CaCl₂·2H₂O and 0.65 g of TRIS were added, while pH was adjusted to 7.0 using aqueous NaOH solution. The reaction was initiated by addition of 26 mg of Thermolysine.
The mixture was soon unclear and was stirred for 2 hours at the same temperature with stirring. pH was then adjusted to 4 using aqueous HCl-solution.
Yield according to analytical HPLC: 61%
(Determined at 254 nm)
Purification of ZArgLysGlu(OBzl)LeuOBzl:
The mixture was diluted in water and purified on a RP-HPLC C₁₈-column, using a gradient of an increasing alcohol-content in 50 mM HoAc. Fractions containing the pure product were combined, evaporated under reduced pressure and finally freezedried.
Yield: 1.48 g (52%)
3) Synthesis of ZArgLysGlu(OBzl)LeuDtrpNH₂
Reaction scheme: 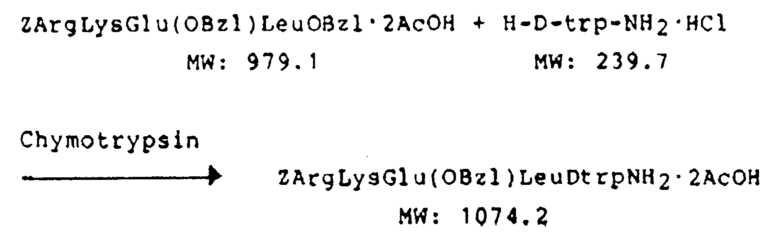 Procedure:
240 mg HCl·DtrpNH₂ (1,0 mmole), which had been analytically shown to contain less than 1% of the corresponding L-isomer, were dissolved in 0.6 ml of DMF and 1.2 ml of H₂O, and pH was adjusted to 8.5 using aqueous NaOH-solution.
88 mg ZArgLysGlu(OBzl)LeuOBzl 2AcOH (0.1 mmole) were then added, and following readjustment of pH to 8.5. The reaction was initiated by addition of 0.3 mg of chymotrypsin. Reaction time 1 hour at room temperature with stirring.
Yield according to HPLC: 41% (determined at 290 nm)

| Analysis: | | |
|---|---|---|
| AAA: | Glu: | 1.04 |
| | Arg: | 1.02 |
| | Leu: | 1.00 |
| | Trp: | 0.78 |
| | Lys: | 0.94 |

UV-absorbance at 290 nm.
4) Synthesis of ZArgLysGlu(OBzl)LeuLTrpNH₂
Reaction scheme: 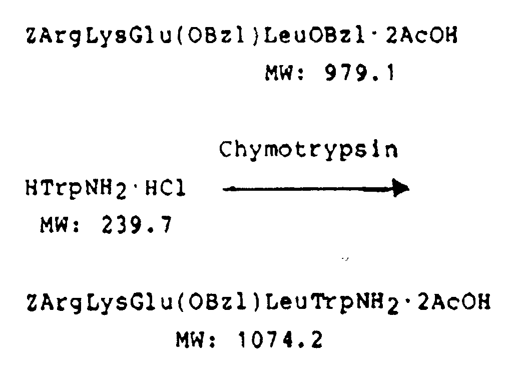 Procedure:
The reaction was carried out analogously to the one described under 3), but using 240 mg and L-TrpNH₂·HCl in stead of the D-form.
Yield according to HPLC: 63% (determined at 290 nm)

| Analysis: | | |
|---|---|---|
| AAA: | Glu: | 1.08 |
| | Arg: | 0.96 |
| | Leu: | 1.04 |
| | Trp: | 1.73 |
| | Lys: | 0.95 |

UV-absorbance at 290 nm.
5) Synthesis of H-ArgLysGluLeutrpNH₂ and H-ArgLysGluLeuTrpNH₂
Following isolation by C₁₈ RP-HPLC of the products prepared under 3) and 4), these products are dissolved in Ethanol/water and after addition of 10% (w/w) of 10% Palladium on carbon, hydrogenated at 4 bar H₂ overnight. The deprotected compounds are then concentrated by evaporation and freezedied. 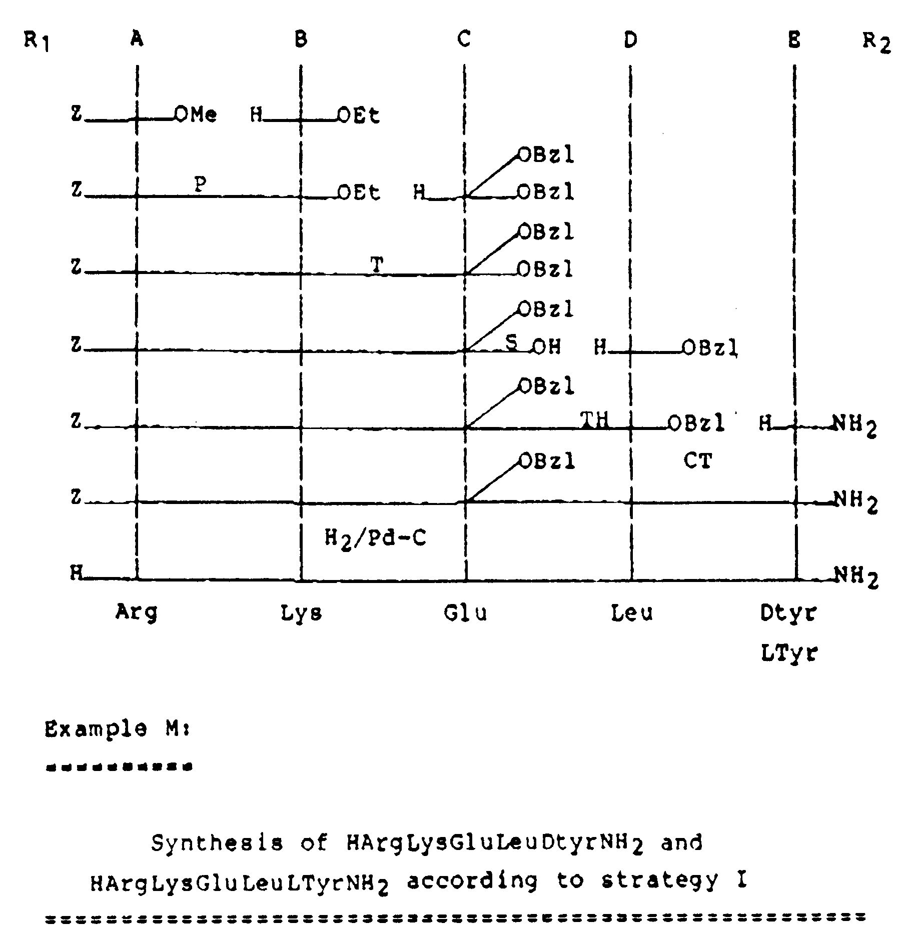
R1 A B C D E R2
Z OMe H OEt
OBzl
Z P OEt H OBzl
OBzl
Z T OBzl
OBzl
Z S OH H OBzl
OBzl
Z TH OBzl H NH2
OBzl CT
Z NH2
H2/Pd-C
H NH2
Arg Lys Glu Leu Dtyr
LTyr

Example M:

Synthesis of HArgLysGluLeuDtyrNH2 and
HArgLysGluLeuLTyrNH2 according to strategy I
 
Example M:
==========

Survey Scheme:

Synthesis of HArgLysGluLeuDtyrNH2 and
HArgLysGluLeuLTyrNH2 according to strategy I
1) Synthesis of ZArgLysGlu(OBzl)LeuOBzl·2AcOH
(Synthesized as described in Example L,1)-2).
2) Synthesis of ZArgLysGlu(OBzl)LeuDtyrNH₂
Reaction scheme: 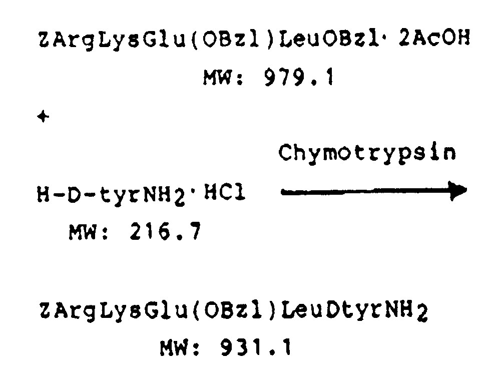 Procedure:
216 mg HCl·DtyrNH₂ (1.0 mmole), which had been analytically shown to contain less than 1% of the corresponding L-isomer, was dissolved in 0.6 ml of DMF and 1.2 ml of H₂O, and pH was adjusted to 8.5 with aqueous NaOH-solution.
88 mg ZArgLysGlu(OBzl)LeuOBzl·2AcOH (0.1 mmole) was then added, and following readjustment of pH to 8.5. The reaction was initiated by addition of 0.3 mg of chymotrypsin. The mixture was then stirred for 2 hours at room temperature.
Yield according to HPLC: 88% (determined at 254 nm)

| Analysis: | | |
|---|---|---|
| AAA: | Glu: | 0.99 |
| | Arg: | 1.04 |
| | Tyr: | 0.80 |
| | Leu: | 1.02 |
| | Lys: | 0.95 |

UV-absorbance at 278 nm.
3) Synthesis of ZArgLysGlu(OBzl)LeuTyrNH₂
Reaction scheme: 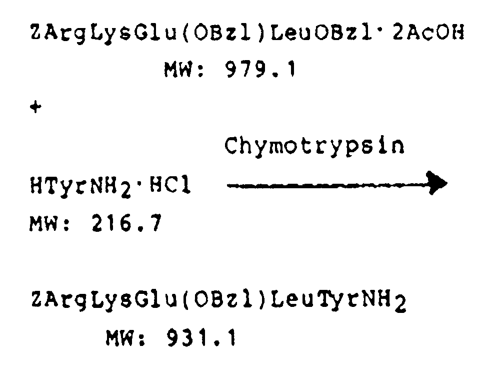 Procedure:
The reaction was carried out analogously to the one described under 2), but using 216 mg and L-TyrNH₂·HCl in stead of the D-form.
Yield according to HPLC: 95% (Determined at 254 nm.)
4) Synthesis of HArgLysGluLeuDtyrNH₂ and HArgLysGluLeuTyrNH₂
These compounds are prepared from the products of 2) and 3) as described in Example M,5. 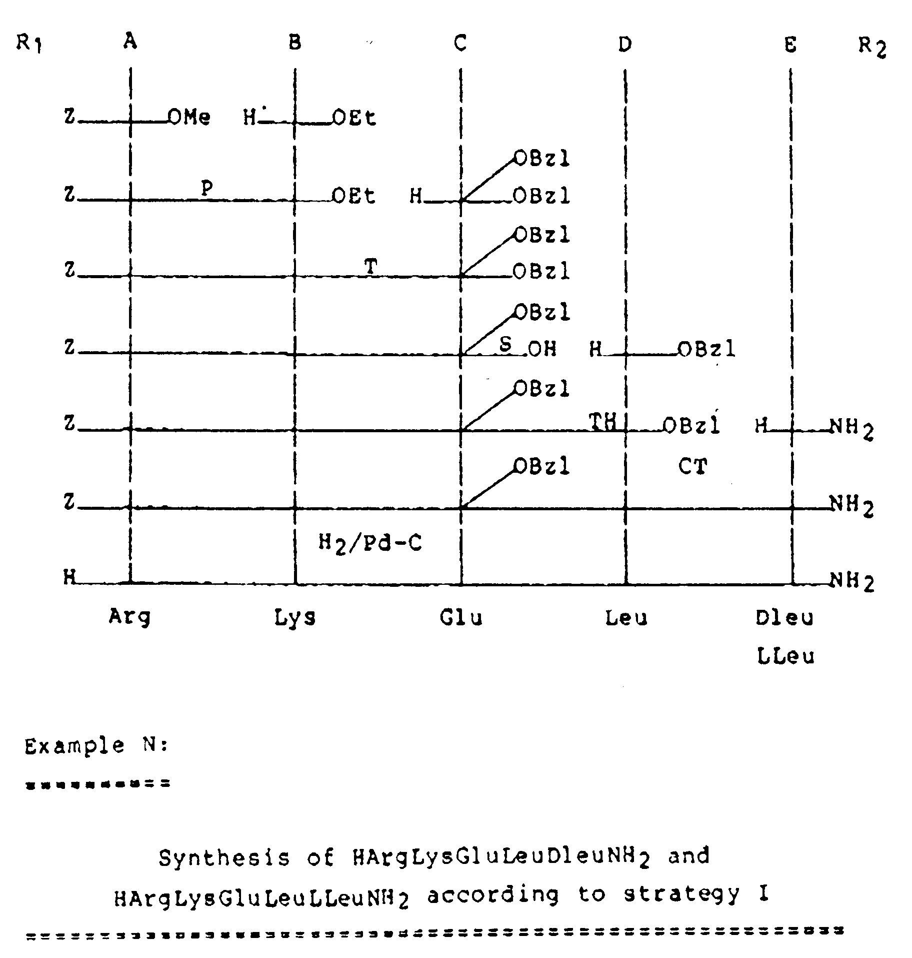
R1 A B C D E R2
Z OMe H OEt
OBzl
Z P OEt H OBzl
OBzl
Z T OBzl
OBzl
Z S OH H OBzl
OBzl
Z TH OBzl H NH2
OBzl CT
Z NH2
H2/Pd-C
H NH2
Arg Lys Glu Leu Dleu
LLeu

Example N:
==========

Synthesis of HArgLysGluLeuDleuNH2 and
HArgLysGluLeuLLeuNH2 according to strategy I
============================================
 
Example N:
==========

Survey Scheme:

Synthesis of HArgLysGluLeuDleuNH2 and
HArgLysGluLeuLLeuNH2 according to strategy I
1) Synthesis of ZArgLysGlu(OBzl)LeuOBzl·2AcOH
(Synthesized as described in Example L,1)-2).
2) Synthesis of ZArgLysGlu(OBzl)LeuDleuNH₂
Reaction scheme: 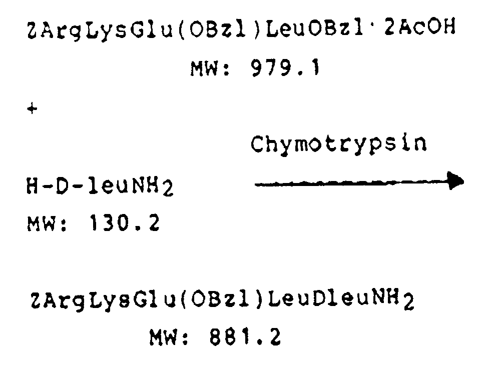 Procedure:
38 mg HCl·DleuNH₂ (0.3 mmole), which had been analytically shown to contain less than 1% of the corresponding L-isomer, was dissolved in 0.6 ml of DMF and 1.2 ml of H₂O, and pH was adjusted to 8.5 with aqueous NaOH-solution.
88 mg ZArgLysGlu(OBzl)LeuOBzl·2AcOH (0.1 mmole) was then added, and following readjustment of pH to 8.5. The reaction was initiated by addition of 0.3 mg of chymotrypsin. The rection mixture was then stirred for 1/2 hour at room temperature.
Yield according to HPLC: 15% (determined at 254 nm)

| Analysis: | | |
|---|---|---|
| AAA: | Glu: | 0.99 |
| | Arg: | 1.05 |
| | Leu: | 1.95 |
| | Lys: | 1.00 |

3) Synthesis of ZArgLysGlu(OBzl)LeuLeuNH₂
Reaction scheme: 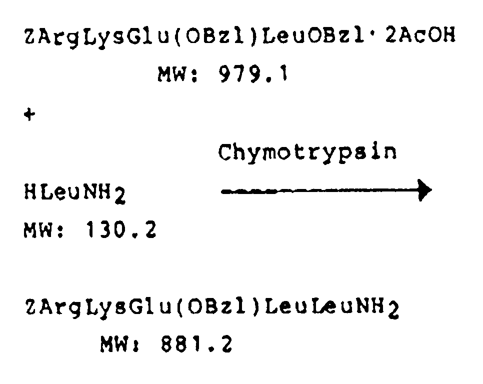 Procedure:
The reaction was carried out analogously to the one described under 2), but using 38 mg and L-LeuNH₂·HCl instead of the D-form.
Yield according to HPLC: 85% (determined at 254 nm.)
4) Synthesis of HArgLysGluLeuDleuNH₂ and HArgLysGluLeuLeuNH₂
These compounds are prepared from the products of 2) and 3) according to the procedure described in Example M,5. 
Example O:
==========

Synthesis of HArgLysAspValDtyrNH2 and
HArgLysAspValTyrNH2 according to strategy III.
==============================================
 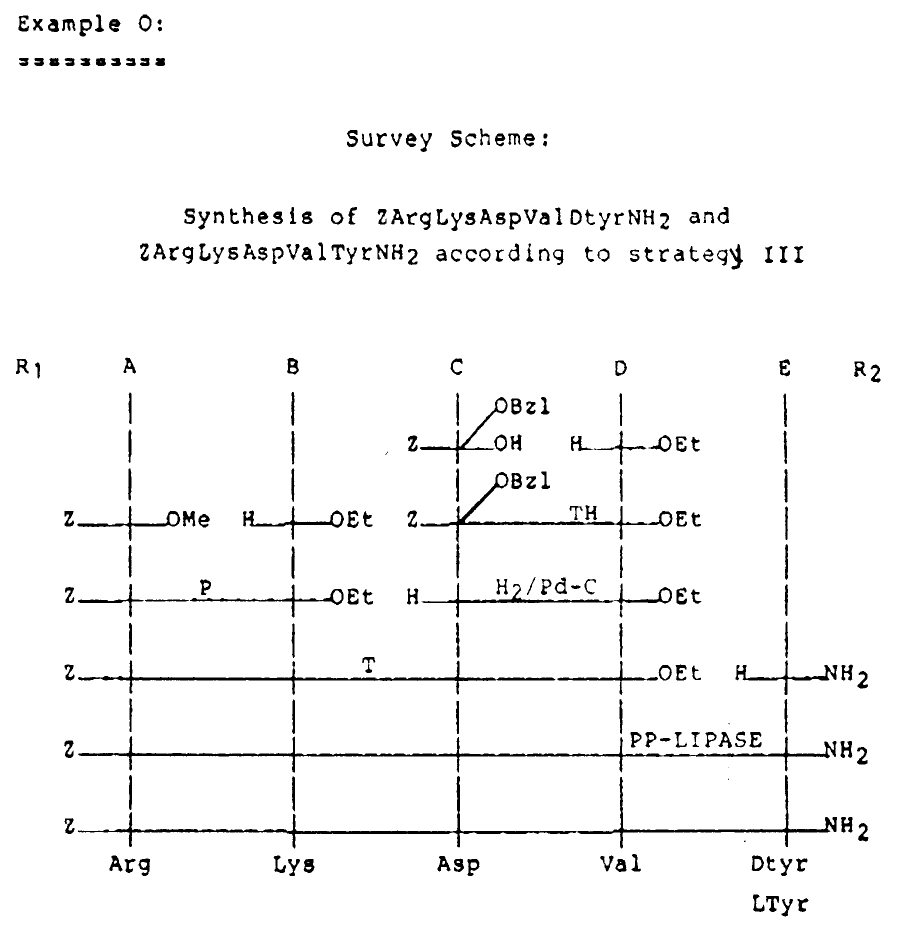
Example O:
==========

Survey Scheme:

Synthesis of ZArgLysAspValDtyrNH2 and
ZArgLysAspValTyrNH2 according to strategy III

R1 A B C D E R2


OBzl
Z OH H OEt
OBzl
Z OMe H OEt Z TH OEt
Z P OEt H H2/Pd-C OEt
Z T OEt H NH2
Z PP-LIPASE NH2
Z NH2

Arg Lys Asp Val Dtyr
LTyr
1) Synthesis of ZArgLysAspValOEt AcOH
(Synthesized as described in Example E,1-5.
2) Synthesis of ZArgLysAspValDtyrNH₂
Reaction scheme: 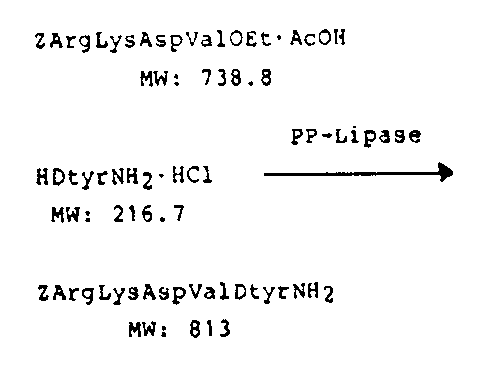 Procedure:
216 mg HCl·DtyrNH₂ (1.0 mmole), which had been analytically shown to contain less than 1% of the corresponding L-isomer, was dissolved in 1.5 ml of water, and pH was adjusted to 8.5 with aqueous sodium hydroxide solution. 68 mg ZArgLysAspValOEt·AcOH (0.1 mmole) were added following readjustment of pH.
The reaction was initiated by addition of 14 mg of Porcine Pancreatic Lipase. The reaction was stirred for 8 hours at room temperature, hereafter the conversion was 75% complete.
Yield according to HPLC: 36% (determined at 254 nm)

| Analysis: | | |
|---|---|---|
| AAA: | Asp: | 1.35 |
| | Arg: | 0.80 |
| | Val: | 0.82 |
| | Tyr: | 1.29 |
| | Lys: | 0.73 |

UV-absorbance at 278 nm
3) Synthesis of ZArgLysAspValTyrNH₂
(Z-TP-5-amide)
Reaction scheme: 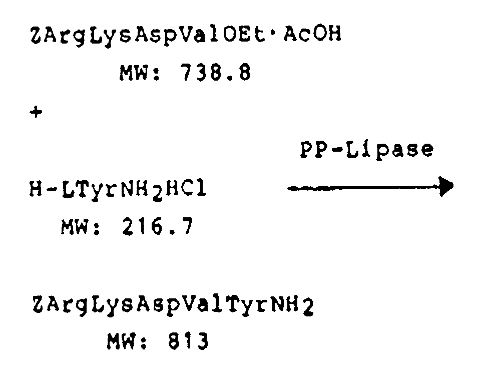 Procedure:
The reaction was carried out analogously to the one described under 3), but 216 mg L-TyrNH₂·HCl in stead of the D-form. Reaction time 4 hours. The conversion was less than 75%.
Yield according to HPLC: 22% (determined at 254 nm.)

| Analysis: | | |
|---|---|---|
| AAA: | Asp: | 0.80 |
| | Arg: | 1.00 |
| | Val: | 1.36 |
| | Tyr: | 0.66 |
| | Lys: | 1.18 |

UV-absorbance at 278 nm. 
Example P:
==========

Synthesis of HArgLysGluAlaValNH2
according to strategy I.
==============================================
 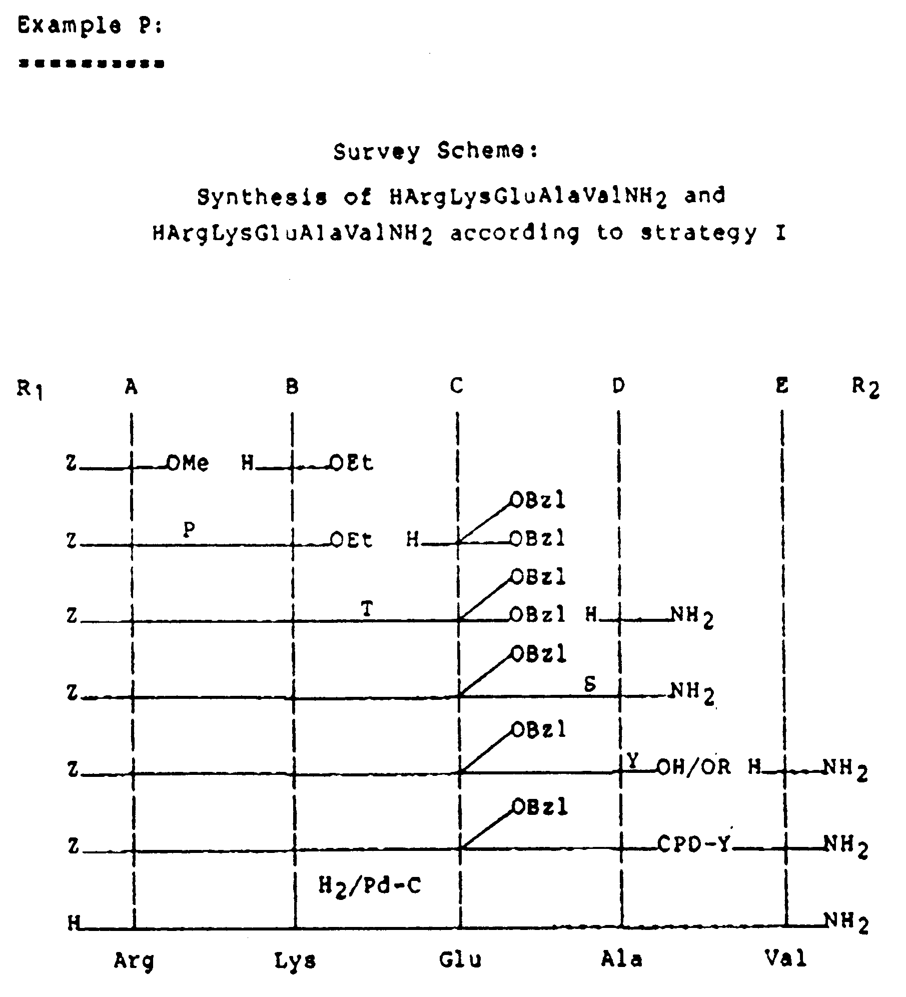
Example P:
==========

Survey Scheme:
Synthesis of HArgLysGluAlaValNH2 and
HArgLysGluAlaValNH2 according to strategy I


R1 A B C D E R2

Z OMe H OEt
OBzl
Z P OEt H OBzl
OBzl
Z T OBzl H NH2
OBzl
Z S NH2
OBzl
Z Y OH/OR H NH2
OBzl
Z CPD-Y NH2
H2/Pd-C
H NH2

Arg Lys Glu Ala Val
1) Synthesis of ZArgLysGlu(OBzl)·2AcOH
Synthesis carried out as described in Example C,2.
2) Synthesis of ZArgLysGlu(OBzl)AlaNH₂
Reaction scheme: 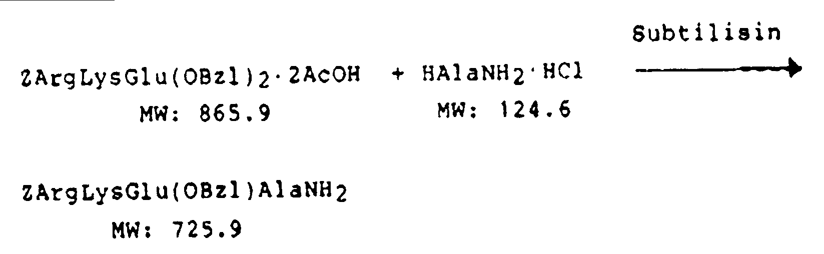 Procedure:
200 mg L-AlaNH₂·HCl (1.6 mmole) were dissolved in 0.4 ml DMF and 1.4 ml O.1 M KCl/0.1 M TRIS, and pH was adjusted to 8.5 using aqueous sodium hydroxide.
86 mg ZArgLysGlu(OBzl)₂·2AcOH (0.1 mmole) were added to the solution and following readjustment of pH. The reaction was initiated by addition of 0.1 mg of Subtilisin and stirred for 20 min. at room temperature, and then terminated by lowering pH to 3.
Yield according to HPLC: 36% (Determined at 254 nm)

| Analysis: | | |
|---|---|---|
| AAA: | Glu: | 1.04 |
| | Arg: | 1.10 |
| | Ala: | 0.95 |
| | Lys: | 0.91 |

3) Synthesis of HArgLysGluAlaValNH₂
The peptide prepared in reaction 2) is isolated by RP-HPLC of the mixture and deamidated by using Elastase or Phenacylbromide modified CPD-Y. Following reesterification it is then coupled to H-ValNH₂ in a reaction as described in Example G,2. Finally it is deprotected using catalytical hydrogenation as described in Example L,5. 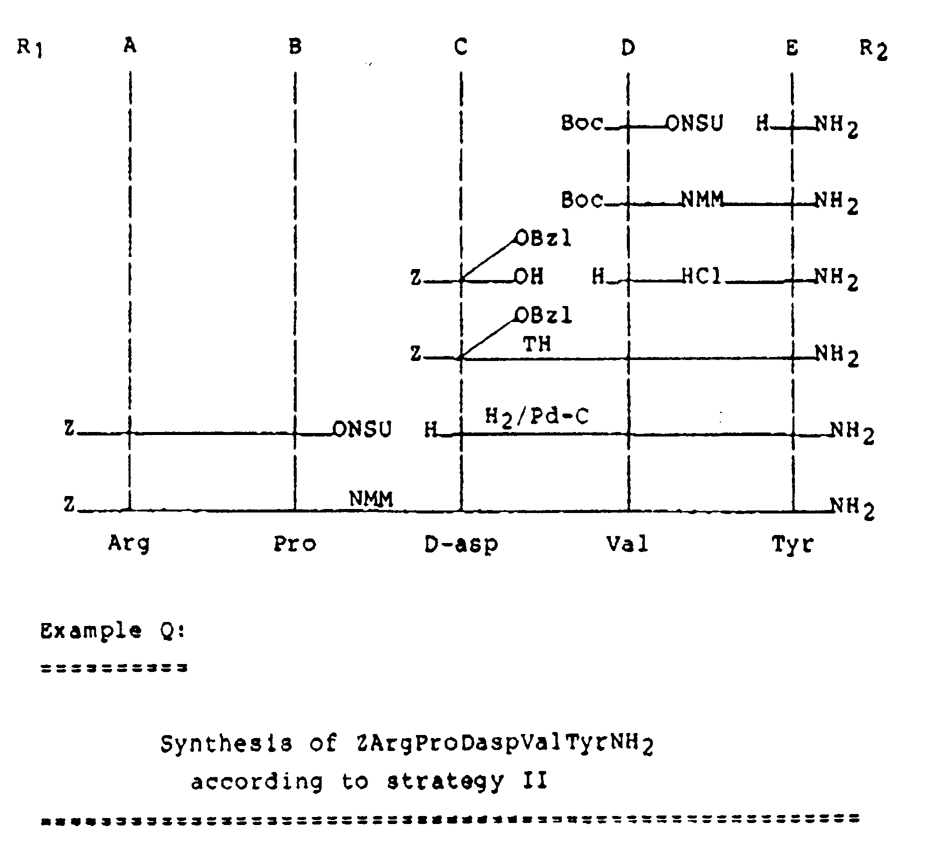
R1 A B C D E R2


Boc ONSU H NH2

Boc NMM NH2

OBzl

Z OH H HCl NH2

OBzl

TH

Z NH2

Z ONSU H H2/Pd-C NH2

Z NMM NH2

Arg Pro D-asp Val Tyr

Example Q:
==========

Synthesis of ZArgProDaspValTyrNH2
according to strategy II
==========================================
 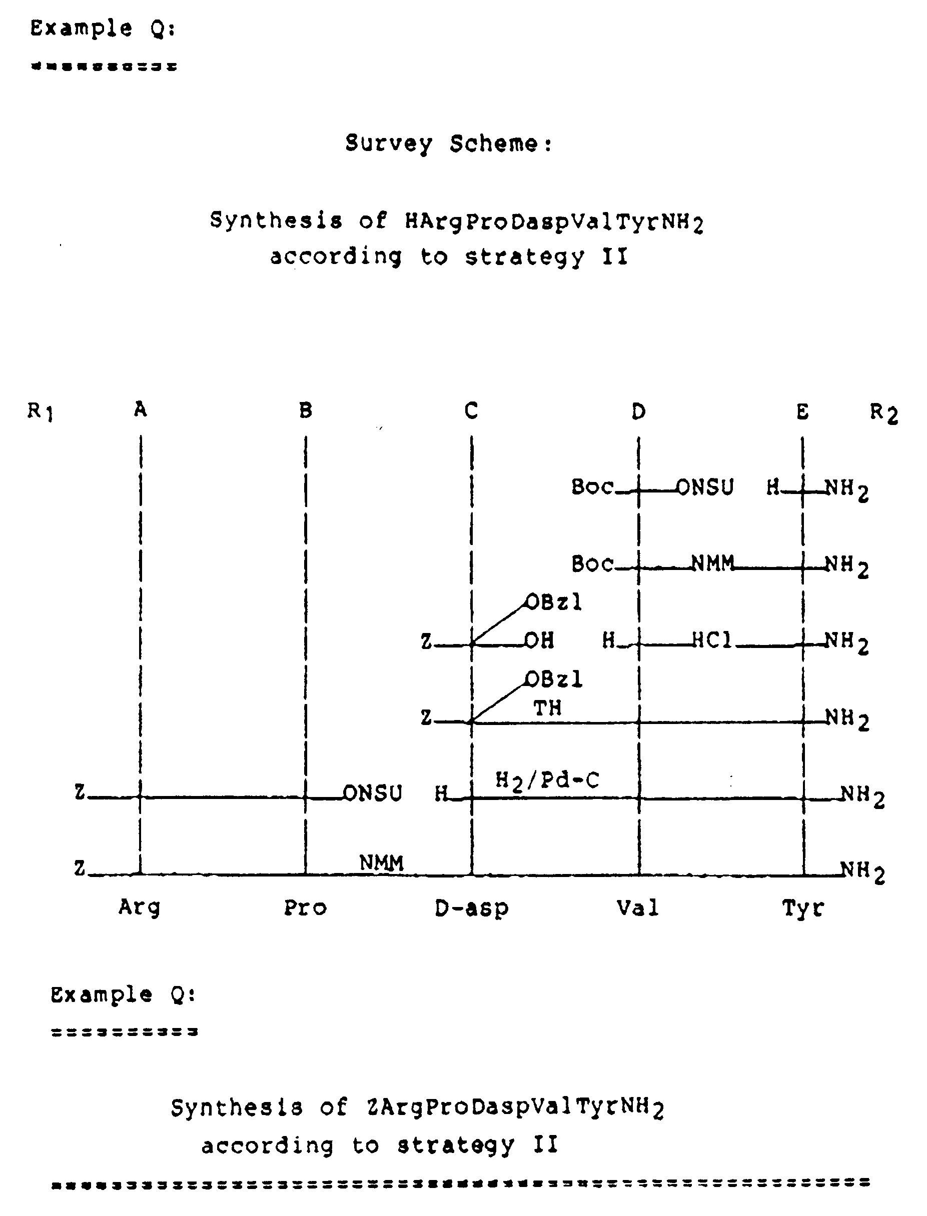
Example Q:
==========

Survey Scheme:

Synthesis of HArgProDaspValTyrNH2
according to strategy II

R1 A B C D E R2
Boc ONSU H NH2
Boc NMM NH2
OBzl
Z OH H HCl NH2
OBzl
TH
Z NH2
Z ONSU H H2/Pd-C NH2
Z NMM NH2
Arg Pro D-asp Val Tyr

Example Q:
==========

Synthesis of ZArgProDaspValTyrNH2
according to strategy II
==================================================
1) Synthesis of H-ValTyrNH₂·HCl
Synthesis carried out as described in Example A,2.
2) Synthesis of ZDasp(OBzl)ValTyrNH₂
Reaction scheme: 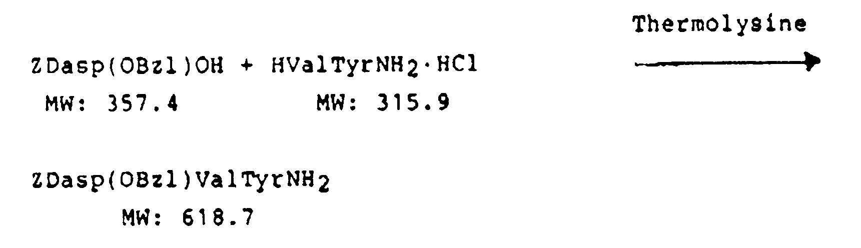 Procedure:
72 mg ZDasp(OBzl)OH (0.2 mmole), which had been shown analytically to contain less than 1% of the corresponding L-isomer, was dissolved in 0.4 ml DMF and added to a solution of 126 mg HCl·ValTyrNH₂ (0.4 mmole) in 1.4 ml H₂O mixed with 0.1 ml DMF, in which pH had been readjusted to 7. The solution also contained 14 mg CaCl₂ and 48 mg TRIS. The reaction was then initiated by addition of 8 mg of Thermolysine. The reaction was stirred for two days at room temperature.
Yield according to HPLC: 12% (Determined at 254 nm)

| Analysis: | | |
|---|---|---|
| AAA: | Asp: | 1.16 |
| | Val: | 0.91 |
| | Tyr: | 0.93 |

UV-absorbance at 278 nm.
3) Synthesis of ZArgProDaspValTyrNH₂
The product prepared as described is hydrogenated as described in Example A,3, but with HCl as the only added acid. It is then reacted with ZArgProONSu·HCl in anhydrous DMF-solution containing equimolar amounts of reactants and NMM. 
Example R:
==========

Synthesis of HArgLysGluTyrTyrOEt
according to strategy IV
==============================================
 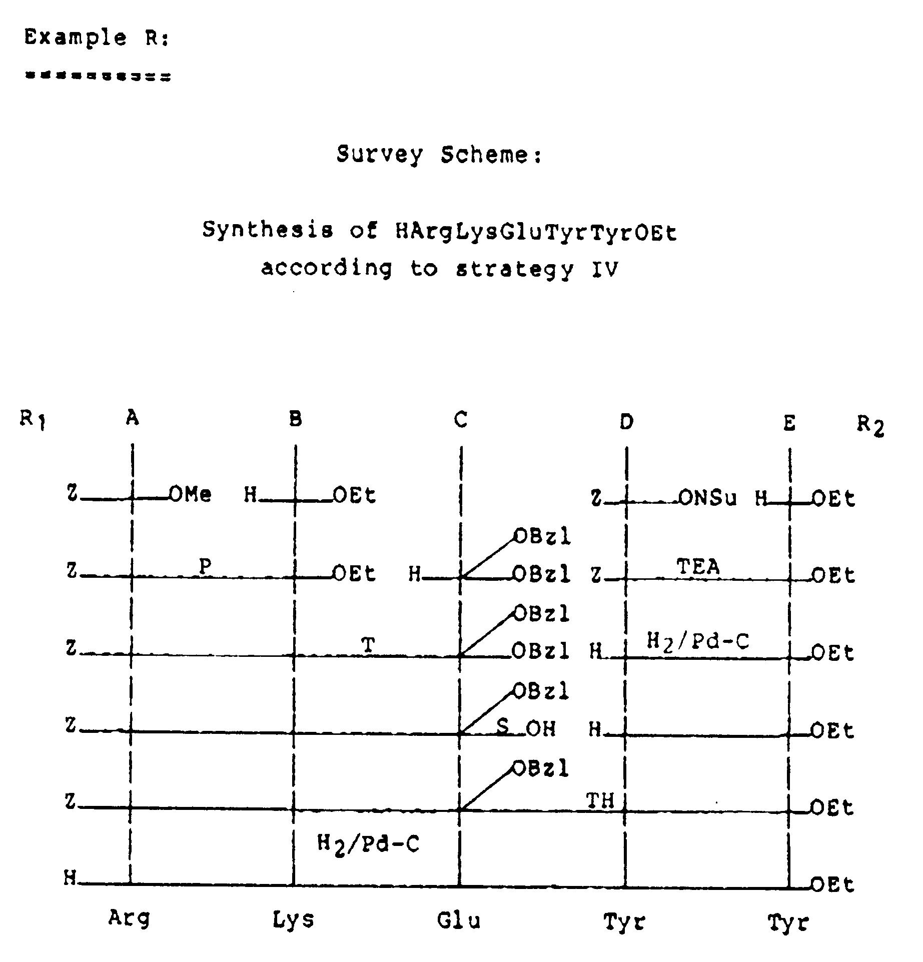
Example R:
==========

Survey Scheme:

Synthesis of HArgLysGluTyrTyrOEt
according to strategy IV

R1 | A | B | C | D | E | R2

Z | OMe | H | OEt | Z | ONSu | H | OEt

Z | P | OEt | H | OBzl | OBzl | Z | TEA | OEt

Z | T | OBzl | OBzl | H | H2/Pd-C | OEt

Z | OBzl | S | OH | H | OEt

Z | OBzl | TH | OEt

H2/Pd-C

H | OEt

Arg | Lys | Glu | Tyr | Tyr

1) Synthesis of ZArgLysGlu(OBzl)OH·AcOH
Synthesis as described in Example L,1.
2) Synthesis of HCl·TyrTyrOEt
ZTyrTyrOEt was prepared using standard chemical methods as outlined in the survey scheme. It was then purified using C₁₈ RP-HPLC and finally hydrogenated in the presence of HCl-solution, and HCl·HTyrTyrOEt was isolated as a white amourphons powder. It was further caracterized by specific optical rotation.
3) Synthesis of ZArgLysGlu(OBzl)TyrTyrOEt
Reaction scheme: 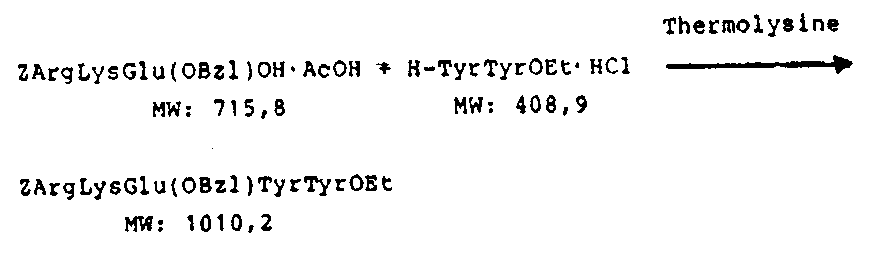 78 mg ZArgLysGlu(OBzl)OH·AcOH (0.1 mmole) and 82 mg HTyrTyrOEt·HCl (0.2 mmole) were dissolved in 0.25 ml of DMF and 0.75 ml H₂O containing 7 mg CaCl₂·2H₂O and 25 mg TRIS, pH being adjusted to 7 by aqueous HCl-solution. The reaction was initiated by addition of 5 mg of Thermolysine, and was stirred at room temperature for 4 hours. The solution remained clear.
Yield according to HPLC: 23% (determined at 254 nm.)

| Analysis: | | |
|---|---|---|
| AAA: | Glu: | 1.07 |
| | Arg: | 1.03 |
| | Tyr: | 1.93 |
| | Lys: | 0.97 |

UV absorbance at 278 nm.
4) Synthesis of HArgLysGluTyrTyrOEt
This compound is prepared from the product of 3) using the procedures described in Example L,5. 
R1 A B C D E R2

Z OMe H OEt Z OEt H NH2

OBzl

Z P OEt H OBzl Z CT NH2

OBzl

Z T OBzl H NH2

Z OH H NH2

OBzl

Z TH NH2

H2/Pd-C

H NH2

Arg Lys Glu Phe Dtyr

Example 5:
==========

Synthesis of HArgLysGluPheDtyrNH2
according to strategy IV
==================================================
 
Example S:
==========

Survey Scheme:

Synthesis of HArgLysGluPheDtyrNH2
according to strategy IV
1) Synthesis of ZArgLysGlu(OBzl)OH·AcOH
Synthesis as described in Example L,1.
2) Synthesis of HTrpDPheNH₂·HoAc
a) Synthesis of ZPheDtyrNH₂
   Reaction scheme: 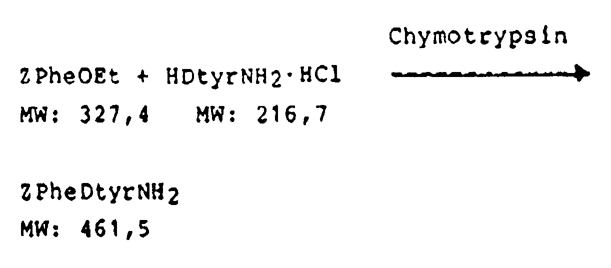 Procedure:
   0.46 g ZPheOEt (1.4 mmole) and 1.21 g H-DtyrNH₂·HCl (5.6 mmole), which had been analytically shown to contain less than 1% of the corresponding L-isomer, were dissolved in 5.6 ml DMF and 7.5 ml H₂O, pH being adjusted to 8.5 using aqueous sodium hydroxide solution. The reaction was initiated by addition of 3.5 mg Chymotrypsin, and was stirred overnight with resulting product precipitation.
   pH was then adjusted to 3 using aqueous HCl-solution, and the reaction mixture was dissolved by addition of NaOH.
   Purification of ZPheDtyrNH₂
   The mixture, which was purified by C₁₈ RP-HPLC and fractions containing pure product, was combined and concentrated under reduced pressure and dried by repeating this procedure with addition of anhydrous Ethanol.
   Yield: 0.37g (57%)
b) Synthesis of HOAc·HPheDtyrNH₂
   Reaction scheme: 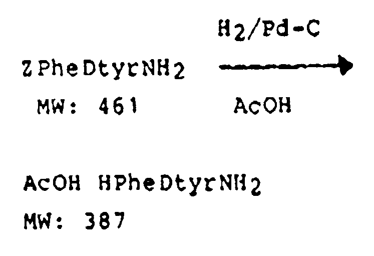 Procedure:
   The product obtained under a) was dissolved in 200 ml of Ethanol, and 1 ml of glacial Acetic Acid was added. It was then hydrogenateted overnight in a PARR-hydrogenator at 3 bar H₂-pressure, following addition of 50 mg Palladium on carbon (10% w/w).
   It was finally evaporated under reduced pressure, following filtration.
   Yield: 3.1 (100%)

3) Synthesis of ZArgLysGlu(OBzl)PheDtyrNH₂
Reaction scheme: 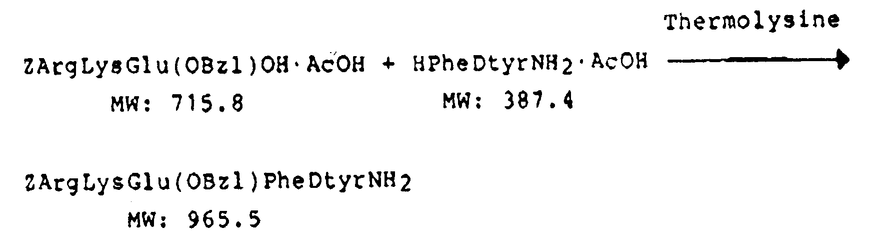 Procedure:
78 mg ZArgLysGlu(OBzl)OH AcOH (0.1 mmole) and 77 mg HPheDtyrNH₂·AcOH (0.2 mmole) were dissolved in 0.9 ml of H₂O containing 7 mg CaCl₂ and 25 mg TRIS, pH being adjusted to 7 using aqueous HCl-solution. The reaction was initiated by addition of 10 mg of Themolysine and was stirred for two days at room temperature.
Yield according to HPLC: 10% (Determined at 254 nm)

| Analysis: | | |
|---|---|---|
| AAA: | Glu: | 1.19 |
| | Arg: | 1.03 |
| | Tyr: | 0.94 |
| | Phe: | 0.92 |
| | Lys: | 0.90 |

UV-absorbance at 278 nm.
4) Synthesis of HArgLysGluPheDtyrNH₂
This compound is prepared from the product of 3) according to the product described in Example L,5. 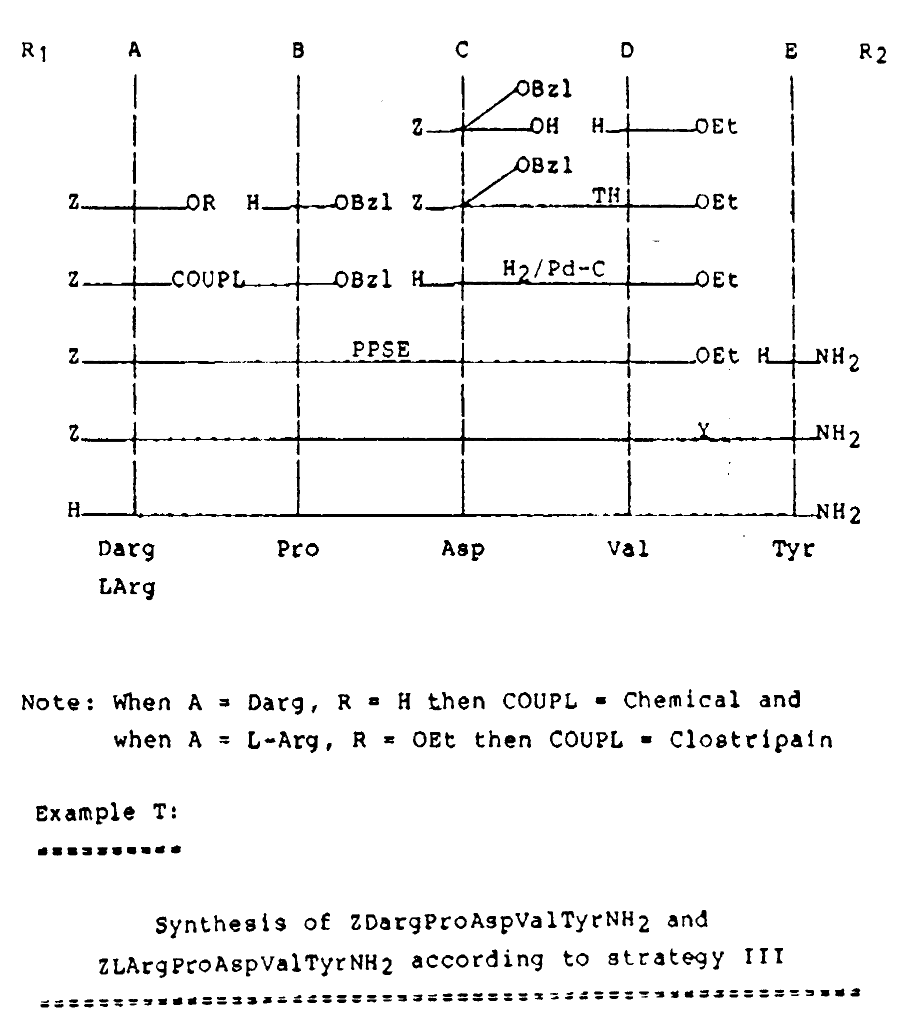
R1 A B C D E R2


Z OBzl OH H OEt
OBzl
Z OR H OBzl Z TH OEt
Z COUPL OBzl H H2/Pd-C OEt
Z PPSE OEt H NH2
Z Y NH2
H NH2

Darg Pro Asp Val Tyr
LArg

Note: When A = Darg, R = H then COUPL = Chemical and
when A = L-Arg, R = OEt then COUPL = Clostripain

Example T:

Synthesis of ZDargProAspValTyrNH2 and
ZLArgProAspValTyrNH2 according to strategy III
 
Example T:
==========

Survey Scheme:

Synthesis of ZDargProAspValTyrNH2 and
ZArgProAspValTyrNH2 according to strategy III (and II)
1) Synthesis of ZDArgProOBzl·AcOH
This method was synthesized by conventional chemical coupling as outlined in the survey scheme. It was purified by C₁₈ RP-HPLC using an alcohol gradient in 50 mM HOAc and from the combined fractions containing the pure product it was isolated as a white amorphous powder following evaporation and freezedrying.
2) Synthesis of AcOH HAspValOEt
This compound was synthesized as described under Example E,3.
3) Synthesis of ZDargProAspValOEt
Reaction scheme: 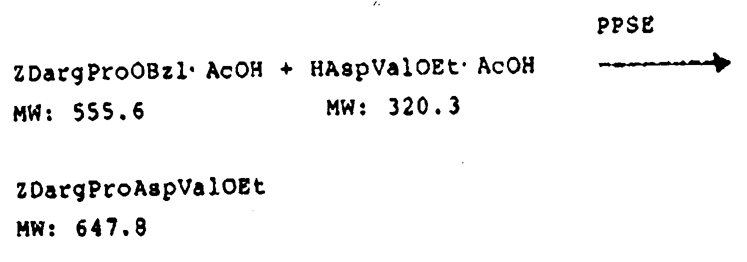 Procedure:
191 mg AspValOEt·AcOH (0.6 mmole) were dissolved in 0.15 ml DMF and 0.1 ml 25 mM Phosphatebuffer, and pH adjusted to 8.5 using aqueous NaOH-solution. 17 mg ZDargProOBzl·AcOH (0.03 mmole) were then added, and the reaction was initiated by addition of 0.5 mg Post Proline Specific Endoprotease. It was then stirred for two days at room temperature, hereafter approx. 10% conversion was achieved.
Yield according to HPLC: 42%
(Determined vs. hydrolysis at 254 nm)

| Analysis: | | |
|---|---|---|
| AAA: | Asp: | 1.05 |
| | Arg: | 0.94 |
| | Pro: | 0.98 |
| | Val: | 1.02 |

4) Synthesis of ZLArgProOBzl·AcOH
Reaction scheme: 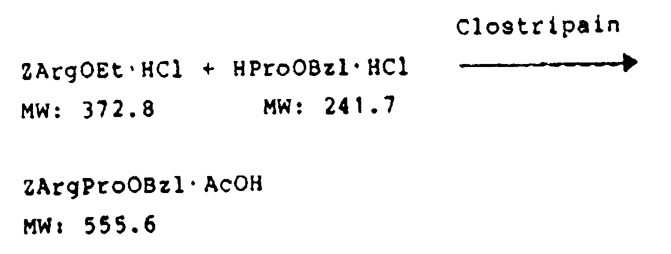 Procedure:
0.15 g ZArgOEt·HCl (0.4 mmole) and 0.48 g HProOBzl·HCl (2.0 mmole) were dissolved in 1.2 ml DMF and 2.8 ml H₂O containing 6 mg CaCl₂ 2H₂O and 1 mg DTT.
pH was adjusted to 8.5 using TEA. The reaction was initiated by addition of 2 mg preactivated Clostripain. After 1/2 hour approx. 10% conversion had been achieved and the reaction was stirred overnight at room temperature.
Yield according to HPLC: 31%
(Determined vs. hydrolysis at 254 nm.)
Analysis:
The product coeluted with a chemically synthesized reference-compound on analytical HPLC and showed the same relative UV-absorbance at 254 nm vs. 230 nm.
5) Synthesis of ZArgProAspValOEt
Reaction scheme: 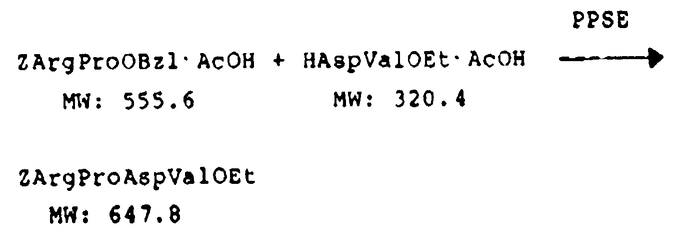 Procedure:
260 mg HAspValOEt·AcOH (0.8 mmole) were dissolved in 0.3 ml DMF and 1.5 ml H₂O and pH was adjusted to 8.5. 56 mg ZArgProOBzl·AcOH (0.1 mmole) were then added and the reaction was initiated by adding 0.5 mg Post Proline Specific Endoprotease. The reaction was stirred for 1 hour at room temperature after which 50% conversion was achieved.
Yield according to HPLC: 51%
(Determined vs. hydrolysis at 254 nm.)

| Analysis: | | |
|---|---|---|
| AAA: | Asp: | 1.00 |
| | Arg: | 1.02 |
| | Pro: | 1.00 |
| | Val: | 0.98 |

The product was base-labile.
6) Synthesis of Z-ArgProAspValTyrNH₂ and ZDArgProAspValTyrNH₂
The products formed in reactions 3) and 5) are purified by C₁₈-RP-HPLC and reacted with Tyrosine-amide in a CPD-Y catalysed reaction analogous to the one described in Example E,6.
Alternatively, direct coupling of AspValTyrNH₂ in a reaction analogous to reaction 5) yielded ZArgProAspValTyrNH₂ with the analysis below:

| Analysis: | | |
|---|---|---|
| AAA: | Asp: | 1.01 |
| | Arg: | 1.32 |
| | Pro: | 0.95 |
| | Tyr: | 0.86 |
| | Val: | 0.86 |

Example U:
==========

Synthesis of ZDargArgAspValTyrNH2 and
ZArgHomoArgAspValTyrNH2 according to strategy II
================================================

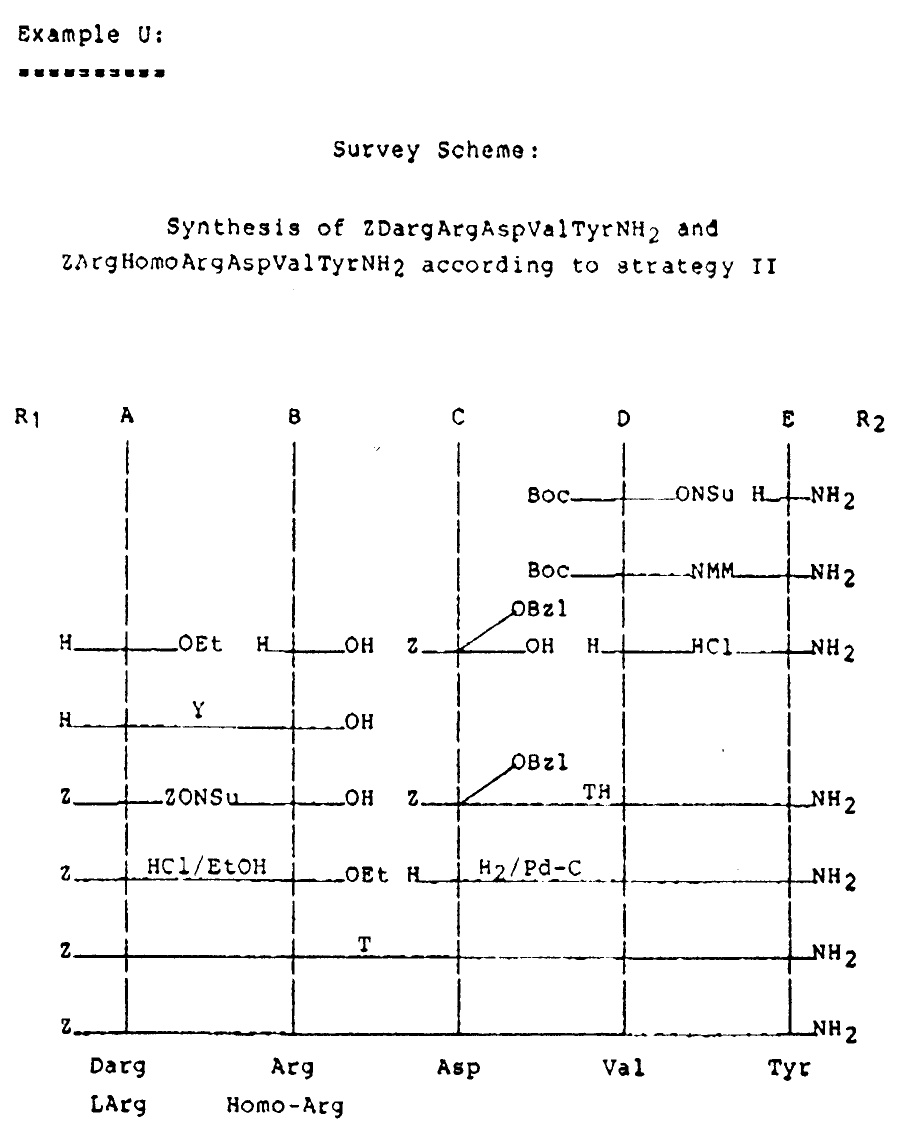
Example U:
==========

Survey Scheme:

Synthesis of ZDargArgAspValTyrNH2 and
ZArgHomoArgAspValTyrNH2 according to strategy II

R1 A B C D E R2


Boc ONSu H NH2
Boc NMM NH2
OBzl
H OEt H OH Z OH H HCl NH2
H Y OH
OBzl
Z ZONSu OH Z TH NH2
Z HCl/EtOH OEt H H2/Pd-C NH2
Z T NH2
Z NH2

Darg Arg Asp Val Tyr
LArg Homo-Arg

1) Synthesis of ZDargArgOEt·2HCl
a) Synthesis of H-D-argArgOH·2AcOH
   Reaction scheme: 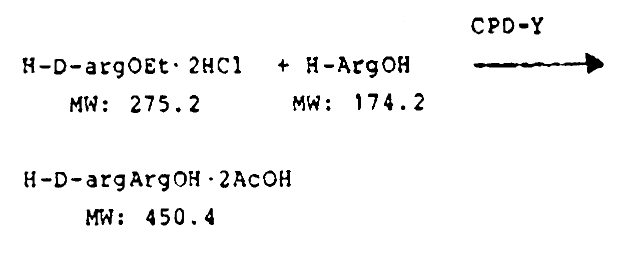 Procedure:
   3.04 g H-DArgOEt·2HCl (11.1 mmole) and 31.0 g HArgOH (178 mmole) were dissolved in 180 ml H₂O containing 80 mg EDTA. pH was adjusted to 9.0 using aqueous HCl-solution. The reaction was initiated by addition of 350 mg CPD-Y and was stirred for two days at room temperature. The reaction was then stopped by raising pH to 11, and the reaction mixture was then purified.
   Yield according to HPLC: 20%
   (Determined at 230 nm)
b) Purification of H-D-argArgOH·2AcOH
   The above-mentioned reaction mixture was purified on a DOWEX AG-50 kationexchange-column at basic pH using an acetate-buffer and a sodium chloride salt-gradient yielding one peptide homogenous in analytical HPLC in several product-fractions. The combined saltcontaining pure fractions, were concentrated under reduced pressure and when some of the salt was removed during filtration, they were finally taken to dryness for further processing.
c) Synthesis of ZDargArgOH·AcOH
   Reaction scheme: 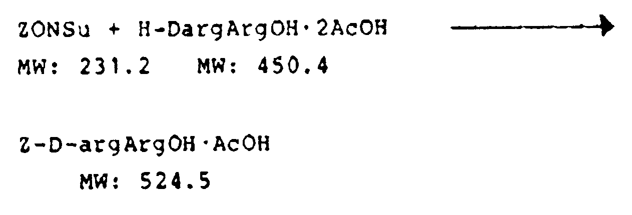 Procedure:
   The saltcontaining residue from b) was redissolved in water, and pH was adjusted to 8.2 using aqueous HCl-solution.
   0.56 g (2.4 mmole) of ZONSu dissolved in Acetonitrile were then added. The mixture was stirred vigorously at room temperature for 1 hour, while pH was kept constant. Finally, any excess reagent was destroyed by shortly raising pH to 10, after which it was lowered to 3.
d) Isolation of Z-D-argArgOH·AcOH
   The reaction mixture from c) was evaporated under reduced pressure and thereby removing Acetonitrile. It was then purified on C₁₈ RP-HPLC using an alcohol gradient in 50 mM HOAc. Combined fractions containing pure product were concentrated by evaporation under reduced pressure and finally freeze-dried.
   Yield: 0.64 g (55%)
   Analysis:
   AAA: Arginine identified
   The product coeluted on analytical HPLC with a reference sample of zDargArgOH prepared by standard chemical methods.
e) Synthesis of ZDargArgOEt·2HCl
   Reaction scheme: 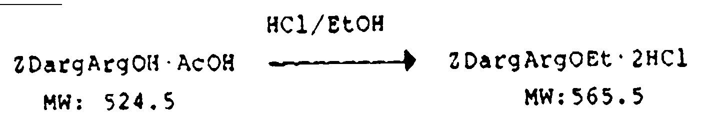 Procedure:
   The product from d) was dissolved in dry Ethanol containing 3 M of dry HCl. It was stirred at room temperature overnight, and finally evaporated to dryness with several repeated additions of dry Ethanol.
   Yield: 0.68 g (98%)

2) Synthesis of HAspValTyrNH₂
This compound was prepared as described in Example A,3.
3) Synthesis of ZDargArgAspValTyrNH₂ 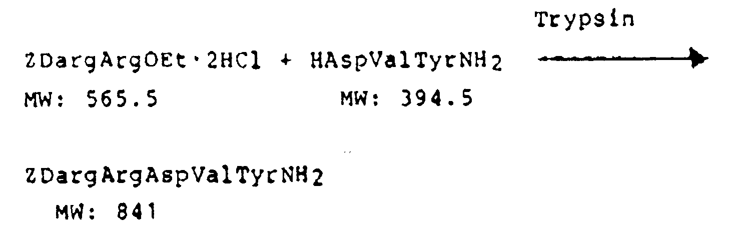 Procedure:
394 mg AspValTyrNH₂ (1.0 mmole) and 57 mg ZDargArgOEt· 2HCl (0.1 mmole) were dissolved in 0.6 ml of DMF and 1.3 ml H₂O, pH being adjusted to 8.7 by TEA and at 35°C. The reaction was initiated by addition of 0.3 mg Trypsin. The reaction was complete within 1 hour.
Yield according to HPLC: 43%
(Determined at 254 nm)

| Analysis: | | |
|---|---|---|
| AAA: | Asp: | 1.07 |
| | Arg: | 2.14 |
| | Tyr: | 0.90 |
| | Val: | 0.90 |

UV-absorbance at 278 nm.
4) Synthesis of HArgHomoArgOH
Reaction scheme: 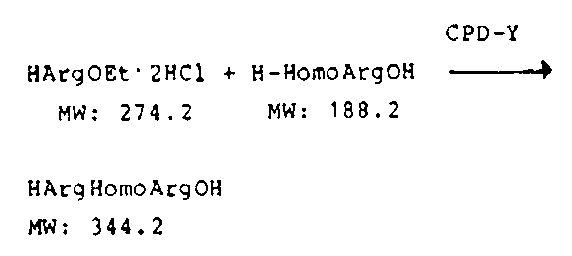 Procedure:
27 mg L-ArgOEt·2HCl (0.1 mmole) and 301 mg L-Homo-Arginine (1.6 mmole) were dissolved in 1.8 ml H₂O containing 4 mg EDTA, and pH was adjusted to 8.5 using aqueous NaOH-solution. The reaction was initiated by addition of 2 mg CPD-Y. The reaction was complete within 3 hours, and pH was adjusted to 3.
Yield according to HPLC: 11%
(Determined at 230 nm)

| Analysis: | | |
|---|---|---|
| AAA: | Arginine: | 0.85 |
| | Homo Arginine: | 1.15 |

5) Synthesis of ZArgHomoArgAspValTyrNH₂
The product of 4) is purified and further processed as described under 1), after which ZArgHomoArgOEt·2HCl is obtained. This is then reacted with AspValTyrNH₂ in a Trypsin-catalysed reaction according to the procedure described under 3), and ZArgHomoArgAspValTyrNH₂ is obtained.

R1 A B C D E R2
H OEt H OH Boc ONSu H NH2
H Y OH
1) ZONSu
2) HCl/EtOH
OEt
Z OEt H OEt Boc NMM NH2
OEt
Z T OEt H HCl NH2
OEt
Z CE OH H NH2
OEt
Z CHEMICAL NH2
Z 0.1 N NaOH NH2
DArg Arg Dasp Val Tyr

CE = Carboxylester Hydrolase

Example W:
==========

Example W:
==========

Survey Scheme:

Synthesis ofZDargArgDaspValTyrNH2
according to strategy IV

### Synthesis of ZDargArgDaspValTyrNH₂ according to strategy IV

1) Synthesis of ZDargArgOEt·2HCl
This compound was synthesized as described in Example U,1.
2) Synthesis of ZDargArgDasp(OEt)₂
Reaction scheme: 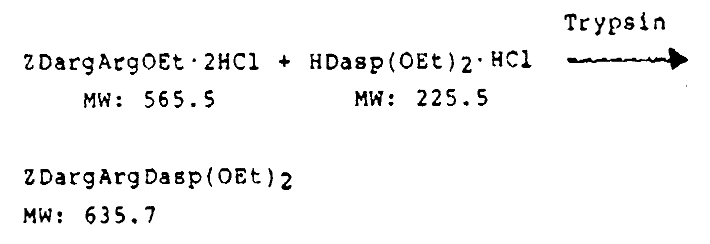 Procedure:
38 mg ZDargArgOEt·2HCl (0.07 mmole) and 20 mg H-Dasp(OEt)₂·HCl (0.9 mmole) were dissolved in 0.6 ml DMF and 1.4 ml H₂O, pH being adjusted to 8.5 by aqueous NaOH-solution. The reaction was initiated by addition of 0.5 mg Trypsin. The reaction was complete within 1/2 hour, and the reaction was stopped by lowering pH to 3.
Yield according to HPLC: 14%
(Determined at 254 nm)

| Analysis: | | |
|---|---|---|
| AAA: | Asp: | 1.01 |
| | Arg: | 1.99 |

3) Synthesis of HCl HValTyrNH₂
This is synthesized as described in Example A,2.
4) Synthesis of ZDargArgDaspValTyrNH₂
The product of 2) is treated with a suitable Carboxylester Hydrolase (e.g. Lipase) in aqueous solution near neutral pH.
The resulting ZDargArgDasp(OEt)OH is then isolated from the reaction mixture using ion-exchange chromatography or preferably C₁₈ RP-HPLC. From the combined pure product-fractions it is then freezedried in the presence of aqueous HCl in twice molar excess. The resulting ZDargArgDasp(OEt)OH·2HCl salt is then used as C-component in a standard chemical coupling mediated by DCC under HO-Bt catalysis, using HValTyrNH₂·HCl as amino-component with addition of equimolar amounts of reactants and NMM in DMF.
ZDargArgDasp(OEt)ValTyrNH₂ is isolated by C₁₈ RP-HPLC and treated with 0.1 N aqueous NaOH to give ZDargArgDaspValTyrNH₂.

## Claims

1. A process for producing peptides of the general formula
R₁-A-B-C-D-E-R₂
wherein A, B, C, D and E represent amino acid residues coupled with peptide bonds and
A is a basic amino acid residue in L- or D-configuration, which is ω-amino or ω-guanidino substituted, or optionally substituted desamino derivatives of such amino acids,
B is a basic L-amino acid residue which is ω-amino- or ω-guanidino substituted, or B is L-Pro or L-dehydroPro,
C is an acidic amino acid residue in L- or D-configuration,
D is an unpolar, hydrophobic aliphatic L-amino acid residue or a homo- or heteroaromatic aryl- or aralkyl group containing L-amino acid residue, optionally being nitro-, hydroxy-, alkoxy-, alkyl- and/or halogen substituted on the aryl group,
E is an unpolar, hydrophobic aliphatic amino acid residue in L- or D-configuration or a homo- or heteroaromatic aryl- or aralkyl group containing amino acid residue in L- or D-configuration, optionally being nitro-, hydroxy-, alkoxy-, alkyl- and/or halogen substituted on the aryl group, or optionally substituted decarboxy derivatives of such amino acids,
R₁ is H or an α-amino substitution group, such as a chemically or enzymatically cleavable α-amino protecting group, or is absent when A is an unsubstituted desamino derivative, and
R₂ is OH or an α-carboxy substituting group, such as a chemically or enzymatically cleavable α-carboxy protecting group, or is absent when E is an unsubstituted decarboxy derivative,
by sequential coupling of optionally protected reactive derivatives of said amino acids and/or by fragment coupling of optionally protected reactive di-, tri-, or tetrapeptide derivatives on the basis of said amino acids according to one or more type reactions suitably selected from A+B, C+D, D+E, AB+C, C+DE, CD+E, ABC+D, AB+CD, AB+CDE, ABC+DE, ABCD+E, and, if desired, removal of possible terminal and/or side chain protecting groups, the groups R₁ and R₂, if desired, independently of each other being introduced after the formation of the desired peptide ABCDE or during or after one of the preceding coupling reactions, **characterized** by using as an intermediate a dipeptide derivative Z₁-AB-Y₂ in which Z₁ is H or an α-amino substituting group which may be the same as or different from R₁, A and B have the above meaning and Y₂ is OH or an α-carboxy substituting group and their respective side chains are unprotected, said intermediate being reacted with a reactive amino acid derivative or peptide derivative selected from C(X₃)-Y₃, C(X₃)-D(X₄)-Y₄ and C(X₃)-D(X₄)-E(X₅)-Y₅, wherein C, D and E have the above meaning, Y₃, Y₄ and Y₅ are OH or an α-carboxy substituting group, which may be the same as or different from R₂, and X₃, X₄ and X₅ are H or a ω-side chain protecting group, in a completely or partly aqueous medium at pH 3-12, preferably at pH 5-10, in the presence, of a carboxylester hydrolase or peptide hydrolase enzyme selected from thiol endoproteases selected among bromelain, clostripain, papain, chymopapain and ficine, or serine endoproteases selected among Achromobacter lyticus protease I, thrombin, subtilisin, endoproteinase ArgC or LysC, trypsin and postprolin specific endopeptidase or carboxypeptidases selected among CPD-MI and preferably CPD-Y or CPD-MII or dipeptidyl peptidase I or dipeptidyl carboxypeptidase II, to form a peptide, said peptide, if desired, being coupled with a reactive amino acid derivative or peptide derivative in the presence of the same or another proteolytic enzyme capable of catalyzing the formation of a peptide bond between the reactants, and that, if necessary, further enzymatic or chemical coupling reactions selected among the above types are carried out to synthesize an optionally protected peptide derivative having the general formula
Z₁ - A - B - C(X₃) - D(X₄) - E(X₅) - Y₅
wherein A, B, C, D, Z₁, X₃, X₄, X₅ and Y₅ have the above meaning, the said substituting groups, if desired, being removed chemically or enzymatically, and the groups R₁ and/or R₂ being introduced, if necessary.

2. A process according to claim 1, **characterized** in that an intermediate Z₁-AB-Y₂ is used which has been prepared by reacting Z₁-A-Y₁ with H-B-Y₂ wherein Z₁, A, B and Y₂ have the meaning stated in claim 1 and Y₁ has the same meaning as Y₂, under the conditions stated in claim 1 in the presence of an enzyme selected from thiol endoproteases selected among bromelain, ficine or preferably clostripain, papain and chymopapain or serine endoproteases selected among Achromobacter lyticus protease I, endoproteinase ArgC or LysC, thrombin, subtilisin and preferably trypsin, dipeptidyl peptidase I or carboxypeptidases selected among CPD-MI or preferably CPD-Y and CPD-MII.

3. A process for producing peptides of the general formula
R₁-A-B-C-D-E-R₂
wherein A, B, C, D, E, R₁ and R₂ have the meaning stated in claim 1,
by sequential coupling of optionally protected reactive derivatives of said amino acids and/or by fragment coupling of optionally protected reactive di-, tri-, or tetrapeptide derivatives on the basis of said amino acids according to one or more type reactions suitably selected from A+B, B+C, D+E, AB+C, A+BC, C+DE, B+CDE, BC+DE, A+BCDE, AB+CDE, ABC+DE, and, if desired, removal of possible terminal and/or side chain protecting groups, the groups R₁ and R₂, if desired, independently of each other being introduced after the formation of the desired peptide ABCDE or during or after one of the preceding coupling reactions, **characterized** by using as an intermediate a dipeptide derivative D(X₄)-E(X₅)-Y₅ in which D, E and Y₅ have the above meaning, said intermediate being reacted with a reactive amino acid derivative or peptide derivative selected from Z₃-C(X₃)-Y₃, Z₂-B(X₂)-C(X₃)-Y₃ and Z₁-A(X₁)-B(X₂)-C(X₃)-Y₃, wherein A, B, C, Z₁, Z₃, X₃ and Y₃ have the above meaning, Z₂ is H or an α-amino substituting group, and X₁ and X₂ are H or a ω-side chain protecting group, in a completely or partly aqueous medium at pH 3-12, preferably at pH 5-10, in the presence of a carboxylester hydrolase or peptide hydrolase enzyme selected from metalloproteinases, preferably thermolysin, serine endoproteases selected among subtilisin, thermitase or preferably drapeau V8 or chymotrypsin, carboxypeptidases selected among CPD-Y and CPD-MII, or dipeptidyl carboxypeptidase II, to form a peptide, said peptide, if desired, being coupled with a reactive amino acid derivative or peptide derivative in the presence of the same or another proteolytic enzyme capable of catalyzing the formation of a peptide bond between the reactants, and that, if necessary, further enzymatic or chemical coupling reactions selected among the above types are carried out to synthesize an optionally protected peptide derivative having the general formula
Z₁ - A(X₁) - B(X₂) - C(X₃) - D(X₄) - E(X₅) - Y₅
wherein A, B, C, D, Z₁, X₁, X₂, X₃ and Y₅ have the above meaning, the said substituting groups, if desired, being removed chemically or enzymatically, and the groups R₁ and/or R₂ being introduced, if necessary.

4. A process according to any of the claims 1-3, **characterized** in that compounds are produced wherein the ω-substituted basic amino acids A or B have the general formula
R₁'R₂'N - (CH₂)ₙ - CH(NH₂)COOH (I)
or 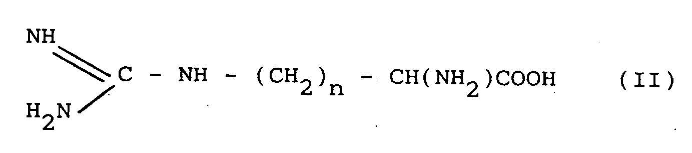 in which formulae n is 1-7, and R₁' and R₂' independently denote H, C₁-C₃ alkyl or pyranosyl, or R₁' and R₂' together form a group (CH₂)_{n'} where n' is 3-8, and A may furthermore be in D-form and/or be a desamino derivative of the said amino acids, which, if desired, may be substituted with C₁-C₄ alkyl, hydroxy C₁-C₄ alkyl or halogen C₁-C₄ alkyl,
the acidic amino acids C are ω-carboxy substituted amino acids having the general formula
HOOC - (CH₂)ₘ - CH(NH₂)COOH (III),
wherein m=0-7
or contain an additional carboxy substituent or ω-sulphonic acid group or phosphonic acid group instead of the ω-carboxy group in formula III,
the unpolar hydrophobic aliphatic amino acids D have the general formula
R₁ R₂ R₃ C - - CH(NH₂)COOH (IV),
wherein
R₁, R₂ and R₃ independently denotes H- or straight chain or branched alkyl or
D is an L-amino acid having the general formula
Ar(CH₂)ₚ - CH(NH₂)COOH (V),
wherein
p is 0-5 and Ar is an optionally nitro-, hydroxy-, alkoxy- and/or halogen substituted hetero- or homoaryl group,
the amino acids E independently may have the same meaning as D, but both in L- and D-configuration, or decarboxy derivatives of the said amino acids which, if desired, may be substituted with C₁-C₄ alkyl, hydroxy C₁-C₄ alkyl or halogen substituted, preferably fluorine substituted derivatives thereof, or nitro-, alkoxy-, hydroxy- and/or halogen substituted Trp, Phe or His, and R₁ and R₂ have the meaning stated in claim 1.

5. A process according to claim 4, **characterized** in that peptides are produced wherein
A is Arg, Lys, Orn, Homo-Arg or Homo-Lys in L- or D-configuration,
B is Arg, Lys, Orn, Homo-Arg, Homo-Lys or Pro in L-configuration,
C is Asp, Glu or Homo-Glu in L- or D-configuration,
D is Val, Ile, Leu, Nor-Leu, Nor-Val, Tert-Leu, Phe, Tyr or Ala in L-configuration,
E is Val, Ile, Leu, Nor-Leu, Nor-Val, Tert-Leu, Tyr, Trp, Phe, His or Nal in L- or D-configuration, or decarboxy derivatives thereof, or nitro-, alkoxy, hydroxy- and/or halogen substituted Trp, Phe or His, and
R₁ and R₂ have the meaning stated in claim 1.

6. A process according to claim 1 or 3, **characterized** by subjecting the dipeptide derivative Z₁-AB-Y₂ as carboxy component to a fragment coupling with an optionally protected tripeptide derivative C(X₃)-D(X₄)-E(X₅)-Y₅ as amine component, in which formulas the symbols used have the meaning stated in claim 1, in the presence of an endoprotease or dipeptidyl peptidase capable of catalyzing the formation of a peptide bond between the components, and that possible protecting groups present, if desired, are removed, and the groups R₁ and/or R₂ are introduced, if necessary.

7. A process according to claim 6, **characterized** in that the tripeptide derivative C(X₃)-D(X₄)-E(X₅)-Y₅ used has been obtained by coupling an amino acid derivative Z₃-C(X₃)-Y₃ with a dipeptide derivative D(X₄)-E(X₅)-Y₅ in the presence of an endoprotease or a dipeptidyl carboxypeptidase.

8. A process according to claim 1 or 3, **characterized** in that an optionally protected tripeptide derivative Z₁-A(X₁)-B(X₂)-C(X₃)-Y₃ as carboxy component is subjected to a fragment coupling with an optionally protected dipeptide derivative Z₄-D(X₄)-E(X₅)-Y₅ as amine component, in which formulae the symbols used have the meaning stated in claim 1, in the presence of an endoprotease, a lipase or dipeptidyl carboxypeptidase capable of catalyzing the formation of a peptide bond between the components, and that possible protecting groups present, if desired, are removed, and the groups R₁ and/or R₂ are introduced, if necessary.

9. A process according to claim 8, **characterized** in that the tripeptide derivative Z₁-A(X₁)-B(X₂)-C(X₃)-Y₃ used has been obtained by coupling an amino acid derivative Z₁-A(X₁)-Y₁ with a dipeptide derivative B(X₂)-C(X₃)-Y₃ in the presence of an endoprotease or a dipeptidyl peptidase or by coupling a dipeptide derivative Z₁-A(X₁)-B(X₂)-Y₂ with an amino acid derivative Z₃-C(X₃)-Y₃ in the presence of an endoprotease or a carboxypeptidase.

10. A process according to claim 1, **characterized** in that an optionally protected tetrapeptide derivative Z₁-A-B-C(X₃)-D(X₄)-Y₄ as carboxy component is coupled with an optionally protected amino acid derivative E(X₅)-Y₅ as amine component, in which formulae the symbols used have the meaning stated in claim 1, in the presence of an endoprotease, lipase or carboxypeptidase capable of catalyzing the formation of a peptide bond between the components, and that possible protecting groups present, if desired, are removed, and the groups R₁ and/or R₂ are introduced, if necessary.

11. A process according to claim 10, **characterized** in that the tetrapeptide derivative Z₁-A-B-C(X₃)-D(X₄)-Y₄ used has been obtained by fragment coupling of the dipeptide derivatives Z₁-A-B-Y₂ and C(X₃)-D(X₄)-Y₄ in the presence of an endoprotease or a dipeptidyl peptidase or a dipeptidyl carboxypeptidase or by coupling a tripeptide derivative Z₁-A-B-C(X₃)-Y₃ with an amino acid derivative D(X₄)-Y₄ in the presence of an endoprotease or a carboxypeptidase.

12. A process according to any of the claims 1-11, **characterized** in that a thiol endoprotease, a serine endoprotease, a carboxypeptidase, a dipeptidase or a dipeptidyl peptidase is used or has been used to form the bond between A and B.

13. A process according to any of the claims 1-12, **characterized** in that a thiol endoprotease, a serine endoprotease, a carboxypeptidase, a dipeptidase, a dipeptidyl peptidase or a dipeptidyl carboxypeptidase is used or has been used to form the bond between B and C.

14. A process according to any of the claims 1-13, **characterized** in that a serine endoprotease, a metalloendoprotease, an aspartate protease, a carboxypeptidase, a dipeptidase, an esterase, a lipase or a dipeptidyl carboxypeptidase is used or has been used to form the bond between C and D.

15. A process according to any of the claims 1-14, **characterized** in that a thiol endoprotease, a serine endoprotease, a metalloendoprotease, a carboxypeptidase, a dipeptidase, an esterase or a lipase is used or has been used to form the bond between D and E.

16. A process according to claim 15, **characterized** in that use is made or has been made of thermolysin or a thiol endoprotease selected among chymopapain, ficin or preferably papain or bromelain, a serine endoprotease selected among thermitase or preferably subtilisin, elastase, valylproteinase or chymotrypsin, or a carboxypeptidase selected among CPD-MII or preferably CPD-Y.

17. A process according to any of the claims 1-16, **characterized** in that a thiol endoprotease, a serine endoprotease, a metalloendoprotease, an aspartate protease, a carboxypeptidase, an esterase or a lipase is used or has been used to form the bond between E and R₂.

18. A process according to claim 17, **characterized** in that use is made or has been made of a thiol endoprotease selected from papain and chymopapain, or a serine endoprotease selected from subtilisin, thermitase or preferably chymotrypsin, or a carboxypeptidase selected from CPD-B, CPD-MII and CPD-Y.

19. A process according to any of the claims 1-18, **characterized** in that a thiol endoprotease, a serine endoprotease, an aminopeptidase or a penicillium acylase is used or has been used to form the bond between R₁ and A.

20. A process according to claim 19, **characterized** in that use is made or has been made of a thiol endoprotease selected from papain and chymopapain, a serine endoprotease selected from trypsin, chymotrypsin, postprolin specific endoprotease, Achromobacter lyticus protease I, endoproteinase ArgC and LysC and thrombin, or an aminopeptidase selected from pyroglutaminase, proliniminopeptidase or N-acyl peptide hydrolase or penicillium V or G acylase.

21. A protected dipeptide, **characterized** in that it is Z₁ABOR, wherein Z₁, A and B have the meaning stated in claim 1 except that A and B cannot both be L-Arg, D- or L-Lys, L-Orn or D- or L-Dab, and when A is L-Orn B cannot be L-Arg and when A is L-Lys B cannot be L-Dab, and when AB is L-Arg-L-Lys or L-Lys-L-Arg Z₁ cannot be H, N-lauroyl or N-palmitoyl, and when AB is L-Arg-L-Pro Z₁ cannot be Fmoc and R denotes straight chain or branched C₁₋₆ alkyl or optionally substituted benzyl.

22. A protected dipeptide according to claim 21, **characterized** in that it is Z₁A'B'OR, wherein A' is L- or D-Arg or L-Orn and B' is L-Lys or L-Pro and Z₁ and R have the meaning stated in claim 21.

23. A protected dipeptide according to claim 22, **characterized** in that it is CbzArgLysOR, wherein R has the meaning stated in claim 21.

24. A protected tripeptide, **characterized** in that it is Z₁ABC(OR')(OR''), wherein Z₁, A, B and C have the meaning stated in claim 1 except that A and B cannot both be Lys and C is different from Asp and R' and R'' independently have the meaning of R stated in claim 21.

25. A protected tripeptide according to claim 25, **characterized** in that it is CbzArgLysGlu(OR)₂ where R has the meaning stated in claim 21.

26. A protected tripeptide, **characterized** in that it is Z₁ABC(OR)OH, where Z₁, A, B, C and R have the meaning stated in claim 24.

27. A protected tripeptide according to claim 26, **characterized** in that it is CbzArgLysGlu(OR)OH, where R has the meaning stated in claim 21.

28. A protected tetrapeptide, **characterized** in that it is CbzArgLysC'(OR')D(OR'''), wherein C' is Asp or Glu, D has the meaning stated in claim 5, R' and R''' are C₁-C₆ alkyl or benzyl and R''' may also be H.

## Patentansprüche

1. Verfahren zur Herstellung von Peptiden der allgemeinen Formel
R₁―A―B―C―D―E―R₂
worin A, B, C, D und E Aminosäurereste darstellen, die durch Peptidbindungen miteinander verbunden sind, und
A einen basischen, ω-amino- oder ω-guanidinosubstituierten Aminosäurerest mit L- oder D-Konfiguration oder ein gegebenenfalls substituiertes Desaminoderivat einer solchen Aminosäure darstellt,
B einen basischen, ω-amino- oder ω-guanidinosubstituierten L-Aminosäurerest oder L-Pro oder L-dehydroPro darstellt,
C einen sauren Aminosäurerest mit L- oder D-Konfiguration darstellt
D einen unpolaren, hydrophoben, aliphatischen L-Aminosäurerest oder einen L-Aminosäurerest, der eine homo- oder heteroaromatische Aryl- oder Aralkylgruppe enthält, wobei die Arylgruppe gegebenenfalls nitro-, hydroxy-, alkoxy-, alkyl- und/oder halogensubstituiert ist, darstellt,
E einen unpolaren, hydrophoben, aliphatischen Aminosäurerest mit L- oder D-Konfiguration oder einen Aminosäurerest mit L- oder D-Konfiguration, der eine homo- oder heteroaromatische Aryl- oder Aralkylgruppe enthält, wobei die Arylgruppe gegebenenfalls nitro-, hydroxy-, alkoxy-, alkyl- und/oder halogensubstituiert ist, oder ein gegebenenfalls substituiertes Decarboxyderivat einer solchen Aminosäure darstellt,
R₁ ein Wasserstoffatom oder eine α-Aminosubstitutionsgruppe, wie zum Beispiel eine chemisch oder enzymatisch spaltbare α-Aminoschutzgruppe, darstellt oder fehlt, wenn A ein unsubstituiertes Desaminoderivat darstellt, und
R₂ OH oder eine α-Carboxysubstitutionsgruppe, wie zum Beispiel eine chemisch oder enzymatisch spaltbare α-Carboxyschutzgruppe, darstellt oder fehlt, wenn E ein unsubstituiertes Decarboxyderivat darstellt,
durch sequentielles Kuppeln gegebenenfalls geschützter, reaktiver Derivate der genannten Aminosäuren und/oder durch Fragmentkuppeln von gegebenenfalls geschützten, reaktiven Di-, Tri- oder Tetrapeptidderivaten auf Grundlage der genannten Aminosäuren gemäß einer oder mehrerer Arten von Reaktionen, die geeignet ausgewählt sind aus A+B, C+D, D+E, AB+C, C+DE, CD+E, ABC+D, AB+CD, AB+CDE, ABC+DE und ABCD+E, und, wenn gewünscht, Entfernen möglicher endständiger und/oder in den Seitenketten angebrachter Schutzgruppen, wobei die Gruppen R₁ und R₂, wenn gewünscht, unabhängig voneinander nach Bildung des gewünschten Peptides ABCDE oder während oder nach einer der vorausgehenden Kupplungsreaktionen eingeführt werden,
dadurch gekennzeichnet, daß
als Zwischenstufe ein Dipeptidderivat Z₁―AB―Y₂ verwendet wird, bei dem Z₁ ein Wasserstoffatom oder eine α-Aminosubstitutionsgruppe darstellt, die gleich oder verschieden sein kann von R₁, A und B die vorstehend genannten Bedeutungen haben und Y₂ OH oder eine α-Carboxysubstitutionsgruppe darstellt und ihre entsprechenden Seitenketten ungeschützt sind, wobei die Zwischenstufe mit einem reaktiven Aminosäurederivat, ausgewählt aus C(X₃)―Y₃, C(X₃)―D(X₄)―Y₄ und C(X₃)―D(X₄)―E(X₅)―Y₅, worin C, D und E die vorstehend genannten Bedeutungen haben, Y₃, Y₄ und Y₅ OH oder eine α-Carboxysubstitutionsgruppe darstellen, die gleich oder verschieden sein kann von R₂, und X₃, X₄ und X₅ ein Wasserstoffatom oder eine ω-Seitenkettenschutzgruppe darstellen, in einem völlig oder teilweise wäßrigen Medium bei einem pH-Wert von 3 bis 12 und bevorzugt bei einem pH-Wert von 5 bis 10 in der Gegenwart eines Carboxylesterhydrolaseenzyms oder eines Peptidhydrolaseenzyms, ausgewählt aus den Thiolendoproteasen und darunter ausgewählt aus Bromelain, Clostripain, Papain, Chymopapain und Ficin, oder ausgewählt aus den Serinendoproteasen und darunter ausgewählt aus Achromobacter-lyticus-Protease I, Thrombin, Subtilisin, Endoproteinase ArgC oder LysC und für Trypsin und Postprolin spezifischer Endopeptidase oder ausgewählt aus den Carboxypeptidasen und darunter ausgewählt aus CPD-MI und bevorzugt CPD-Y oder CPD-MII oder ausgewählt aus Dipeptidylpeptidase I oder Dipeptidylcarboxypeptidase II, umgesetzt wird, um ein Peptid zu bilden, wobei das Peptid, wenn gewünscht, mit einem reaktiven Aminosäurederivat oder Peptidderivat in der Gegenwart des gleichen oder eines anderen proteolytischen Enzyms, das in der Lage ist, die Bildung einer Peptidbindung zwischen den Reaktanten zu katalysieren, gekuppelt werden kann und daß, wenn erforderlich, weitere enzymatische oder chemische Kupplungsreaktionen, die aus den genannten Arten ausgewählt sind, durchgeführt werden, um ein gegebenenfalls geschütztes Peptidderivat zu synthetisieren, das die allgemeine Formel
Z₁―A―B―C(X₃)―D(X₄)―E(X₅)―Y₅
besitzt, worin A, B, C, D, Z₁, X₃, X₄, X₅ und Y₅ die vorstehend genannten Bedeutungen besitzen, wobei die Substitutionsgruppen, wenn gewünscht, chemisch oder enzymatisch entfernt werden und die Gruppen R₁ und/oder R₂, wenn erforderlich, eingeführt werden.

2. Verfahren nach Anspruch 1, dadurch gekennzeichnet, daß eine Zwischenstufe Z₁―AB―Y₂ verwendet wird, die hergestellt wurde durch Umsetzen von Z₁―A―Y₁ mit H―B―Y₂, worin Z₁, A, B und Y₂ die Bedeutung haben, die in Anspruch 1 genannt wurde, und Y₁ die gleiche Bedeutung wie Y₂ hat, unter den Bedingungen, die in Anspruch 1 genannt sind, in der Gegenwart eines Enzyms, das ausgewählt ist aus den Thiolendoproteasen und darunter ausgewählt aus Bromelain, Ficin oder bevorzugt Clostripain, Papain und Chymopapain oder ausgewählt ist aus den Serinendoproteasen und darunter ausgewählt aus Achromobacter-lyticus-Protease I, Endoproteinase ArgC oder LysC, Thrombin, Subtilisin und bevorzugt Trypsin, Dipeptidylpeptidase I oder ausgewählt ist aus den Carboxypeptidasen und darunter ausgewählt aus CPD-MI oder bevorzugt CPD-Y und CPD-MII.

3. Verfahren zur Herstellung von Peptiden der allgemeinen Formel
R₁―A―B―C―D―E―R₂
worin A, B, C, D, E, R₁ und R₂ die Bedeutungen haben, die in Anspruch 1 genannt wurden,
durch sequentielles Kuppeln gegebenenfalls geschützter, reaktiver Derivate der genannten Aminosäuren und/oder durch Fragmentkuppeln von gegebenenfalls geschützten, reaktiven Di-, Tri- oder Tetrapeptidderivaten auf Grundlage der genannten Aminosäuren gemäß einer oder mehrerer Arten von Reaktionen, die geeignet ausgewählt sind aus A+B, B+C, D+E, AB+C, A+BC, C+DE, B+CDE, BC+DE, A+BCDE, AB+CDE und ABC+DE, und, wenn gewünscht, Entfernen möglicher endständiger und/oder in den Seitenketten angebrachter Schutzgruppen, wobei die Gruppen R₁ und R₂, wenn gewünscht, unabhängig voneinander nach Bildung des gewünschten Peptides ABCDE oder während oder nach einer der vorausgehenden Kupplungsreaktionen eingeführt werden,
dadurch gekennzeichnet, daß
als Zwischenstufe ein Dipeptidderivat D(X₄)―E(X₅)―Y₅ verwendet wird, bei dem D, E und Y₅ die vorstehend genannten Bedeutungen haben, wobei die Zwischenstufe mit einem reaktiven Aminosäurederivat oder Peptidderivat, ausgewählt aus Z₃―C(X₃)―Y₃, Z₂―B(X₂)―C(X₃)―Y₃ und Z₁―A(X₁)―B(X₂)―C(X₃)―Y₃, worin A, B, C, Z₁, Z₃, X₃ und Y₃ die vorstehend genannten Bedeutungen haben, Z₂ ein Wasserstoffatom oder eine α-Aminosubstitutionsgruppe darstellt und X₁ und X₂ ein Wasserstoffatom oder eine ω-Seitenkettenschutzgruppe darstellen, in einem völlig oder teilweise wäßrigen Medium bei einem pH-Wert von 3 bis 12 und bevorzugt bei einem pH-Wert von 5 bis 10 in der Gegenwart eines Carboxylesterhydrolaseenzyms oder eines Peptidhydrolaseenzyms, ausgewählt aus den Metalloproteinasen, bevorzugt Thermolysin, den Serinendoproteasen, ausgewählt aus Subtilisin, Thermitase oder bevorzugt Drapeau V8 oder Chymotrypsin, Carboxypeptidasen, ausgewählt aus CPD-Y und CPD-MII, oder Dipeptidylcarboxypeptidase II, umgesetzt wird, um ein Peptid zu bilden, wobei das Peptid, wenn gewünscht, mit einem reaktiven Aminosäurederivat oder Peptidderivat in der Gegenwart des gleichen oder eines anderen proteolytischen Enzyms, das in der Lage ist, die Bildung einer Peptidbindung zwischen den Reaktanten zu katalysieren, gekuppelt werden kann und daß, wenn erforderlich, weitere enzymatische oder chemische Kupplungsreaktionen, die aus den genannten Arten ausgewählt sind, durchgeführt werden, um ein gegebenenfalls geschütztes Peptidderivat zu synthetisieren, das die allgemeine Formel
Z₁―A(X₁)―B(X₂)―C(X₃)―D(X₄)―E(X₅)―Y₅
besitzt, worin A, B, C, D, Z₁, X₁, X₂, X₃ und Y₅ die vorstehend genannten Bedeutungen besitzen, wobei die Substitutionsgruppen, wenn gewünscht, chemisch oder enzymatisch entfernt werden und die Gruppen R₁ und/oder R₂, wenn erforderlich, eingeführt werden.

4. Verfahren nach einem der Ansprüche 1 bis 3, dadurch gekennzeichnet, das Verbindungen hergestellt werden, worin die ω-substituierten, basischen Aminosäuren A oder B die allgemeine Formel
R₁'R₂'N―(CH₂)ₙ―CH(NH₂)COOH (I)
oder 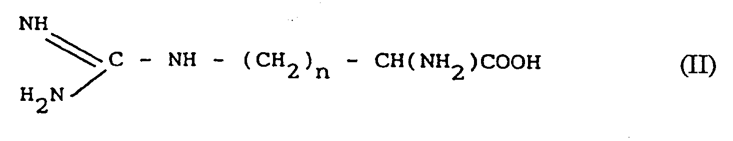 besitzen, wobei in den Formeln n 1 bis 7 ist und R₁' und R₂' unabhängig voneinander ein Wasserstoffatom, einen Alkylrest mit 1 bis 3 Kohlenstoffatomen oder einen Pyranosylrest darstellen oder R₁' und R₂' gemeinsam eine Gruppe (CH₂)_{n'} bilden, worin n' 3 bis 8 ist,
A weiter in der D-Form und/oder als ein Desaminoderivat der genannten Aminosäuren vorliegen und, wenn gewünscht, mit Alkylresten mit 1 bis 4 Kohlenstoffatomen, Hydroxyalkylresten mit 1 bis 4 Kohlenstoffatomen oder Halogenalkylresten mit 1 bis 4 Kohlenstoffatomen substituiert werden kann,
die sauren Aminosäuren C ω-carboxysubstituierte Aminosäuren mit der allgemeinen Formel
HOOC―(CH₂)ₘ―CH(NH₂)COOH (III),
worin m 0 bis 7 ist
sind oder einen zusätzlichen Carboxysubstituenten oder eine ω-Sulfonsäuregruppe oder ω-Phosphonsäuregruppe an Stelle der ω-Carboxygruppe in Formel III enthalten,
die unpolaren, hydrophoben, aliphatischen Aminosäuren D die allgemeine Formel
R₁R₂R₃C―CH(NH₂)COOH (IV)
besitzen, worin R₁, R₂ und R₃ unabhängig voneinander ein Wasserstoffatom oder eine geradkettige oder verzweigte Alkylgruppe bedeuten, oder D eine L-Aminosäure mit der allgemeinen Formel
Ar(CH₂)ₚ―CH(NH₂)COOH (V)
ist, worin p 0 bis 5 ist und Ar eine gegebenenfalls nitro-, hydroxy-, alkoxy- und/oder halogensubstituierte Hetero- oder Homoarylgruppe ist,
die Aminosäuren E unabhängig voneinander die gleiche Bedeutung wie D haben können, aber sowohl in der L- als auch der D-Konfiguration vorliegen können, oder Decarboxyderivate der genannten Aminosäuren sind, die, wenn gewünscht, mit Alkylgruppen mit 1 bis 4 Kohlenstoffatomen oder Hydroxyalkylgruppen mit 1 bis 4 Kohlenstoffatomen substituiert sein können oder halogensubstituierte und bevorzugt fluorsubstituierte Derivate dieser Aminosäuren sein können oder nitro-, alkoxy-, hydroxy- und/oder halogensubstituiertes Trp, Phe oder His sein können und R₁ und R₂ die Bedeutungen haben, die in Anspruch 1 genannt wurden.

5. Verfahren nach Anspruch 4, dadurch gekennzeichnet, daß Peptide hergestellt werden, worin
A Arg, Lys, Orn, Homo-Arg oder Homo-Lys in der L- oder D-Konfiguration ist,
B Arg, Lys, Orn, Homo-Arg, Homo-Lys oder Pro in der L-Konfiguration ist,
C Asp, Glu oder Homo-Glu in der L- oder D-Konfiguration ist,
D Val, Ile, Leu, Nor-Leu, Nor-Val, Tert-Leu, Phe, Tyr oder Ala in der L-Konfiguration ist,
E Val, Ile, Leu, Nor-Leu, Nor-Val, Tert-Leu, Tyr, Trp, Phe, His oder Nal in der L- oder D-Konfiguration oder ein Decarboxyderivat davon oder nitro-, alkoxy-, hydroxy- und/oder halogensubstituiertes Trp, Phe oder His ist, und
R₁ und R₂ die Bedeutung haben, die in Anspruch 1 genannt ist.

6. Verfahren nach Anspruch 1 oder Anspruch 3, dadurch gekennzeichnet, daß das Dipeptidderivat Z₁-AB-Y₂ als Carboxykomponente einer Fragmentkupplung mit einem gegebenenfalls geschützten Tripeptidderivat
C(X₃)―D(X₄)―E(X₅)―Y₅ als Aminkomponente, wobei in den Formeln die verwendeten Symbole die Bedeutungen haben, die in Anspruch 1 genannt wurden, in der Gegenwart einer Endoprotease oder einer Dipeptidylpeptidase, die in der Lage ist, die Bildung einer Peptidbindung zwischen den Komponenten zu katalysieren, unterzogen wird und das möglicherweise vorhandene Schutzgruppen, wenn gewünscht, entfernt werden und die Gruppen R₁ und/oder R₂, wenn erforderlich, eingeführt werden.

7. Verfahren nach Anspruch 6, dadurch gekennzeichnet, daß das verwendete Tripeptidderivat C(X₃)―D(X₄)―E(X₅)―Y₅ durch Kupplung eines Aminosäurederivates Z₃―C(X₃)―Y₃ mit einem Dipeptidderivat D(X₄)―E(X₅)―Y₅ in der Gegenwart einer Endoprotease oder Dipeptidylcarboxypeptidase erhalten wurde.

8. Verfahren nach Anspruch 1 oder Anspruch 3, dadurch gekennzeichnet, daß ein gegebenenfalls geschütztes Tripeptidderivat Z₁―A(X₁)―B(X₂)―C(X₃)―Y₃ als Carboxykomponente einer Fragmentkupplung mit einem gegebenenfalls geschützten Dipeptidderivat Z₄―D(X₄)―E(X₅)―Y₅ als Aminkomponente, wobei in den Formeln die verwendeten Symbole die Bedeutungen haben, die in Anspruch 1 genannt wurden, in der Gegenwart einer Endoprotease, einer Lipase oder einer Dipeptidylcarboxypeptidase, die in der Lage ist, die Bildung einer Peptidbindung zwischen den Komponenten zu katalysieren, unterzogen wird und daß möglicherweise anwesende Schutzgruppen, wenn gewünscht, entfernt werden und die Gruppen R₁ und/oder R₂, wenn erforderlich, eingeführt werden.

9. Verfahren nach Anspruch 8, dadurch gekennzeichnet, daß das verwendete Tripeptidderivat Z₁―A(X₁)―B(X₂)―C(X₃)―Y₃ erhalten wurde durch Kupplung eines Aminosäurederivates Z₁―A(X₁)―Y₁ mit einem Dipeptidderivat B(X₂)―C(X₃)―Y₃ in der Gegenwart einer Endoprotease oder Dipeptidylpeptidase oder durch Kuppeln eines Dipeptidderivates Z₁―A(X₁)―B(X₂)―Y₂ mit einem Aminosäurederivat Z₃―C(X₃)―Y₃ in Gegenwart einer Endoprotease oder einer Carboxypeptidase erhalten wurde.

10. Verfahren nach Anspruch 1, dadurch gekennzeichnet, daß ein gegebenenfalls geschütztes Tetrapeptidderivat Z₁―A―B―C(X₃)―D(X₄)―Y₄ als Carboxykomponente mit einem gegebenenfalls geschützten Aminosäurederivat E(X₅)―Y₅ als Aminkomponente, wobei in den Formeln die verwendeten Symbole die Bedeutungen haben, die in Anspruch 1 genannt wurden, in der Gegenwart einer Endoprotease, Lipase oder Carboxypeptidase, die in der Lage ist, die Bildung einer Peptidbindung zwischen den Komponenten zu katalysieren, gekuppelt wird und daß möglicherweise vorhandene Schutzgruppen, wenn gewünscht, entfernt werden, und die Gruppen R₁ und/oder R₂, wenn erforderlich, eingeführt werden.

11. Verfahren nach Anspruch 10, dadurch gekennzeichnet, daß das verwendete Tetrapeptidderivat Z₁―A―B―C(X₃)―D(X₄)―Y₄ erhalten wurde durch Fragmentkupplung der Dipeptidderivate Z₁―A―B―Y₂ und C(X₃)―D(X₄)―Y₄ in der Gegenwart einer Endoprotease oder einer Dipeptidylpeptidase oder einer Dipeptidylcarboxypeptidase oder durch Kupplung eines Tripeptidderivates Z₁―A―B―C(X₃)―Y₃ mit einem Aminosäurederivat D(X₄)―Y₄ in der Gegenwart einer Endoprotease oder einer Carboxypeptidase.

12. Verfahren nach einem der Ansprüche 1 bis 11, dadurch gekennzeichnet, daß eine Thiolendoprotease, eine Serinendoprotease, eine Carboxypeptidase, eine Dipeptidase oder eine Dipeptidylpeptidase verwendet wird oder verwendet wurde, um die Bindung zwischen A und B zu bilden.

13. Verfahren nach einem der Ansprüche 1 bis 12, dadurch gekennzeichnet, daß eine Thiolendoprotease, eine Serinendoprotease, eine Carboxypeptidase, eine Dipeptidase, eine Dipeptidylpeptidase oder Dipeptidylcarboxypeptidase verwendet wird oder verwendet wurde, um die Bindung zwischen B und C zu bilden.

14. Verfahren nach einem der Ansprüche 1 bis 13, dadurch gekennzeichnet, daß eine Serinendoprotease, ein Metalloendoprotease, eine Aspartatprotease, eine Carboxypeptidase, eine Dipeptidase, eine Esterase, eine Lipase oder eine Dipeptidylcarboxypeptidase verwendet wird oder verwendet wurde, um die Bindung zwischen C und D zu bilden.

15. Verfahren nach einem der Ansprüche 1 bis 14, dadurch gekennzeichnet, daß eine Thiolendoprotease, eine Serinendoprotease, eine Metalloendoprotease, eine Carboxypeptidase, eine Dipeptidase, eine Esterase oder eine Lipase verwendet wird oder verwendet wurde, um die Bindung zwischen D und E zu bilden.

16. Verfahren nach Anspruch 15, dadurch gekennzeichnet, daß Thermolysin oder eine Thiolendoprotease, ausgewählt aus Chymopapain, Ficin oder bevorzugt Papain oder Bromelain, eine Serinendoprotease, ausgewählt aus Thermitase oder bevorzugt Subtilisin, Elastase, Valylproteinase oder Chymotrypsin, oder eine Carboxypeptidase, ausgewählt aus CPD-MII oder bevorzugt CPD-Y verwendet wird oder verwendet wurde.

17. Verfahren nach einem der Ansprüche 1 bis 16, dadurch gekennzeichnet, daß eine Thiolendoprotease, eine Serinendoprotease, eine Metalloendoprotease, eine Aspartatprotease, eine Carboxypeptidase, eine Esterase oder eine Lipase verwendet wird oder verwendet wurde zur Bildung der Bindung zwischen E und R₂.

18. Verfahren nach Anspruch 17, dadurch gekennzeichnet, daß eine Thiolendoprotease, ausgewählt aus Papain und Chymopapain, oder eine Serinendoprotease, ausgewählt aus Subtilisin, Thermitase oder bevorzugt Chymotrypsin, oder eine Carboxypeptidase, ausgewählt aus CPD-B, CPD-MII und CPD-Y, verwendet wird oder verwendet wurde.

19. Verfahren nach einem der Ansprüche 1 bis 18, dadurch gekennzeichnet, daß eine Thiolendoprotease, eine Serinendoprotease, eine Aminopeptidase oder eine Penicilliumacylase verwendet wurde oder verwendet wird zur Herstellung der Bindung zwischen R₁ und A.

20. Verfahren nach Anspruch 19, dadurch gekennzeichnet, daß eine Thiolendoprotease, ausgewählt aus Papain und Chymopapain, eine Serinendoprotease, ausgewählt aus Trypsin, Chymotrypsin, für Postprolin spezifischer Endoprotease, Achromobacter-Lyticus-Protease I, Endoprotease ArgC und LysC und Thrombin, oder eine Aminopeptidase, ausgewählt aus Pyroglutaminase, Proliniminopeptidase oder N-Acylpeptidhydrolase, oder Penicillium-V- oder -G-Acylase verwendet wird oder verwendet wurde.

21. Geschütztes Dipeptid, dadurch gekennzeichnet, daß es durch die Formel Z₁ABOR darstellt ist, worin Z₁, A und B die Bedeutungen haben, die in Anspruch 1 festgelegt wurden, mit der Ausnahme, daß A und B nicht gleichzeitig L-Arg, D- oder L-Lys, L-Orn oder D- oder L-Dab sein können und, wenn A L-Orn ist, B nicht L-Arg sein kann, und wenn A L-Lys ist, B nicht L-Dab sein kann, und wenn A―B L-Arg―L-Lys oder L-Lys―L-Arg ist, Z₁ nicht ein Wasserstoffatom, ein N-Lauroylrest oder ein N-Palmitoylrest sein kann, und wenn A―B L-Arg―L-Pro ist, Z₁ nicht Fmoc sein kann und R eine geradkettige oder verzweigte Alkylgruppe mit 1 bis 6 Kohlenstoffatomen oder einen gegebenenfalls substituierten Benzylrest darstellt.

22. Geschütztes Dipeptid nach Anspruch 21, dadurch gekennzeichnet, daß es durch die Formel Z₁A'B'OR dargestellt wird, worin A' L- oder D-Arg oder L-Orn und B' L-Lys oder L-Pro ist und Z₁ und R die Bedeutungen haben, die in Anspruch 21 genannt wurden.

23. Geschütztes Dipeptid nach Anspruch 22, dadurch gekennzeichnet, daß es durch die Formel CbzArgLysOR dargestellt wird, worin R die Bedeutung hat, die in Anspruch 21 genannt wurde.

24. Geschütztes Dipeptid, dadurch gekennzeichnet, daß es durch die Formel Z₁ABC(OR')(OR'') dargestellt wird, worin Z₁, A, B und C die Bedeutungen haben, die in Anspruch 1 genannt wurden, mit der Ausnahme, daß A und B nicht beide gleichzeitig Lys sein können, C etwas anderes als Asp sein muß und R' und R'' unabhängig voneinander die Bedeutung haben, die für R in Anspruch 21 genannt wurde.

25. Geschütztes Tripeptid nach Anspruch 25, dadurch gekennzeichnet, daß es durch die Formel CbzArgLysGlu(OR)₂ dargestellt wird, wobei R die Bedeutung hat, die in Anspruch 21 genannt wurde.

26. Geschütztes Tripeptid, dadurch gekennzeichnet, daß es durch die Formel Z₁ABC(OR)OH dargestellt wird, worin Z₁, A, B, C und R die Bedeutungen haben, die in Anspruch 24 genannt wurden.

27. Geschütztes Tripeptid nach Anspruch 26, dadurch gekennzeichnet, daß es durch die Formel CbzArgLysGlu(OR)OH dargestellt wird, worin R die Bedeutung hat, die in Anspruch 21 genannt wurde.

28. Geschütztes Tetrapeptid, dadurch gekennzeichnet, daß es durch die Formel CbzArgLysC'(OR')D(OR''') dargestellt wird, worin C' Asp oder Glu ist, D die Bedeutung hat, die in Anspruch 5 genannt wurde, R' und R''' eine Alkylgruppe mit 1 bis 6 Kohlenstoffatomen oder ein Benzyl darstellen und R''' auch ein Wasserstoffatom sein kann.

## Revendications

1. Procédé pour la production de peptides répondant à la formule générale
**R₁-A-B-C-D-E-R₂**
dans laquelle A, B, C, D et E représentent des résidus d'acides aminés couplés entre eux par des liaisons peptidiques et
A est un résidu d'acide aminé basique de configuration L ou D, qui est substitué en position ω par le groupe amino ou guanidino, ou un dérivé désamino éventuellement substitué de tels acides aminés,
B est un résidu d'acide aminé L basique qui est substitué en position ω par le groupe amino ou guanidino, ou B est L-Pro ou L-déshydroPro,
C est un résidu d'acide aminé acide de configuration L ou D,
D est un résidu d'acide aminé L hydrophobe aliphatique non polaire ou un résidu d'acide aminé contenant un groupe aryle ou aralkyle homo- ou hétéroaromatique, portant éventuellement un substituant nitro, hydroxy, alcoxy, alkyle et/ou halogène sur le groupe aryle,
E est un résidu d'acide aminé aliphatique hydrophobe non polaire de configuration L ou D ou un résidu d'acide aminé de configuration L ou D contenant un groupe aryle ou aralkyle homo- ou hétéroaromatique, portant éventuellement un substituant nitro, hydroxy, alcoxy, alkyle et/ou halogène sur le groupe aryle, ou est un dérivé décarboxy éventuellement substitué de tels acides aminés,
R₁ est H ou un groupe substituant sur la fonction α-amino, tel qu'un groupe protecteur de la fonction α-amino susceptible de subir un clivage enzymatique ou chimique, ou est absent lorsque A est un dérivé désamino non substitué, et
R₂ est OH ou un groupe substituant sur la fonction α-carboxy, tel qu'un groupe protecteur de la fonction α-carboxy susceptible de subir un clivage enzymatique ou chimique, ou est absent lorsque E est un dérivé décarboxy non substitué,
par couplage successif des dérivés réactifs éventuellement protégés desdits acides aminés et/ou par couplage de fragments de dérivés de di-, tri- ou tétrapeptides réactifs éventuellement protégés correspondant auxdits acides aminés selon un ou plusieurs modèles réactionnels convenablement choisis parmi A+B, C+D, D+E, AB+C, C+DE, CD+E, ABC+D, AB+CD, AB+CDE, ABC+DE, ABCD+E, et lorsque cela s'avère souhaitable, par élimination des groupes protecteurs des chaînes terminales et/ou latérales éventuels, les groupes R₁ et R₂, lorsque cela s'avère souhaitable, étant introduits indépendamment l'un de l'autre suite à la formation du peptide recherché ABCDE ou pendant ou suite à l'une des réactions de couplage précédentes, procédé caractérisé en ce qu'il utilise en tant qu'intermédiaire un dérivé de dipeptide Z₁-AB-Y₂ dans lequel Z₁ est H ou un groupe substituant sur la fonction α-amino qui peut être identique ou différent de R₁, A et B ont la même signification que précédemment et Y₂ est OH ou un groupe substituant sur la fonction α-carboxy et dont les chaînes latérales respectives sont non protégées, ledit intermédiaire étant mis à réagir avec un dérivé de peptide ou d'acide amitié réactif choisi parmi C(X₃)-Y₃, C(X₃)-D(X₄)-Y₄ et C(X₃)-D(X₄)-E(X₅)-Y₅, dans lesquels C, D et E ont la même signification que précédemment, Y₃, Y₄ et Y₅ sont OH ou un groupe substituant sur la fonction α-carboxy, qui peuvent être identiques ou différents de R₂, et X₃, X₄ et X₅ sont H ou un groupe protecteur de la chaîne latérale ω, dans un milieu en partie ou entièrement aqueux à *p*H 3-12, de préférence à *p*H 5-10, en présence d'une enzyme carboxylester hydrolase ou peptide hydrolase choisie parmi les thiol endoprotéases choisies parmi la bromélaïne, la clostripaïne, la papaïne, la chymopapaïne et la ficine, ou de sérine endoprotéases choisies parmi la protéase I de *Achromobacter lyticus*, la thrombine, la subtilisine, l'endoprotéinase ArgC ou LysC, la trypsine et l'endopeptidase spécifique de la postproline ou les carboxypeptidases choisies parmi CPD-MI et de préférence CPD-Y ou CPD-MII ou la dipeptidyl peptidase I ou la dipeptidyl carboxypeptidase II, en vue de former un peptide, ledit peptide, lorsque cela s'avère souhaitable, étant couplé avec un dérivé d'acide aminé ou de peptide réactif en présence d'une enzyme protéolytique identique ou différente capable de catalyser la formation d'une liaison peptidique entre les réactifs, et le recours si besoin est, à d'autres réactions de couplage chimiques ou enzymatiques choisies parmi les modèles ci-dessus en vue de synthétiser un dérivé peptidique éventuellement protégé répondant à la formule générale
Z₁-A-B-C(X₃)-D(X₄)-E(X₅)-Y₅
dans laquelle A, B, C, D, Z₁, X₃, X₄, X₅ et Y₅ ont la même signification que précédemment, lesdits groupes substituants, lorsque cela s'avère souhaitable, étant éliminés par voie chimique ou enzymatique, et les groupes R₁ et/ou R₂ étant introduits, si nécessaire.

2. Procédé selon la revendication 1, caractérisé en ce qu'un intermédiaire Z₁-AB-Y₂ préparé préalablement en faisant réagir Z₁-A-Y₁ sur H-B-Y₂ est utilisé, dans lequel Z₁, A, B et Y₂ sont tels que définis dans la revendication 1 et Y₁ a la même signification que Y₂, dans les conditions définies dans la revendication 1 en présence d'une enzyme choisie parmi les thiol endoprotéases eux-mêmes choisies parmi la broméline, la ficine ou de préférence la clostripaïne, la papaïne et la chymopapaïne ou les sérine endoprotéases choisies parmi la protéase I d'*Achromobacter lyticus*, l'endoprotéinase ArgC ou LysC, la thrombine, la subtilisine et de préférence la trypsine, la dipeptidyl peptidase I ou les carboxypeptidases choisies parmi CPD-MI ou de préférence CPD-Y et CPD-MII.

3. Procédé pour la production de peptides répondant à la formule générale
**R₁-A-B-C-D-E-R₂**
dans laquelle A, B, C, D, E, R₁ et R₂ sont tels que définis dans la revendication 1,
par couplage successif des dérivés réactifs éventuellement protégés desdits acides aminés et/ou par couplage de fragments de dérivés de di-, tri- ou tétrapeptides réactifs éventuellement protégés correspondant auxdits acides aminés selon un ou plusieurs modèles réactionnels convenablement choisis parmi A+B, B+C, D+E, AB+C, A+BC, C+DE, B+CDE, BC+DE, A+BCDE, AB+CDE, ABC+DE, et, lorsque cela s'avère souhaitable, par élimination des groupes protecteurs des chaînes terminales et/ou latérales éventuels, les groupes R₁ et R₂, lorsque cela s'avère souhaitable, étant introduits indépendamment l'un de l'autre suite à la formation du peptide recherché ABCDE ou pendant ou suite à l'une des réaction de couplage précédentes, procédé caractérisé en ce qu'il utilise en tant qu'intermédiaire un dérivé de dipeptide D(X₄)-E(X₅)-Y₅ dans lequel D, E et Y₅ ont la même signification que précédemment, ledit intermédiaire étant mis à réagir avec un dérivé de peptide ou d'acide aminé réactif choisi parmi Z₃-C(X₃)-Y₃, Z₂-B(X₂)-C(X₃)-Y₃ et Z₁-A(X₁)-B(X₂)-C(X₃)-Y₃, dans lesquels A, B, C, Z₁, Z₃, X₃ et Y₃ ont la même signification que précédemment, Z₂ est H ou un groupe substituant de la fonction α-amino, et X₁ et X₂ sont H ou un groupe protecteur de la chaîne latérale ω, en milieu en partie ou entièrement aqueux à *p*H 3-12, de préférence à *p*H 5-10, en présence d'une enzyme carboxylester hydrolase ou de peptide hydrolase choisie parmi les métalloprotéinases, de préférence la thermolysine, les sérine endoprotéases choisies parmi la subtilisine, la thermitase ou de préférence l'enzyme de Drapeau V8 ou la chymotrypsine, les carboxypeptidases choisies parmi CPD-Y et CPD-MII, ou la dipeptidyl carboxypeptidase II, en vue de former un peptide, ledit peptide, lorsque cela s'avère souhaitable, étant couplé avec un dérivé de peptide ou d'acide amitié réactif en présence d'une enzyme protéolytique identique ou différente capable de catalyser la formation d'une liaison peptidique entre les réactifs, et le recours si nécessaire, à d'autres réactions de couplage chimiques ou enzymatiques choisies parmi les modèles ci-dessus en vue de synthétiser un dérivé peptidique éventuellement protégé répondant à la formule générale
Z₁-A(X₁)-B(X₂)-C(X₃)-D(X₄)-E(X₅)-Y₅
dans laquelle A, B, C, D, Z₁, X₁, X₂, X₃ et Y₅ ont la même signification que précédemment, lesdits groupes substituants, lorsque cela s'avère souhaitable, étant éliminés par voie chimique ou enzymatique, et les groupes R₁ et/ou R₂ étant introduits, si nécessaire.

4. Procédé selon l'une quelconque des revendications 1 à 3, caractérisé en ce que les composés sont produits dans lesquels les acides aminés basiques A ou B substitués en position ω répondent à la formule générale
R₁'R₂'N - (CH₂)ₙ - CH(NH₂)COOH (I)
or 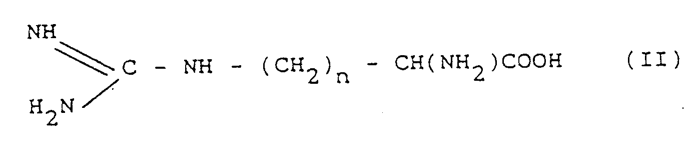 formules dans lesquelles n varie de 1 à 7, R₁' et R₂' désirent indépendamment H, un groupe alkyle en C₁-C₃ ou pyranosyle, ou R₁' et R₂' forment conjointement un groupe (CH₂)ₙ, où n' varie de 3 à 8, et A peut adopter en outre la forme D et/ou être un dérivé désamino desdits acides aminés, qui, lorsque cela s'avère souhaitable, peut être substitué avec un groupe alkyle en C₁-C₄, un groupe alkyle en C₁-C₄ hydroxylé ou un groupe alkyle en C₁-C₄ halogéné,
les acides amitiés acides C sont des acides amitiés substitués par un groupe carboxy en position ω répondant à la formule générale
HOOC - (CH₂)ₘ - CH(NH₂)COOH (III),
dans laquelle m = 0-7,
ou contiennent un autre groupe carboxy substituant ou un groupe de type acide sulfonique ou acide phosphorique en position ω au lieu du groupe carboxy en position ω de la formule III,
les acides aminés aliphatiques hydrophobes non polaires D répondent à la formule générale
R₁R₂R₃C- - CH(NH₂)COOH (IV)
dans laquelle
R₁, R₂ et R₃ désignent indépendamment H- ou une chaîne alkyle droite ou ramifiée ou
D est un acide amitié L répondant à la formule générale
Ar(CH₂)p - CH(NH₂)COOH (V),
dans laquelle
p varie de 0 à 5 et Ar est un groupe hétéro- ou homoaryle substitué éventuellement par un groupe nitro, hydroxy, alcoxy, et/ou halogène,
les acides aminés E peuvent avoir indépendamment la même signification que D, mais ont simultanément soit la configuration L soit la configuration D, ou sont des dérivés décarboxy desdits acides aminés qui, lorsque cela s'avère souhaitable, peuvent être substitués par des groupes alkyle en C₁-C₄, des groupes alkyle en C₁-C₄ hydroxylés ou encore être substitués par des atomes d'halogène, de préférence sont des dérivés fluorés, ou peuvent être Trp, Phe ou His substitués par des groupes nitro-, alcoxy-, hydroxy, et/ou des atomes d'halogène, et R₁ et R₂ sont tels que définis dans la revendication 1.

5. Procédé selon la revendication 4, caractérisé en ce que des peptides sont produits dans lesquels
A est Arg, Lys, Orn, homo-Arg ou homo-Lys de configuration L ou D,
B est Arg, Lys, Orn, homo-Arg, homo-Lys ou Pro de configuration L,
C est Asp, Glu, ou homo-Glu de configuration L ou D,
D est Val, Ile, Leu, Nor-Leu, Nor-Val, Tert-Leu, Phe, Tyr ou Ala de configuration L,
E est val, Ile, Leu, Nor-Leu, Nor-Val, Tert-Leu, Tyr, Trp, Phe, His ou Nal de configuration L ou D, ou un dérivé décarboxy de ceux-ci, ou Trp, Phe ou His substitué par un groupe nitro, alcoxy, hydroxy et/ou un atome d'halogène, et
R₁ et R₂ sont tels que définis dans la revendication 1.

6. Procédé selon la revendication 1 ou 3, caractérisé en ce que le dérivé de dipeptide Z₁-AB-Y₂ en tant que composant carboxy subit un couplage de fragment avec un dérivé de tripeptide éventuellement protégé C(X₃)-D(X₄)-E(X₅)Y₅ en tant que composant de type amine, formules dans lesquelles les symboles utilisés sont tels que définis dans la revendication 1, en présence d'une endoprotéase ou de dipeptidyl peptidase capable de catalyser la formation d'une liaison peptidique entre les composants, et en ce que les groupes protecteurs éventuellement présents, si on le souhaite, sont éliminés, et en ce que les groupes R₁ et/ou R₂ sont introduits, si nécessaire.

7. Procédé selon la revendication 6, caractérisé en ce que le dérivé de tripeptide C(X₃)-D(X₄)-E(X₅)-Y₅ utilisé a été obtenu par couplage d'un dérivé d'aicide aminé Z₃-C(X₃)-Y₃ avec un dérivé de dipeptide D(X₄)-E(X₅)-Y₅ en présence d'une endoprotéase ou d'une dipeptidyl carboxypeptidase.

8. Procédé selon la revendication 1 ou 3, caractérisé en ce qu'un dérivé de tripeptide éventuellement protégé Z₁-A(X₁)-B(X₂)-C(X₃)-Y₃ en tant que composant de type carboxy subit un couplage de fragment avec un dérivé de dipeptide éventuellement protégé Z₄-D(X₄)-E(X₅)-Y₅ en tant que composant de type amine, formules dans lesquelles les symboles utilisés sont tels que définis dans la revendication 1, en présence d'une endoprotéase, d'une lipase ou d'une dipeptidylcarboxypeptidase capable de catalyser la formation d'une liaison peptidique entre les composants, et en ce que les groupes protecteurs éventuellement présents, si on le souhaite, sont éliminés, et en ce que les groupes R₁ et/ou R₂ sont introduits, si nécessaire.

9. Procédé selon la revendication 8, caractérisé en ce que le dérivé de tripeptide Z₁-A(X₁)-B(X₂)-C(X₃)-Y₃ utilisé a été obtenu par couplage d'un dérivé d'acide amitié Z₁-A(X₁)-Y₁ avec un dérivé de dipeptide B(X₂)-C(X₃)-Y₃ en présence d'une endoprotéase ou d'une dipeptidyl peptidase ou par couplage d'une dérivé de dipeptide Z₁-A(X₁)-B(X₂)-Y₂ avec un dérivé d'acide aminé Z₃-C(X₃)-Y₃ en présence d'une endoprotéase ou d'une carboxypeptidase.

10. Procédé selon la revendication 1, caractérisé en ce qu'un dérivé de tétrapeptide éventuellement protégé Z₁-A-B-C(X₃)-D(X₄)-Y₄ en tant que composant de type carboxy est couplé avec un dérivé d'acide aminé éventuellement protégé E(X₅)-Y₅ en tant que composant de type amine, formules dans lesquelles les symboles utilisés sont tels que définis dans la revendication 1, en présence d'une endoprotéase, d'une lipase ou d'une carboxypeptidase capable de catalyser la formation d'une liaison peptidique entre les composants, et en ce que les groupes protecteurs éventuellement présents, si on le souhaite, sont éliminés, et en ce que les groupes R₁ et/ou R₂ sont introduits, si nécessaire.

11. Procédé selon la revendication 10, caractérisé en ce que le dérivé de tétrapeptide Z₁-A-B-C(X₃)-D(X₄)-Y₄ utilisé a été obtenu par couplage de fragments des dérivés de dipeptide Z₁-A-B-Y₂ et de C(X₃)-D(X₄)-Y₄ en présence d'une endoprotéase ou d'une dipeptidyl peptidase ou d'une dipeptidyl carboxypeptidase ou par couplage d'un dérivé de tripeptide Z₁-A-B-C(X₃)-Y₃ avec un dérivé d'acide aminé D(X₄)-Y₄ en présence d'une endoprotéase ou d'une carboxypeptidase.

12. Procédé selon l'une quelconque des revendications 1 à 11, caractérisé en ce qu'une thiol endoprotéase, une sérine endoprotéase, une carboxypeptidase, une dipeptidase ou une dipeptidyl peptidase est utilisée ou a été utilisée pour former la liaison entre A et B.

13. Procédé selon l'une quelconque des revendications 1 à 12, caractérisé en ce qu'une thiol endoprotéase, une sérine endoprotéase, une carboxypeptidase, une dipeptidase, une dipeptidyl peptidase ou une dipeptidyl carboxypeptidase est utilisée ou a été utilisée pour former la liaison entre B et C.

14. Procédé selon l'une quelconque des revendications 1 à 13, caractérisé en ce qu'une sérine endoprotéase, une métalloendoprotéase, une aspartate protéase, une carboxypeptidase, une dipeptidase, une estérase, une lipase ou une dipeptidyl carboxypeptidase est utilisée ou a été utilisée pour former la liaison entre C et D.

15. Procédé selon l'une quelconque des revendications 1 à 14, caractérisé en ce qu'une thiol endoprotéase, une sérine endoprotéase, une métalloendoprotéase, une carboxypeptidase, une dipeptidase, une estérase ou une lipase est utilisée ou a été utilisée pour former la liaison entre D et E.

16. Procédé selon la revendication 15, caractérisé en ce qu'une thermolysine ou une thiol endoprotéase choisie parmi la chymopapaïne, la ficine ou de préférence la papaïne ou la broméline, une sérine endoprotéase choisie parmi la thermitase ou de préférence la subtilisine, l'élastase, la valylprotéinase ou la chymotrypsine, ou une carboxypeptidase choisie parmi CPD-MII ou de préférence CPD-Y.

17. Procédé selon l'une quelconque des revendications 1 à 16, caractérisé en ce qu'une sérine endoprotéase, une métalloendoprotéase, une aspartate protéase, une carboxypeptidase, une estérase ou une lipase est utilisée ou a été utilisée pour former la liaison entre E et R₂.

18. Procédé selon la revendication 17, caractérisé en ce qu'une thiol endoprotéase choisie parmi la papaïne la chymopapaïne, ou une sérine endoprotéase choisie parmi la subtilisine, la thermitase ou de préférence la chymotrypsine, ou une carboxypeptidase choisie parmi CPD-B, CPD-MII et CPD-Y est utilisée ou a été utilisée.

19. Procédé selon l'une quelconque des revendications 1 à 18, caractérisé en ce qu'une thiol endoprotéase, une sérine endoprotéase, une aminopeptidase ou une pénicilline acylase est utilisée ou a été utilisée pour former la liaison entre R₁ et A.

20. Procédé selon la revendication 19, caractérisé en ce qu'une thiol endoprotéase choisie parmi la papaïne et la chymopapaïne, une sérine endoprotéase choisie parmi la trypsine, la chymotrypsine, l'endoprotéase spécifique de la postproline, la protéase I de *Achromobacter lyticus*, l'endoprotéinase ArgC et LysC et la thrombine, ou une aminopeptidase choisie parmi la pyroglutaminase, la proliniminopeptidase ou la N-acyl peptide hydrolase ou la pénicilline V ou G acylase est utilisée ou a été utilisée.

21. Dipeptide protégé, caractérisé en ce que qu'il s'agit de Z₁ABOR, dans lequel Z₁, A et B sont tels que définis dans la revendication 1 si ce n'est que A et B ne peuvent pas être tous les deux L-Arg, D- ou L-Lys, L-Orn ou D- ou L-Dab, et lorsque A est L-Orn alors B ne peut pas être L-Arg et lorsque A est L-Lys alors B ne peut pas être L-Dab, et lorsque AB est L-Arg-L-Lys ou L-Lys-L-Arg alors Z₁ ne peut pas être H, N-lauryle ou N-palimityle, et lorsque AB est L-Arg-L-Pro alors Z₁ ne peut pas être Fmoc et R désigne une chaîne alkyle en C₁-₆ droite ou ramifiée ou un groupe benzyle éventuellement substitué.

22. Dipeptide protégé selon la revendication 21, caractérisé en ce qu'il s'agit de Z₁A'B'OR, dans lequel A' est L- ou D-Arg ou L-Orn et B' est L-Lys ou L-Pro et Z₁ et R sont tels que définis dans la revendication 21.

23. Dipeptide protégé selon la revendication 22, caractérisé en ce qu'il s'agit de CbzArgLysOR, dans lequel R est tel que défini dans la revendication 21.

24. Tripeptide protégé caractérisé en ce qu'il s'agit de Z₁ABC(OR')(OR''), dans lequel Z₁, A, B et C sont tels que définis dans la revendication 1 si ce n'est que A et B ne peuvent pas être tous les deux Lys et C est différent de Asp et R' et R'' ont indépendamment la même signification que R telle que définie dans la revendication 21.

25. Tripeptide protégé selon la revendication 25, caractérisé en ce qu'il s'agit de CbzArgLysGlu(OR)₂ où R est tel que défini dans la revendication 21.

26. Tripeptide protégé, caractérisé en ce qu'il s'agit de Z₁ABC(OR)OH, où Z₁, A, B, C et R sont tels que définis dans la revendication 24.

27. Tripeptide protégé selon la revendication 26, caractérisé en ce qu'il s'agit de CbzArgLysGlu(OR)OH, où R est tel que défini dans la revendication 21.

28. Tripeptide protégé, caractérisé en ce qu'il s'agit de CbzArgLysC'(OR')D(OR'''), dans lequel C' est Asp ou Glu, D est tel que défini dans la revendication 5, R' et R''' sont des groupes alkyle en C₁-C₆ ou benzyle et R''' peut être également H.
